# EUROPEAN PATENT APPLICATION

(11) **EP 3 699 282 A1**
(43) Date of publication of application: **26.08.2020**
(21) Application number: 18868086.2
(22) Date of filing: 19.10.2018
(51) Int. Cl.: C12N 15/53, A23L 33/11, C07J 9/00, C12N 1/13, C12N 15/54, C12P 33/00

(54) **METHOD FOR PRODUCING STEROL**

(30) Priority: 19.10.2017 JP 2017202347
(71) Applicant: Ajinomoto Co., Inc., Tokyo 104-8315 (JP)
(72) Inventor: SUZUKI, Shigeo, Kawasaki-shi Kanagawa 210-8681 (JP); KISELEVA, Evgeniya Mikhailovna, Kawasaki-shi Kanagawa 210-8681 (JP)
(74) Representative: Strehl Schübel-Hopf & Partner
(86) International application number: PCT/JP2018/039009
(87) International publication number: WO 2019/078359

(57) **Abstract**

A method for producing a sterol is provided. A sterol is produced by culturing a Labyrinthulea microorganism having a sterol-producing ability, which has been modified so that the activity of 24-dehydrocholesterol reductase (DHCR24), 7-dehydrocholesterol reductase (DHCR7), or sterol 24-C-methyltransferase (SMT1) is reduced, and collecting the sterol from a culture broth.

## Description

### Technical Field

The present invention relates to a method for producing a sterol using a Labyrinthulea microorganism.

### Background Art

Sterols are steroid derivatives found in various organisms such as animals, plants, and fungi. Sterols are produced by, for example, extraction from such bioresources containing sterols. In addition, some Labyrinthulea microorganisms, which are heterotrophic microorganisms, are known to produce sterols such as cholesterol (Non-patent document 1). In addition, there have been reported methods for producing sterols using Labyrinthulea microorganisms.

For example, there have been reported a method for producing 7-dehydrocholesterol using Labyrinthulea microorganisms such as Aurantiochytrium limacinum having a reduced activity of 7-dehydrocholesterol reductase (also referred to as "DHCR7") (Patent document 1).

Also, for example, there have been reported a method for producing 7-dehydrocholesterol using Labyrinthulea microorganisms such as Aurantiochytrium limacinum having a reduced activity of sterol 24-C-methyltransferase (also referred to as "SMT1") (Patent document 2). According to Patent document 2, it was shown that accumulation of 7-dehydrocholesterol is increased by reducing activity of SMT1, whereas accumulation of cholesterol is reduced.

SMT1 is an enzyme introducing a methyl group at position C24 of sterols (EC 2.1.1.41). SMT1 is one of enzymes involved in biosynthesis of sterols, and specifically, it is involved in biosynthesis of plant sterols and fungal sterols. There has been known no method for producing cholesterol using Labyrinthulea microorganisms having a reduced activity of SMT1.

DHCR7 is an enzyme reducing a double bond at position C7 of sterols (EC 1.3.1.21). DHCR7 is one of enzymes involved in biosynthesis of sterols, and specifically, it is involved in biosynthesis of animal sterols such as cholesterol and 7-dehydrocholesterol.

24-dehydrocholesterol reductase (also referred to as "DHCR24") is an enzyme reducing a double bond at position C24 of sterols (EC 1.3.1.72). DHCR24 is one of enzymes involved in biosynthesis of sterols, and specifically, it is involved in biosynthesis of animal sterols such as cholesterol and 7-dehydrocholesterol. There has been known no method for producing plant sterols or fungal sterols using Labyrinthulea microorganisms having a reduced activity of DHCR24.

### Prior art references

### Patent documents

Patent document 1: WO2015/108058
Patent document 2: WO2016/056610

### Non-patent documents

Non-patent document 1: Weete JD, et al., Lipids and ultrastructure of Thraustochytrium sp. ATCC 26185., Lipids. 1997 Aug;32(8):839-45.

### Summary of the Invention

### Object to be Achieved by the Invention

An object of the present invention is to provide a method for producing a sterol.

### Means for Achieving the Object

In order to achieve the aforementioned object, the inventors of the present invention conducted various researches. As a result, they found that a sterol-producing ability of a Labyrinthulea microorganism is improved by modifying the microorganism so that the activity of DHCR24, DHCR7, or SMT1 is reduced, and accomplished the present invention.

The present invention can be thus embodied, for example, as follows.
[1] A Labyrinthulea microorganism,
   which has a sterol-producing ability,
   which is Aurantiochytrium sp. 1,
   which has been modified so that the activity of 24-dehydrocholesterol reductase is reduced as compared with a non-modified strain, and
   wherein the sterol is at least one sterol selected from plant sterols and fungal sterols.
[2] The microorganism mentioned above, wherein the activity of the 24-dehydrocholesterol reductase is reduced by reducing the expression of a gene encoding the 24-dehydrocholesterol reductase or disrupting the gene.
[3] The microorganism mentioned above, wherein the activity of the 24-dehydrocholesterol reductase is reduced by deletion of a part or the whole of the amino acid sequence of the 24-dehydrocholesterol reductase.
[4] The microorganism mentioned above, wherein the sterol is stigmasterol and/or brassicasterol.
[5] A Labyrinthulea microorganism,
   which has a sterol-producing ability,
   which is Aurantiochytrium sp. 1,
   which has been modified so that the activity of 7-dehydrocholesterol reductase is reduced as compared with a non-modified strain, and
   wherein the sterol is 7-dehydrocholesterol and/or ergosterol.
[6] The microorganism mentioned above, wherein the activity of the 7-dehydrocholesterol reductase is reduced by reducing the expression of a gene encoding the 7-dehydrocholesterol reductase or disrupting the gene.
[7] The microorganism mentioned above, wherein the activity of the 7-dehydrocholesterol reductase is reduced by deletion of a part or the whole of the amino acid sequence of the 7-dehydrocholesterol reductase.
[8] The microorganism mentioned above, which has been further modified so that the activity of sterol 24-C-methyltransferase is reduced as compared with a non-modified strain.
[9] A Labyrinthulea microorganism,
   which has a sterol-producing ability,
   which is Aurantiochytrium sp. 1,
   which has been modified so that the activity of sterol 24-C-methyltransferase is reduced as compared with a non-modified strain, and
   wherein the sterol is at least one sterol selected from animal sterols.
[10] The microorganism mentioned above, wherein the sterol 24-C-methyltransferase is a protein defined in (a), (b), or (c) mentioned below:
   (a) a protein comprising the amino acid sequence of SEQ ID NO: 38 or 56;
   (b) a protein comprising the amino acid sequence of SEQ ID NO: 38 or 56, but which includes substitution, deletion, insertion, and/or addition of 1 to 10 amino acid residues, and having a sterol 24-C-methyltransferase activity;
   (c) a protein comprising an amino acid sequence showing an identity of 90% or higher to the amino acid sequence of SEQ ID NO: 38 or 56, and having a sterol 24-C-methyltransferase activity.
[11] The microorganism mentioned above, wherein the activity of the 24-dehydrocholesterol reductase is reduced by reducing the expression of a gene encoding the 24-dehydrocholesterol reductase or disrupting the gene.
[12] The microorganism mentioned above, wherein the activity of the 24-dehydrocholesterol reductase is reduced by deletion of a part or the whole of the amino acid sequence of the 24-dehydrocholesterol reductase.
[13] The microorganism mentioned above, wherein the sterol is cholesterol and/or 7-dehydrocholesterol.
[14] The microorganism mentioned above, wherein the sterol is cholesterol.
[15] The microorganism mentioned above, which further has at least one characteristic selected from (A), (B), and (C) mentioned below:
   (A) the microorganism has been modified so that the activity of sterol C-22 desaturase is reduced as compared with a non-modified strain;
   (B) the microorganism has been modified so that the activity of 24-dehydrocholesterol reductase is increased as compared with a non-modified strain;
   (C) the microorganism has been modified so that the activity of 7-dehydrocholesterol reductase is increased as compared with a non-modified strain.
[16] The microorganism mentioned above, wherein the sterol C-22 desaturase is a protein defined in (a), (b), or (c) mentioned below:
   (a) a protein comprising the amino acid sequence of SEQ ID NO: 42;
   (b) a protein comprising the amino acid sequence of SEQ ID NO: 42, but which includes substitution, deletion, insertion, and/or addition of 1 to 10 amino acid residues, and having a sterol C-22 desaturase activity;
   (c) a protein comprising an amino acid sequence showing an identity of 90% or higher to the amino acid sequence of SEQ ID NO: 42, and having a sterol C-22 desaturase activity.
[17] The microorganism mentioned above, 15, and 16, wherein the 24-dehydrocholesterol reductase is a protein defined in (a), (b), or (c) mentioned below:
   (a) a protein comprising the amino acid sequence of SEQ ID NO: 40;
   (b) a protein comprising the amino acid sequence of SEQ ID NO: 40, but which includes substitution, deletion, insertion, and/or addition of 1 to 10 amino acid residues, and having a 24-dehydrocholesterol reductase activity;
   (c) a protein comprising an amino acid sequence showing an identity of 90% or higher to the amino acid sequence of SEQ ID NO: 40, and having a 24-dehydrocholesterol reductase activity.
[18] The microorganism mentioned above and 15 to 17, wherein the 7-dehydrocholesterol reductase is a protein defined in (a), (b), or (c) mentioned below:
   (a) a protein comprising the amino acid sequence of SEQ ID NO: 39;
   (b) a protein comprising the amino acid sequence of SEQ ID NO: 39, but which includes substitution, deletion, insertion, and/or addition of 1 to 10 amino acid residues, and having a 7-dehydrocholesterol reductase activity;
   (c) a protein comprising an amino acid sequence showing an identity of 90% or higher to the amino acid sequence of SEQ ID NO: 39, and having a 7-dehydrocholesterol reductase activity.
[19] The microorganism mentioned above, wherein the nucleotide sequence of the 18S rDNA of the microorganism has an identity of 97% or higher to the nucleotide sequence of SEQ ID NO: 3.
[20] The microorganism mentioned above, which is a modified strain derived from Aurantiochytrium sp.1 strain AJ7869 (FERM BP-22342), AJ7868 (FERM BP-22290), or AJ7879 (FERM BP-22367).
[21] A method for producing a sterol, comprising:
   culturing the microorganism mentioned above in a culture medium; and
   collecting the sterol from cells generated by the culturing.
[22] The method mentioned above, wherein the sterol is a free sterol, a sterol ester, a steryl glucoside, an acyl steryl glucoside, or a mixture thereof.

### Modes for Carrying out the Invention

Hereinafter, the present invention will be explained in detail.

### <1> Microorganism of the present invention

The microorganism of the present invention is a Labyrinthulea microorganism having a sterol-producing ability, which has been modified so that the activity of DHCR24, DHCR7, or SMT1 is reduced.

### <1-1> Labyrinthulea microorganism

The Labyrinthulea microorganism used in the present invention is Aurantiochytrium sp. 1. The phrase "Aurantiochytrium sp.1" refers to one specific species of the genus Aurantiochytrium, and specifically refers to the species to which the Aurantiochytrium sp.1 strain AJ7869 (FERM BP-22342) belongs. In other words, the phrase "Aurantiochytrium sp.1" specifically refers to a group of microorganisms consisting of the Aurantiochytrium sp.1 strain AJ7869 and strains belonging to the same species as that strain. Examples of the strains belonging to the same species as the Aurantiochytrium sp.1 strain AJ7869 include strains having 18S rDNA of which the nucleotide sequence has an identity of 97% or higher to the nucleotide sequence of 18S rDNA of the Aurantiochytrium sp.1 strain AJ7869 (SEQ ID NO: 3). Specific examples of Aurantiochytrium sp.1 include strains AJ7867 (FERM BP-22304), AJ7868 (FERM BP-22290), AJ7879 (FERM BP-22367), BURABG162, SKE217, and SKE218, as well as the strain AJ7869. In addition, derivative strains, such as modified strains, derived from the strains belonging to Aurantiochytrium sp.1 exemplified above are included in Aurantiochytrium sp.1.

The strain AJ7869 was originally deposited at independent administrative agency, National Institute of Technology and Evaluation, International Patent Organism Depositary (#120, 2-5-8 Kazusakamatari, Kisarazu-shi, Chiba-ken, 292-0818, Japan) on August 8, 2017 with an accession number of FERM P-22342, and then converted to an international deposit under the provisions of the Budapest Treaty on July 8, 2018, and assigned an accession number of FERM BP-22342.

The strain AJ7868 was originally deposited at independent administrative agency, National Institute of Technology and Evaluation, International Patent Organism Depositary (#120, 2-5-8 Kazusakamatari, Kisarazu-shi, Chiba-ken, 292-0818, Japan) on August 5, 2015 with an accession number of FERM P-22290, and then converted to an international deposit under the provisions of the Budapest Treaty on August 31, 2016, and assigned an accession number of FERM BP-22290.

The strain AJ7879 was originally deposited at independent administrative agency, National Institute of Technology and Evaluation, International Patent Organism Depositary (#120, 2-5-8 Kazusakamatari, Kisarazu-shi, Chiba-ken, 292-0818, Japan) on August 27, 2018 as an international deposit under the provisions of the Budapest Treaty, and assigned an accession number of FERM BP-22367.

These strains are available from, for example, the American Type Culture Collection (Address: 12301 Parklawn Drive, Rockville, Maryland 20852, P.O. Box 1549, Manassas, VA 20108, United States of America). That is, registration numbers are given to the respective strains, and the strains can be ordered by using these registration numbers (refer to http://www.atcc.org/). The registration numbers of the strains are listed in the catalogue of the American Type Culture Collection. These strains can also be obtained from, for example, the depositories at which the strains were deposited.

The microorganism of the present invention can be obtained by appropriately modifying a Labyrinthulea microorganism such as those exemplified above. That is, the microorganism of the present invention may be a modified strain derived from a Labyrinthulea microorganism such as those exemplified above. The microorganism of the present invention may be, particularly, a modified strain derived from the Aurantiochytrium sp.1 strain AJ7869, AJ7868, or AJ7879. The microorganism of the present invention may be, more particularly, a modified strain derived from the Aurantiochytrium sp.1 strain AJ7869.

### <1-2> Sterol-producing ability

The microorganism of the present invention has a sterol-producing ability. The phrase "a microorganism having a sterol-producing ability" refers to a microorganism having an ability to generate an objective sterol and accumulate it in cells of the microorganism in such a degree that the sterol can be collected, when the microorganism is cultured in a culture medium.

The microorganism of the present invention may be a microorganism inherently having the sterol-producing ability or may be a microorganism modified so as to have the sterol-producing ability. For example, a Labyrinthulea microorganism such as those exemplified above can be used as a microorganism having the sterol-producing ability, as it is, or after impartation or enhancement of the sterol-producing ability. Methods for imparting or enhancing the sterol-producing ability are not particularly limited. For example, as described below, the sterol-producing ability can be imparted or enhanced by modifying a Labyrinthulea microorganism so that the activity of DHCR24, DHCR7, or SMT1 is reduced. Also, for example, a cholesterol-producing ability can be imparted or enhanced by modifying a Labyrinthulea microorganism so that expression of an enzyme involved in biosynthesis of cholesterol is enhanced or the like (WO2016/056610).

Examples of the objective sterol when the microorganism of the present invention has been modified so that the activity of DHCR24 is reduced include plant sterols and fungal sterols. Particular examples of the objective sterol when the microorganism of the present invention has been modified so that the activity of DHCR24 is reduced include plant sterols. The total production amount of plant sterol and fungal sterol to the total production amount of sterol may be, for example, 90%(w/w) or more, 95%(w/w) or more, 97%(w/w) or more, or 99%(w/w) or more. The phrases "plant sterol" and "fungal sterol" refers to, respectively, a sterol found in a plant and a sterol found in a fungus. Examples of the plant sterols include 7-dehydropolyferosterol, stigmasterol, 4-methyl-7,22-stigmasteradienol, β-sitosterol, brassicasterol, campesterol, diosgenin, oleanolic acid, betulinic acid, ursolic acid, hecogenin, sarsasapogenin, isofucosterol, avenasterol, Δ7-stigmastenol, A7-campestenol, fucosterol, and salgasterol. Particular examples of the plant sterols include those having a methyl group or ethyl group at position C24. More particular examples of the plant sterols include stigmasterol and brassicasterol. Examples of the fungal sterols include ergosterol and 7-dihydroergosterol. Particular examples of the fungal sterols include ergosterol.

Examples of the objective sterol when the microorganism of the present invention has been modified so that the activity of DHCR7 is reduced include provitamins D. Examples of the provitamins D include 7-dehydrocholesterol (provitamin D3) and ergosterol (provitamin D2). Particular examples of the provitamins D include 7-dehydrocholesterol. The production amount of provitamin D to the total production amount of sterol may be, for example, 50%(w/w) or more, 70%(w/w) or more, 80%(w/w) or more, or 85%(w/w) or more.

Examples of the objective sterol when the microorganism of the present invention has been modified so that the activity of SMT1 is reduced include animal sterols. The production amount of animal sterol to the total production amount of sterol may be, for example, 90%(w/w) or more, 95%(w/w) or more, 97%(w/w) or more, or 99%(w/w) or more. The phrase "animal sterol" refers to a sterol found in an animal. Examples of the animal sterols include cholesterol, 7-dehydrocholesterol, glycocholic acid, taurocholic acid, cholic acid, estradiol, estrone, ethinylestradiol, estriol, dehydroepiandrosterone, methylandrostenediol, 5β-pregnane-3α, 20α-diol, pregnenolone, chenodeoxycholic acid, dehydrocholic acid, dihydroxycholesterol, digitoxygenin, desmosterol, and lathosterol. Particular examples of the animal sterols include cholesterol and 7-dehydrocholesterol. More particular examples of the animal sterols include cholesterol.

When the microorganism of the present invention has two or more modifications, the objective sterol can be appropriately selected according to various conditions such as a combination of modifications. For example, examples of the objective sterol when the microorganism of the present invention has been modified so that the activity of DHCR7 and SMT1 is reduced include 7-dehydrocholesterol (provitamin D3). Also, for example, examples of the objective sterol when the microorganism of the present invention has been modified so that the activity of SMT1 and sterol C-22 desaturase is reduced include animal sterols such as cholesterol. Also, for example, examples of the objective sterol when the microorganism of the present invention has been modified so that the activity of SMT1 is reduced and the activity of DHCR24 is increased include a part of animal sterols such as cholesterol, lathosterol, and 7-dehydrocholesterol. Also, for example, examples of the objective sterol when the microorganism of the present invention has been modified so that the activity of SMT1 is reduced and the activity of DHCR7 is increased include cholesterol.

The microorganism of the present invention may have an ability to produce a single kind of sterol, or two or more kinds of sterols.

Sterols may be present not only in a free form, but also in a form of various derivatives. Hence, the phrase "sterol" may collectively refer to a free sterol and derivatives thereof, unless otherwise stated. That is, the phrase "sterol" may refer to a free sterol, a derivative thereof, or a mixture thereof. Examples of the derivatives of sterols include sterol esters, steryl glucosides, and acyl steryl glucosides. That is, the phrase "sterol" may refer to, specifically, a free sterol, a sterol ester, a steryl glucoside, an acyl steryl glucoside, or a mixture thereof.

The phrase "sterol ester" refers to an acyl ester of a free sterol, i.e. a compound having a structure in which a free sterol and a fatty acid is mutually bound via an ester bond. In other words, a sterol ester has a moiety corresponding to a free sterol and a moiety corresponding to a fatty acid, wherein these moieties mutually bound via an ester bond. The moiety corresponding to a free sterol is also referred to as "sterol moiety". The moiety corresponding to a fatty acid is also referred to as "fatty acid moiety" or "acyl moiety". The ester bond can be formed, for example, between a hydroxyl group at position C3 of a sterol and a carboxyl group of a fatty acid. Hence, when a sterol is present in a form of an ester, it is acceptable that the hydroxyl group at position C3 of the sterol does not remain. The type of the acyl group is not particularly limited. That is, the chain length and the unsaturation degree of the acyl group are variable. The chain length of the acyl group may be, for example, C14-C26 (e.g. C14, C16, C18, C20, C22, C24, or C26). The acyl group may be saturated or unsaturated. The acyl group may have one or more (e.g. one, two, three, four, five, or six) unsaturated double bonds. Specific examples of fatty acids corresponding to the acyl group include, for example, myristic acid (14:0), palmitic acid (16:0), stearic acid (18:0), arachidic acid (20:0), behenic acid (22:0), lignoceric acid (24:0), cerotic acid (26:0), myristoleic acid (14:1), palmitoleic acid (16:1), oleic acid (18:1), linoleic acid (18 :2), linolenic acid (18:3), arachidonic acid (20:4), eicosapentaenoic acid (EPA, 20:5), docosapentaenoic acid (DPA, 22:5), docosahexaenoic acid (DHA, 22:6). Particular examples of fatty acids corresponding to the acyl group include docosahexaenoic acid (DHA, 22:6), docosapentaenoic acid (DPA, 22:5), and palmitic acid (16:0).

The phrase "steryl glucoside" refers to a glucoside of a free sterol. The phrase "acyl steryl glucoside" refers to a glucoside of a sterol ester.

### <1-3> Reduction in activity of DHCR24, DHCR7, or SMT1

The microorganism of the present invention has been modified so that the activity of DHCR24, DHCR7, or SMT1 is reduced. The microorganism of the present invention has been modified, specifically, so that the activity of DHCR24, DHCR7, or SMT1 is reduced as compared with a non-modified strain. By modifying a Labyrinthulea microorganism so that the activity of DHCR24, DHCR7, or SMT1 is reduced, the sterol-producing ability of the microorganism can be improved, and that is, sterol production by the microorganism can be increased. Examples of an increase in sterol production include an increase in sterol production amount, an increase in sterol production rate, and an increase in sterol yield. Examples of the increase in sterol production amount include an increase in sterol production amount per weight of cells and an increase in sterol production amount per amount of a culture broth. Examples of an increase in sterol production also include an increase in the ratio of the production amount of an objective sterol to the total production amount of sterol(s). The ratio of the production amount of an objective sterol to the total production amount of sterol(s) may be, for example, 50%(w/w) or more, 70%(w/w) or more, 80%(w/w) or more, 85%(w/w) or more, 90%(w/w) or more, 95%(w/w) or more, 97%(w/w) or more, or 99%(w/w) or more. Specifically, a reduction in the activity of DHCR24 can provide an increased production of a plant sterol and/or a fungal sterol. Also, specifically, a reduction in the activity of DHCR7 can provide an increased production of a provitamin D. Also, specifically, a reduction in the activity of SMT1 can provide an increased production of an animal sterol.

The activity of DHCR7 and SMT1 may be reduced in combination. In other words, when the activity of DHCR7 is reduced, the activity of SMT1 may further be reduced. Also, when the activity of SMT1 is reduced, the activity of DHCR7 may further be reduced. For example, a reduction in the activity of DHCR7 and SMT1 can provide an increased production of 7-dehydrocholesterol (provitamin D3).

The microorganism of the present invention can be obtained by modifying a Labyrinthulea microorganism having a sterol-producing ability so that the activity of DHCR24, DHCR7, or SMT1 is reduced. The microorganism of the present invention can also be obtained by modifying a Labyrinthulea microorganism so that the activity of DHCR24, DHCR7, or SMT1 is reduced, and then imparting or enhancing a sterol-producing ability. The microorganism of the present invention may also be a microorganism that has acquired a sterol-producing ability by being modified so that the activity of DHCR24, DHCR7, or SMT1 is reduced. A strain before being modified so that the activity of DHCR24, DHCR7, or SMT1 is reduced may have an ability to produce a sterol other than the objective sterol. That is, for example, a Labyrinthulea microorganism having an ability to produce an animal sterol may be modified so that the activity of DHCR24 is reduced. Also, for example, a Labyrinthulea microorganism having an ability to produce cholesterol may be modified so that the activity of DHCR7 is reduced. Also, for example, a Labyrinthulea microorganism having an ability to produce a plant sterol and/or a fungal sterol may be modified so that the activity of SMT1 is reduced. Modifications for constructing the microorganism of the present invention can be performed in any order.

Hereinafter, DHCR24, DHCR7, and SMT1, and genes encoding them are described. Incidentally, the descriptions provided below can be applied not only to DHCR24, DHCR7, and SMT1 of which the activity is reduced in the microorganism of the present invention, but also to DHCR24 and DHCR7 of which the activity is increased as other modifications described below. DHCR24, DHCR7, and SMT1 of which the activity is reduced in the microorganism of the present invention are those possessed by a Labyrinthulea microorganism to be modified.

The phrase "24-dehydrocholesterol reductase (DHCR24)" refers to a protein (enzyme) having an activity of catalyzing a reaction of reducing a double bond at position C24 (a C24-C25 double bond) of a sterol (EC 1.3.1.72). This activity is also referred to as "24-dehydrocholesterol reductase activity (DHCR24 activity)". A gene encoding DHCR24 is also referred to as "24-dehydrocholesterol reductase gene (DHCR24 gene)" or "dhcr24 gene". The phrase "position C24" referred to herein refers to a carbon corresponding to position C24 of zymosterol.

The DHCR24 activity can be measured by, for example, incubating an enzyme with a substrate (a sterol having a double bond at position C24) in the presence of an electron donor, and measuring the enzyme- and substrate-dependent generation of a product (a sterol of which the double bond at position C24 was reduced). Examples of the electron donor include, for example, NADPH. Examples of the substrate and product include, for example, zymosterol and 5a-cholest-8-en-3b-ol, respectively.

The phrase "7-dehydrocholesterol reductase (DHCR7)" refers to a protein (enzyme) having an activity of catalyzing a reaction of reducing a double bond at position C7 (C7-C8 double bond) of a sterol (EC 1.3.1.21). This activity is also referred to as "7-dehydrocholesterol reductase activity (DHCR7 activity)". A gene encoding DHCR7 is also referred to as "7-dehydrocholesterol reductase gene (DHCR7 gene)" or "dhcr7 gene". The phrase "position C7" referred to herein refers to a carbon corresponding to position C7 of 7-dehydrocholesterol.

The DHCR7 activity can be measured by, for example, incubating an enzyme with a substrate (a sterol having a double bond at position C7) in the presence of an electron donor, and measuring the enzyme- and substrate-dependent generation of a product (a sterol of which the double bond at position C7 was reduced). Examples of the electron donor include, for example, NADPH. Examples of the substrate and product include, for example, 7-dehydrocholesterol and cholesterol, respectively.

The phrase "sterol 24-C-methyltransferase (SMT1)" refers to a protein (enzyme) having an activity of catalyzing a reaction of introducing a methyl group at position C24 of a sterol (EC 2.1.1.41). This activity is also referred to as "sterol 24-C-methyltransferase activity (SMT1 activity)". A gene encoding SMT1 is also referred to as "sterol 24-C-methyltransferase gene (SMT1 gene)" or "smt1 gene". Examples of sterols to be used as a substrate of SMT1 include sterols having a double bond at position C24 (a C24-C25 double bond). Introduction of a methyl group may be accompanied by reduction of a double bond at position C24. In addition, the introduced methyl group may also remain after conversion to a methylene group. In other words, the product of SMT1 may also be a sterol of which the double bond at position C24 was reduced and which was introduced with a methylene group at position C24. The phrase "position C24" referred to herein refers to a carbon corresponding to position C24 of zymosterol.

The SMT1 activity can be measured by, for example, incubating an enzyme with a substrate (a sterol) in the presence of a methyl group donor, and measuring the enzyme- and substrate-dependent generation of a product (a sterol introduced with a methyl group or a methylene group at position C24). Examples of the methyl group donor include, for example, S-adenosyl-L-methionine. Examples of the substrate and product include, for example, zymosterol and fecosterol, respectively.

A reduction in the activity of DHCR24, DHCR7, or SMT1 can be confirmed by measuring the activity of them or the like as described below. A reduction in the activity of DHCR24, DHCR7, or SMT1 can also be confirmed on the basis of an increase in production of an objective sterol as an indicator. A reduction in the activity of DHCR24, DHCR7, or SMT1 can also be confirmed on the basis of a reduction in production of a sterol other than the objective sterol as an indicator.

Examples of DHCR24, DHCR7, and SMT1, and genes encoding them include those of various organisms such as Labyrinthulea microorganisms. The nucleotide sequences of DHCR24, DHCR7, and SMT1 genes possessed by various organisms such as Labyrinthulea microorganisms and the amino acid sequences of DHCR24, DHCR7, and SMT1 encoded thereby can be obtained from, for example, public databases such as NCBI. The nucleotide sequence of DHCR24 gene of the Aurantiochytrium sp.1 strain AJ7869 and the amino acid sequence of DHCR24 encoded by the gene are shown in SEQ ID NOS: 29 and 40, respectively. That is, DHCR24 gene may be, for example, a gene having the nucleotide sequence shown as SEQ ID NO: 29. Also, DHCR24 may be, for example, a protein having the amino acid sequence shown as SEQ ID NO: 40. The nucleotide sequence of DHCR7 gene of the Aurantiochytrium sp.1 strain AJ7869 and the amino acid sequence of DHCR7 encoded by the gene are shown in SEQ ID NOS: 18 and 39, respectively. That is, DHCR7 gene may be, for example, a gene having the nucleotide sequence shown as SEQ ID NO: 18. Also, DHCR7 may be, for example, a protein having the amino acid sequence shown as SEQ ID NO: 39. The nucleotide sequence of SMT1 gene of the Aurantiochytrium sp.1 strain AJ7869 and the amino acid sequence of SMT1 encoded by the gene are shown in SEQ ID NOS: 7 and 38, respectively. The nucleotide sequence of SMT1 gene of the Aurantiochytrium sp.1 strains AJ7868 and AJ7879 and the amino acid sequence of SMT1 encoded by the gene are shown in SEQ ID NOS: 55 and 56, respectively. The Aurantiochytrium sp.1 strains AJ7868 has two copies of SMT1 gene. That is, SMT1 gene may be, for example, a gene having the nucleotide sequence shown as SEQ ID NO: 7 or 55. Also, SMT1 may be, for example, a protein having the amino acid sequence shown as SEQ ID NO: 38 or 56. The phrase "having a nucleotide or amino acid sequence" refers to cases of comprising the nucleotide or amino acid sequence unless otherwise stated, and also includes cases of consisting of the nucleotide or amino acid sequence.

DHCR24 gene may be a variant of any of the DHCR24 genes exemplified above (such as a gene having the nucleotide sequence shown as SEQ ID NO: 29), so long as the original function thereof is maintained. Similarly, DHCR24 may be a variant of any of the DHCR24s exemplified above (such as a protein having the amino acid sequence shown as SEQ ID NO: 40), so long as the original function thereof is maintained. DHCR7 gene may be a variant of any of the DHCR7 genes exemplified above (such as a gene having the nucleotide sequence shown as SEQ ID NO: 18), so long as the original function thereof is maintained. Similarly, DHCR7 may be a variant of any of the DHCR7s exemplified above (such as a protein having the amino acid sequence shown as SEQ ID NO: 39), so long as the original function thereof is maintained. SMT1 gene may be a variant of any of the SMT1 genes exemplified above (such as a gene having the nucleotide sequence shown as SEQ ID NO: 7 or 55), so long as the original function thereof is maintained. Similarly, SMT1 may be a variant of any of the SMT1s exemplified above (such as a protein having the amino acid sequence shown as SEQ ID NO: 38 or 56), so long as the original function thereof is maintained. Such a variant is also referred to as "conservative variant". The phrases "DHCR24 gene", "DHCR7 gene", and "SMT1 gene" include not only the DHCR24 genes, DHCR7 genes, and SMT1 genes exemplified above, but also include conservative variants thereof, respectively. Similarly, the phrases "DHCR24", "DHCR7", and "SMT1" include not only the DHCR24s, DHCR7s, and SMT1s exemplified above, but also include conservative variants thereof, respectively. Examples of the conservative variants include, for example, homologues and artificially modified versions of the DHCR24 genes, DHCR7 genes, and SMT1 genes and DHCR24s, DHCR7s, and SMT1s exemplified above.

The expression "the original function is maintained" means that a variant of a gene or protein has a function (such as activity or property) corresponding to the function (such as activity or property) of the original gene or protein. The expression "the original function is maintained" used for a gene means that a variant of the gene encodes a protein that maintains the original function. That is, the expression "the original function is maintained" used for DHCR24 gene means that a variant of the gene encodes a protein having the DHCR24 activity. Also, the expression "the original function is maintained" used for DHCR24 means that a variant of the protein has the DHCR24 activity. Also, the expression "the original function is maintained" used for DHCR7 gene means that a variant of the gene encodes a protein having the DHCR7 activity. Also, the expression "the original function is maintained" used for DHCR7 means that a variant of the protein has the DHCR7 activity. Also, the expression "the original function is maintained" used for SMT1 gene means that a variant of the gene encodes a protein having the SMT1 activity. Also, the expression "the original function is maintained" used for SMT1 means that a variant of the protein has the SMT1 activity.

Hereinafter, examples of the conservative variants will be explained.

Homologues of DHCR24, DHCR7, or SMT1 gene and homologues of DHCR24, DHCR7, or SMT1 can be easily obtained from public databases by, for example, BLAST search or FASTA search using any of the nucleotide sequences of the DHCR24 genes, DHCR7 genes, and SMT1 genes exemplified above or any of the amino acid sequences of the DHCR24s, DHCR7s, and SMTls exemplified above as a query sequence. Furthermore, homologues of DHCR24, DHCR7, or SMT1 gene can be obtained by, for example, PCR using a chromosome of organisms such as Labyrinthulea microorganisms as the template, and oligonucleotides prepared on the basis of any of the nucleotide sequences of these known DHCR24, DHCR7, or SMT1 genes as primers.

DHCR24, DHCR7, or SMT1 gene may be a gene encoding a protein having any of the amino acid sequences exemplified above (such as the amino acid sequence shown as SEQ ID NO: 40 for DHCR24, the amino acid sequence shown as SEQ ID NO: 39 for DHCR7, and the amino acid sequence shown as SEQ ID NO: 38 or 56 for SMT1), but which includes substitution, deletion, insertion, and/or addition of one or several amino acid residues at one or several positions, so long as the original function thereof is maintained. For example, the N-terminus and/or the C-terminus of the encoded protein may be elongated or shortened. Although the number meant by the term "one or several" mentioned above may differ depending on the positions of amino acid residues in the three-dimensional structure of the protein or the types of amino acid residues, specifically, it is, for example, 1 to 50, 1 to 40, 1 to 30, preferably 1 to 20, more preferably 1 to 10, still more preferably 1 to 5, particularly preferably 1 to 3.

The aforementioned substitution, deletion, insertion, and/or addition of one or several amino acid residues is a conservative mutation that maintains the normal function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, or addition of amino acid residues as mentioned above includes a naturally occurring mutation due to an individual difference, or a difference of species of the organism from which the gene is derived (mutant or variant).

DHCR24, DHCR7, or SMT1 gene may also be a gene encoding a protein having an amino acid sequence showing a homology of, for example, 50% or more, 65% or more, 80% or more, preferably 90% or more, more preferably 95% or more, still more preferably 97% or more, particularly preferably 99% or more, to the total amino acid sequence of any of the aforementioned amino acid sequences, so long as the original function thereof is maintained. In this description, "homology" means "identity".

DHCR24, DHCR7, or SMT1 gene may also be DNA that is able to hybridize under stringent conditions with a probe that can be prepared from any of the nucleotide sequences exemplified above (such as the nucleotide sequence shown as SEQ ID NO: 29 for DHCR24 gene, the nucleotide sequence shown as SEQ ID NO: 18 for DHCR7 gene, and the nucleotide sequence shown as SEQ ID NO: 7 or 55 for SMT1 gene), such as a complementary sequence of a part or the whole of any of the aforementioned nucleotide sequences, so long as the original function thereof is maintained. The term "stringent conditions" refers to conditions under which a so-called specific hybrid is formed, and a non-specific hybrid is not formed. Examples of the stringent conditions include those under which highly homologous DNAs hybridize to each other, for example, DNAs not less than 50%, 65%, or 80% homologous, preferably not less than 90% homologous, more preferably not less than 95% homologous, still more preferably not less than 97% homologous, particularly preferably not less than 99% homologous, hybridize to each other, and DNAs less homologous than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

As described above, the probe to be used for hybridization may be a part of a complementary sequence of a gene. Such a probe can be prepared by PCR using oligonucleotides prepared on the basis of the nucleotide sequences of known genes as primers and a DNA fragment containing the aforementioned gene as a template. As the probe, for example, a DNA fragment having a length of about 300 bp can be used. In a case of using a DNA fragment having a length of about 300 bp as the probe, the washing conditions of the hybridization may be, for example, 50°C, 2 x SSC and 0.1% SDS.

Furthermore, DHCR24, DHCR7, or SMT1 gene may be a gene in which any codon(s) is/are replaced with respective equivalent codon(s). That is, DHCR24, DHCR7, or SMT1 gene may be a variant of any of the DHCR24, DHCR7, or SMT1 genes exemplified above due to the degeneracy of the genetic code.

The percentage of the sequence identity between two sequences can be determined by, for example, using a mathematical algorithm. Non-limiting examples of such a mathematical algorithm include the algorithm of Myers and Miller (1988) CABIOS 4:11-17, the local homology algorithm of Smith et al (1981) Adv. Appl. Math. 2:482, the homology alignment algorithm of Needleman and Wunsch (1970) J. Mol. Biol. 48:443-453, the method for searching homology of Pearson and Lipman (1988) Proc. Natl. Acad. Sci. 85:2444-2448, and an modified version of the algorithm of Karlin and Altschul (1990) Proc. Natl. Acad. Sci. USA 87:2264, such as that described in Karlin and Altschul (1993) Proc. Natl. Acad. Sci. USA 90:5873-5877.

By using a program based on such a mathematical algorithm, sequence comparison (i.e. alignment) for determining the sequence identity can be performed. The program can be appropriately executed by a computer. Examples of such a program include, but not limited to, CLUSTAL of PC/Gene program (available from Intelligenetics, Mountain View, Calif.), ALIGN program (Version 2.0), and GAP, BESTFIT, BLAST, FASTA, and TFASTA of Wisconsin Genetics Software Package, Version 8 (available from Genetics Computer Group (GCG), 575 Science Drive, Madison, Wis., USA). Alignment using these programs can be performed by using, for example, initial parameters. The CLUSTAL program is well described in Higgins et al. (1988) Gene 73:237-244, Higgins et al. (1989) CABIOS 5:151-153, Corpet et al. (1988) Nucleic Acids Res. 16:10881-90, Huang et al. (1992) CABIOS 8:155-65, and Pearson et al. (1994) Meth. Mol. Biol. 24:307-331.

In order to obtain a nucleotide sequence homologous to a target nucleotide sequence, in particular, for example, BLAST nucleotide search can be performed by using BLASTN program with score of 100 and word length of 12. In order to obtain an amino acid sequence homologous to a target protein, in particular, for example, BLAST protein search can be performed by using BLASTX program with score of 50 and word length of 3. See http://www.ncbi.nlm.nih.gov for BLAST nucleotide search and BLAST protein search. In addition, Gapped BLAST (BLAST 2.0) can be used in order to obtain an alignment including gap(s) for the purpose of comparison. In addition, PSI-BLAST (BLAST 2.0) can be used in order to perform repetitive search for detecting distant relationships between sequences. See Altschul et al. (1997) Nucleic Acids Res. 25:3389 for Gapped BLAST and PSI-BLAST. When using BLAST, Gapped BLAST, or PSI-BLAST, initial parameters of each program (e.g. BLASTN for nucleotide sequences, and BLASTX for amino acid sequences) can be used. Alignment can also be manually performed.

The sequence identity between two sequences is calculated as the ratio of residues matching in the two sequences when aligning the two sequences so as to fit maximally with each other. The term "identity" between amino acid sequences may mean an identity calculated by blastp with default scoring parameters (i.e. Matrix, BLOSUM62; Gap Costs, Existence = 11, Extension = 1; Compositional Adjustments, Conditional compositional score matrix adjustment), unless otherwise stated. The term "identity" between nucleotide sequences may mean an identity calculated by blastn with default scoring parameters (i.e. Match/Mismatch Scores = 1, -2; Gap Costs = Linear), unless otherwise stated.

The aforementioned descriptions concerning variants of the genes and proteins can also be applied *mutatis mutandis* to any proteins and genes encoding them.

### <1-4> Other modifications

The microorganism of the present invention may have another modification, so long as the sterol-producing ability is not spoiled. Examples of the other modification include modification for improving the sterol-producing ability. Specific examples of the other modification include a reduction in the activity of sterol C-22 desaturase, an increase in the activity of DHCR24, and an increase in the activity of DHCR7. The sterol-producing ability may be enhanced by these modifications. These modifications can be appropriately selected according to various conditions such as the type of sterol to be produced. These modifications can be independently used, or can be used in an appropriate combination. That is, the microorganism of the present invention may have one or more modifications selected from these modifications.

These modifications each can be used, for example, in combination with a reduction in the activity of SMT1. That is, for example, the microorganism of the present invention may have been modified so that the activity of SMT1 is reduced, and may further have one or more modifications selected from these modifications. However, when the microorganism of the present invention has been modified so that the activity of SMT1 and DHCR7 is reduced, an increase in the activity of DHCR7 shall not be selected as the other modification.

Thus, the microorganism of the present invention may have been modified so that the activity of sterol C-22 desaturase is reduced. The microorganism of the present invention may have been modified, specifically, so that the activity of sterol C-22 desaturase is reduced as compared with a non-modified strain. A reduction in the activity of sterol C-22 desaturase may provide, for example, an increased production of an animal sterol such as cholesterol. A reduction in the activity of sterol C-22 desaturase may provide, specifically, for example, a reduction in by-production of a C22 unsaturated sterol (a sterol having a double bond at position C22), to thereby provide an increased production of an animal sterol.

Also, the microorganism of the present invention may have been modified so that the activity of DHCR24 is increased. The microorganism of the present invention may have been modified, specifically, so that the activity of DHCR24 is increased as compared with a non-modified strain. An increase in the activity of DHCR24 may provide, for example, an increased production of a part of animal sterols such as cholesterol, lathosterol, and 7-dehydrocholesterol.

Also, the microorganism of the present invention may have been modified so that the activity of DHCR7 is increased. The microorganism of the present invention may have been modified, specifically, so that the activity of DHCR7 is increased as compared with a non-modified strain. An increase in the activity of DHCR7 may provide, for example, an increased production of cholesterol.

The phrase "sterol C-22 desaturase" refers to a protein (enzyme) having an activity of catalyzing a reaction of introducing a double bond at position C22 (a C22-C23 double bond) of a sterol (EC 1.14.19.41). This activity is also referred to as "sterol C-22 desaturase activity". A gene encoding sterol C-22 desaturase is also referred to as "sterol C-22 desaturase gene". The phrase "position C22" referred to herein refers to a carbon corresponding to position C22 of zymosterol.

The sterol C-22 desaturase activity can be measured by, for example, incubating an enzyme with a substrate (a sterol not having a double bond at position C22) in the presence of an electron donor and oxygen, and measuring the enzyme- and substrate-dependent generation of a product (a sterol introduced with a double bond at position C22). Examples of the electron donor include, for example, NADPH. Examples of the substrate and product include, for example, ergosta-5,7,24(28)-trien-3-beta-ol and ergosta-5,7,22,24(28)-tetraen-3-beta-ol, respectively. The sterol C-22 desaturase activity can be measured, specifically, for example, according to the procedure of a previous report (Morikawa T et al. Cytochrome P450 CYP710A encodes the sterol C-22 desaturase in Arabidopsis and tomato. Plant Cell. 2006 Apr;18(4): 1008-22.).

The nucleotide sequences and the amino acid sequences of the sterol C-22 desaturase genes and the sterol C-22 desaturases possessed by Labyrinthulea microorganisms can be obtained from, for example, public databases such as NCBI. The nucleotide sequence of the sterol C-22 desaturase gene (cyp710a gene) of the Aurantiochytrium sp.1 strain AJ7869 and the amino acid sequence of the sterol C-22 desaturase (CYP710A) encoded by the gene are shown in SEQ ID NOS: 41 and 42, respectively. That is, the sterol C-22 desaturase gene may be, for example, a gene having the nucleotide sequence shown as SEQ ID NO: 41. Also, the sterol C-22 desaturase may be, for example, a protein having the amino acid sequence shown as SEQ ID NO: 42.

The sterol C-22 desaturase gene and sterol C-22 desaturase may also be conservative variants of the sterol C-22 desaturase genes and sterol C-22 desaturases exemplified above (such as conservative variants of a gene having the nucleotide sequence shown as SEQ ID NO: 41 and a protein having the amino acid sequence shown as SEQ ID NO: 42), respectively. The descriptions concerning conservative variants of SMT1 gene and SMT1 etc. can be applied *mutatis mutandis* to conservative variants of the sterol C-22 desaturase gene and sterol C-22 desaturase. The expression "the original function is maintained" used for the sterol C-22 desaturase means that a variant of the protein has the sterol C-22 desaturase activity. The sterol C-22 desaturase gene may be, for example, a gene encoding a protein having any of the amino acid sequences exemplified above, but which includes substitution, deletion, insertion, and/or addition of one or several amino acid residues at one or several positions, so long as the original function thereof is maintained. The sterol C-22 desaturase gene may also be, for example, a gene encoding a protein having an amino acid sequence showing a homology of, for example, 50% or more, 65% or more, 80% or more, preferably 90% or more, more preferably 95% or more, still more preferably 97% or more, particularly preferably 99% or more, to the total amino acid sequence of any of the amino acid sequences exemplified above, so long as the original function thereof is maintained.

DHCR24 and DHCR7 are as described above. DHCR24, DHCR7, and SMT1 of which the activity is increased are not limited to those possessed by a Labyrinthulea microorganism to be modified, and any DHCR24, DHCR7, and SMT1 can be used.

### <1-5> Method for reducing activity of protein

Hereinafter, the methods for reducing the activity of a protein such as DHCR24, DHCR7, SMT1, and sterol C-22 desaturase will be explained.

The expression "the activity of a protein is reduced" means that the activity of the protein is reduced as compared with a non-modified strain. Specifically, the expression "the activity of a protein is reduced" means that the activity of the protein per cell is reduced as compared with that of a non-modified strain. The term "non-modified strain" used herein refers to a control strain that has not been modified so that the activity of an objective protein is reduced. Examples of the non-modified strain include a wild-type strain and parent strain. Specific examples of the non-modified strain include strains exemplified above in relation to the description of Labyrinthulea microorganisms. That is, in an embodiment, the activity of a protein may be reduced as compared with the Aurantiochytrium sp.1 strain AJ7869. In another embodiment, the activity of a protein may also be reduced as compared with the Aurantiochytrium sp.1 strain AJ7868. In another embodiment, the activity of a protein may also be reduced as compared with the Aurantiochytrium sp.1 strain AJ7879. The state that "the activity of a protein is reduced" also includes a state that the activity of the protein has completely disappeared. More specifically, the expression "the activity of a protein is reduced" may mean that the number of molecules of the protein per cell is reduced, and/or the function of each molecule of the protein is reduced as compared with those of a non-modified strain. That is, the term "activity" in the expression "the activity of a protein is reduced" is not limited to the catalytic activity of the protein, but may also mean the transcription amount of a gene (i.e. the amount of mRNA) encoding the protein or the translation amount of the gene (i.e. the amount of the protein). The state that "the number of molecules of the protein per cell is reduced" also includes a state that the protein does not exist at all. The state that "the function of each molecule of the protein is reduced" also includes a state that the function of each protein molecule has completely disappeared. The degree of the reduction in the activity of a protein is not particularly limited, so long as the activity is reduced as compared with that of a non-modified strain. The activity of a protein may be reduced to, for example, 50% or less, 20% or less, 10% or less, 5% or less, or 0% of that of a non-modified strain.

The modification for reducing the activity of a protein can be attained by, for example, reducing the expression of a gene encoding the protein. The expression "the expression of a gene is reduced" means that the expression of the gene is reduced as compared with a non-modified strain. Specifically, the expression "the expression of a gene is reduced" means that the expression of the gene per cell is reduced as compared with that of a non-modified strain. More specifically, the expression "the expression of a gene is reduced" may mean that the transcription amount of the gene (i.e. the amount of mRNA) is reduced, and/or the translation amount of the gene (i.e. the amount of the protein expressed from the gene) is reduced. The state that "the expression of a gene is reduced" also includes a state that the gene is not expressed at all. The state that "the expression of a gene is reduced" is also referred to as "the expression of a gene is attenuated". The expression of a gene may be reduced to, for example, 50% or less, 20% or less, 10% or less, 5% or less, or 0% of that of a non-modified strain.

The reduction in gene expression may be due to, for example, a reduction in the transcription efficiency, a reduction in the translation efficiency, or a combination of them. The expression of a gene can be reduced by, for example, modifying an expression control sequence of the gene. The term "expression control sequence" collectively refers to sites that affect the expression of a gene, such as promoter. Expression control sequences can be identified by, for example, using a promoter search vector or gene analysis software such as GENETYX. When an expression control sequence is modified, preferably one or more nucleotides, more preferably two or more nucleotides, particularly preferably three or more nucleotides, of the expression control sequence are modified. The transcription efficiency of a gene can be reduced by, for example, replacing the promoter of the gene on a chromosome with a weaker promoter. The term "weaker promoter" means a promoter providing an attenuated transcription of a gene compared with an inherently existing wild-type promoter of the gene. Examples of weaker promoters include, for example, inducible promoters. That is, an inducible promoter may function as a weaker promoter under a non-induced condition, such as in the absence of the corresponding inducer. Furthermore, a partial or entire region of an expression control sequence may be deleted. The expression of a gene can also be reduced by, for example, manipulating a factor responsible for expression control. Examples of the factor responsible for expression control include low molecules responsible for transcription or translation control (inducers, inhibitors, etc.), proteins responsible for transcription or translation control (transcription factors etc.), nucleic acids responsible for transcription or translation control (siRNA etc.), and so forth. Furthermore, the expression of a gene can also be reduced by, for example, introducing a mutation that reduces the expression of the gene into the coding region of the gene. For example, the expression of a gene can be reduced by replacing a codon in the coding region of the gene with a synonymous codon used less frequently in a host. Furthermore, for example, the gene expression may be reduced due to disruption of a gene as described later.

The modification for reducing the activity of a protein can also be attained by, for example, disrupting a gene encoding the protein. The expression "a gene is disrupted" means that a gene is modified so that a protein that can normally function is not produced. The state that "a protein that normally functions is not produced" includes a state that the protein is not produced at all from the gene, and a state that the protein of which the function (activity or property) per molecule is reduced or eliminated is produced from the gene.

Disruption of a gene can be attained by, for example, deleting the gene on a chromosome. The term "deletion of a gene" refers to deletion of a partial or entire region of the coding region of the gene. Furthermore, the whole of a gene including sequences upstream and downstream from the coding region of the gene on a chromosome may be deleted. Sequences upstream and downstream from the coding region of a gene may contain, for example, an expression control sequence of the gene. The region to be deleted may be any region such as an N-terminal region (region encoding an N-terminal region of a protein), an internal region, or a C-terminal region (region encoding a C-terminal region of a protein), so long as the activity of the protein can be reduced. Deletion of a longer region can usually more surely inactivate the gene. The region to be deleted may be, for example, a region having a length of 10% or more, 20% or more, 30% or more, 40% or more, 50% or more, 60% or more, 70% or more, 80% or more, 90% or more, or 95% or more of the total length of the coding region of the gene. Furthermore, it is preferred that reading frames of the sequences upstream and downstream from the region to be deleted are not the same. Inconsistency of reading frames may cause a frameshift downstream of the region to be deleted.

Disruption of a gene can also be attained by, for example, introducing an amino acid substitution (missense mutation), a stop codon (nonsense mutation), addition or deletion of one or two nucleotide residues (frame shift mutation), or the like into the coding region of the gene on a chromosome (Journal of Biological Chemistry, 272:8611-8617 (1997); Proceedings of the National Academy of Sciences, USA, 95 5511-5515 (1998); Journal of Biological Chemistry, 26 116, 20833-20839 (1991)).

Disruption of a gene can also be attained by, for example, inserting another nucleotide sequence into a coding region of the gene on a chromosome. Site of the insertion may be in any region of the gene, and insertion of a longer nucleotide sequence can usually more surely inactivate the gene. It is preferred that reading frames of the sequences upstream and downstream from the insertion site are not the same. Inconsistency of reading frames may cause a frameshift downstream of the insertion site. The other nucleotide sequence is not particularly limited so long as a sequence that reduces or eliminates the activity of the encoded protein is chosen, and examples thereof include, for example, a marker gene such as antibiotic resistance genes, and a gene useful for production of an objective substance.

Particularly, disruption of a gene may be carried out so that the amino acid sequence of the encoded protein is deleted. In other words, the modification for reducing the activity of a protein can be attained by, for example, deleting the amino acid sequence of the protein, specifically, modifying a gene so as to encode a protein of which the amino acid sequence is deleted. The term "deletion of the amino acid sequence of a protein" refers to deletion of a partial or entire region of the amino acid sequence of the protein. In addition, the term "deletion of the amino acid sequence of a protein" means that the original amino acid sequence disappears in the protein, and also includes cases where the original amino acid sequence is changed to another amino acid sequence. That is, for example, a region that was changed to another amino acid sequence by frameshift may be regarded as a deleted region. When the amino acid sequence of a protein is deleted, the total length of the protein is typically shortened, but there can also be cases where the total length of the protein is not changed or is extended. For example, by deletion of a partial or entire region of the coding region of a gene, a region encoded by the deleted region can be deleted in the encoded protein. In addition, for example, by introduction of a stop codon into the coding region of a gene, a region encoded by the downstream region of the introduction site can be deleted in the encoded protein. In addition, for example, by frameshift in the coding region of a gene, a region encoded by the frameshift region can be deleted in the encoded protein. The aforementioned descriptions concerning the position and length of the region to be deleted in deletion of a gene can be applied *mutatis mutandis* to the position and length of the region to be deleted in deletion of the amino acid sequence of a protein.

Such modification of a gene on a chromosome as described above can be attained by, for example, preparing a disruption-type gene modified so that it is unable to produce a protein that normally functions, and transforming a host with a recombinant DNA containing the disruption-type gene to cause homologous recombination between the disruption-type gene and the wild-type gene on a chromosome and thereby substitute the disruption-type gene for the wild-type gene on the chromosome. In this procedure, if a marker gene selected according to the characteristics of the host such as auxotrophy is included in the recombinant DNA, the operation becomes easier. Examples of the disruption-type gene include a gene of which a partial or entire region of the coding region is deleted, gene including a missense mutation, gene including a nonsense mutation, gene including a frame shift mutation, and gene introduced with an insertion sequence such as a transposon or marker gene. The protein encoded by the disruption-type gene has a conformation different from that of the wild-type protein, even if it is produced, and thus the function thereof is reduced or eliminated. The structure of the recombinant DNA to be used for homologous recombination is not particularly limited as long as it causes homologous recombination in a desired manner. For example, a host can be transformed with a linear DNA containing a disruption-type gene and further containing upstream and downstream sequences of a wild-type gene on the chromosome at the respective ends, so that homologous recombination occurs at each of upstream and downstream sides of the wild-type gene, to thereby replace the wild-type gene with the disruption-type gene. Such methods for modifying a chromosome using homologous recombination can be used for any modification on a chromosome, such as a modification of an expression control sequence, as well as for disruption of an objective gene.

When the host has two or more copies of a gene encoding a target protein, all of those two or more copies of the gene may be modified (e.g. disrupted), or only a part of those two or more copies of the gene may be modified (e.g. disrupted), so long as the activity of the protein is reduced to a desired extent.

Modification for reducing activity of a protein can also be attained by, for example, a mutagenesis treatment. Examples of the mutagenesis treatment include irradiation of X-ray or ultraviolet and treatment with a mutation agent such as N-methyl-N'-nitro-N-nitrosoguanidine (MNNG), ethyl methanesulfonate (EMS), and methyl methanesulfonate (MMS).

Such methods for reducing the activity of a protein as mentioned above may be used independently or in any appropriate combination.

A reduction in the activity of a protein can be confirmed by measuring the activity of the protein.

A reduction in the activity of a protein can also be confirmed by confirming a reduction in the expression of a gene encoding the protein. A reduction in the expression of a gene can be confirmed by confirming a reduction in the transcription amount of the gene or a reduction in the amount of the protein expressed from the gene.

A reduction in the transcription amount of a gene can be confirmed by comparing the amount of mRNA transcribed from the gene with that of a non-modified strain. Examples of the method for evaluating the amount of mRNA include Northern hybridization, RT-PCR, microarray, RNA-seq, and so forth (Molecular Cloning (Cold Spring Harbor Laboratory Press, Cold Spring Harbor (USA), 2001)). The amount of mRNA (such as the number of molecules of the mRNA per cell) may be reduced to, for example, 50% or less, 20% or less, 10% or less, 5% or less, or 0% of that of a non-modified strain.

A reduction in the amount of a protein can be confirmed by carrying out SDS-PAGE and confirming the intensity of separated protein bands. A reduction in the amount of a protein can also be confirmed by Western blotting using antibodies (Molecular Cloning (Cold Spring Harbor Laboratory Press, Cold Spring Harbor (USA), 2001)). The amount of the protein (such as the number of molecules of the protein per cell) may be reduced to, for example, 50% or less, 20% or less, 10% or less, 5% or less, or 0% of that of a non-modified strain.

Disruption of a gene can be confirmed by determining nucleotide sequence of a part or the whole of the gene, restriction enzyme map, full length, or the like of the gene depending on the means used for the disruption.

Transformation can be carried out by, for example, a method generally used for transformation of Labyrinthulea microorganisms. Examples of such a method include electroporation.

The aforementioned methods for reducing the activity of a protein as mentioned above can be applied to reduction in the activities of any proteins and reduction in the expression of any genes.

### <1-6> Methods for increasing activity of protein

Hereinafter, the methods for increasing the activity of a protein such as DHCR24 and DHCR7 will be explained.

The expression "the activity of a protein is increased" means that the activity of the protein is increased as compared with a non-modified strain. Specifically, the expression "the activity of a protein is increased" means that the activity of the protein per cell is increased as compared with that of a non-modified strain. The term "non-modified strain" used herein refers to a control strain that has not been modified so that the activity of an objective protein is increased. Examples of the non-modified strain include a wild-type strain and parent strain. Specific examples of the non-modified strain include strains exemplified above in relation to the description of Labyrinthulea microorganisms. That is, in an embodiment, the activity of a protein may be increased as compared with the Aurantiochytrium sp.1 strain AJ7869. In another embodiment, the activity of a protein may also be increased as compared with the Aurantiochytrium sp.1 strain AJ7868. In another embodiment, the activity of a protein may also be increased as compared with the Aurantiochytrium sp.1 strain AJ7879. The state that "the activity of a protein is increased" may also be expressed as "the activity of a protein is enhanced". More specifically, the expression "the activity of a protein is increased" may mean that the number of molecules of the protein per cell is increased, and/or the function of each molecule of the protein is increased as compared with those of a non-modified strain. That is, the term "activity" in the expression "the activity of a protein is increased" is not limited to the catalytic activity of the protein, but may also mean the transcription amount of a gene (i.e. the amount of mRNA) encoding the protein, or the translation amount of the gene (i.e. the amount of the protein). Furthermore, the state that "the activity of a protein is increased" includes not only a state that the activity of an objective protein is increased in a strain inherently having the activity of the objective protein, but also a state that the activity of an objective protein is imparted to a strain not inherently having the activity of the objective protein. Furthermore, so long as the activity of the protein is eventually increased, the activity of an objective protein inherently contained in a host may be attenuated and/or eliminated, and then an appropriate type of the objective protein may be imparted to the host.

The degree of the increase in the activity of a protein is not particularly limited, so long as the activity of the protein is increased as compared with a non-modified strain. The activity of the protein may be increased to, for example, 1.5 times or more, 2 times or more, or 3 times or more of that of a non-modified strain. Furthermore, when the non-modified strain does not have the activity of the objective protein, it is sufficient that the protein is produced as a result of introduction of the gene encoding the protein, and for example, the protein may be produced to such an extent that the activity thereof can be measured.

The modification for increasing the activity of a protein can be attained by, for example, increasing the expression of a gene encoding the protein. The expression "the expression of a gene is increased" means that the expression of the gene is increased as compared with a non-modified strain such as a wild-type strain and parent strain. Specifically, the expression "the expression of a gene is increased" means that the expression amount of the gene per cell is increased as compared with that of a non-modified strain. More specifically, the expression "the expression of a gene is increased" may mean that the transcription amount of the gene (i.e. the amount of mRNA) is increased, and/or the translation amount of the gene (i.e. the amount of the protein expressed from the gene) is increased. The state that "the expression of a gene is increased" may also be referred to as "the expression of a gene is enhanced". The expression of a gene may be increased to, for example, 1.5 times or more, 2 times or more, or 3 times or more of that of a non-modified strain. Furthermore, the state that "the expression of a gene is increased" includes not only a state that the expression amount of an objective gene is increased in a strain that inherently expresses the objective gene, but also a state that the gene is introduced into a strain that does not inherently express the objective gene, and expressed therein. That is, the phrase "the expression of a gene is increased" may also mean, for example, that an objective gene is introduced into a strain that does not possess the gene, and is expressed therein.

The expression of a gene can be increased by, for example, increasing the copy number of the gene.

The copy number of a gene can be increased by introducing the gene into the chromosome of a host. A gene can be introduced into a chromosome by, for example, using homologous recombination. Specifically, a target gene can be introduced into the chromosome of a host by transforming the host with a recombinant DNA containing the gene to thereby induce homologous recombination between the recombinant DNA and a target region of the chromosome of the host. The structure of the recombinant DNA to be used for homologous recombination is not particularly limited as long as it causes homologous recombination in a desired manner. For example, a host can be transformed with a linear DNA containing a target gene and further containing nucleotide sequence homologous to upstream and downstream sequences of a target region on the chromosome at the respective ends, so that homologous recombination occurs at each of upstream and downstream sides of the target region, to thereby replace the target region with the target gene. The recombinant DNA to be used for homologous recombination may contain a marker gene for selection of transformants. Examples of the marker gene include antibiotic resistance genes such as hygromycin resistance gene (HygR), neomycin resistance gene (NeoR), and blasticidin resistance gene (BlaR). Only one copy, or two or more copies of a gene may be introduced. For example, by performing homologous recombination using a nucleotide sequence which is present in multiple copies on a chromosome as a target, multiple copies of a gene can be introduced into the chromosome. Such methods for modifying a chromosome using homologous recombination can be used for any modification on a chromosome, such as a modification of an expression control sequence, as well as for introduction of an objective gene.

Introduction of an objective gene into a chromosome can be confirmed by Southern hybridization using a probe having a sequence complementary to the whole gene or a part thereof, PCR using primers prepared on the basis of the sequence of the gene, or the like.

Furthermore, the copy number of a gene can also be increased by introducing a vector containing the gene into a host. For example, the copy number of an objective gene can be increased by ligating a DNA fragment containing the objective gene with a vector that functions in a host to construct an expression vector of the gene, and transforming the host with the expression vector. The DNA fragment containing the objective gene can be obtained by, for example, PCR using the genomic DNA of a microorganism having the objective gene as the template. As the vector, a vector autonomously replicable in the cell of the host can be used. The vector may be a single-copy vector or a multi-copy vector. The vector may contain a marker gene for selection of transformants.

When a gene is introduced, it is sufficient that the gene is expressibly harbored by a host. Specifically, it is sufficient that the gene is harbored by a host so that it is expressed under control by a promoter that functions in the host. The promoter is not particularly limited so long as it functions in the host. The term "promoter that functions in a host" refers to a promoter that shows a promoter activity in the host. The promoter may be a promoter derived from the host, or a heterogenous promoter. The promoter may be the native promoter of the gene to be introduced, or a promoter of another gene. Specific examples of the promoter include actin gene promoter, glyceraldehyde-3-phosphate dehydrogenase gene promoter, pyruvate kinase gene promoter, elongation factor 1α (EF1α) gene promoter, ubiquitin promoter (UbiP), Simian Virus 40 promoter (SV40P), tubulin gene promoter, and virus immediate-early gene promoter (Smp1P) (Japanese Patent Laid-open (Kokai) No. 2018-064551, Japanese Patent Laid-open (Kokai) No. 2006-304686, WO2016/056610, and WO02/083869). As the promoter, for example, such a stronger promoter as mentioned later may also be used.

A terminator for termination of gene transcription may be located downstream of the gene. The terminator is not particularly limited so long as it functions in the host. The terminator may be a terminator derived from the host, or a heterogenous terminator. The terminator may be the native terminator of the gene to be introduced, or a terminator of another gene. Specific examples of the terminator include actin gene terminator, glyceraldehyde-3-phosphate dehydrogenase gene terminator, pyruvate kinase gene terminator, elongation factor 1α (EF1α) gene terminator, ubiquitin terminator (UbiT), and Simian Virus 40 terminator (SV40T) (Japanese Patent Laid-open (Kokai) No. 2018-064551, Japanese Patent Laid-open (Kokai) No. 2006-304686, and WO2016/056610).

Vectors, promoters, and terminators available in various microorganisms are disclosed in detail in "Fundamental Microbiology Vol. 8, Genetic Engineering, KYORITSU SHUPPAN CO., LTD, 1987", and those can be used.

Furthermore, when two or more of genes are introduced, it is sufficient that the genes each are expressibly harbored by the host. For example, all the genes may be carried by a single expression vector or a chromosome. Furthermore, the genes may be separately carried by two or more expression vectors, or separately carried by a single or two or more expression vectors and a chromosome. An operon constituted by two or more genes may also be introduced.

The gene to be introduced is not particularly limited so long as it encodes a protein that functions in the host. The gene to be introduced may be a gene derived from the host, or may be a heterogenous gene. The gene to be introduced can be obtained by, for example, PCR using primers designed on the basis of the nucleotide sequence of the gene, and using the genomic DNA of an organism having the gene, a plasmid carrying the gene, or the like as a template. The gene to be introduced may also be totally synthesized, for example, on the basis of the nucleotide sequence of the gene (Gene, 60(1), 115-127 (1987)). The obtained gene can be used as it is, or after being modified as required. That is, a gene can be modified to obtain a variant thereof. A gene can be modified by a known technique. For example, an objective mutation can be introduced into an objective site of DNA by the site-specific mutation method. That is, the coding region of a gene can be modified by the site-specific mutation method so that a specific site of the encoded protein include substitution, deletion, insertion, and/or addition of amino acid residues. Examples of the site-specific mutation method include the method utilizing PCR (Higuchi, R., 61, in PCR Technology, Erlich, H.A. Eds., Stockton Press (1989); Carter, P., Meth. in Enzymol., 154, 382 (1987)), and the method utilizing phage (Kramer, W. and Frits, H.J., Meth. in Enzymol., 154, 350 (1987); Kunkel, T.A. et al., Meth. in Enzymol., 154, 367 (1987)). Alternatively, a variant of a gene may be totally synthesized.

Furthermore, the expression of a gene can be increased by improving the transcription efficiency of the gene. In addition, the expression of a gene can also be increased by improving the translation efficiency of the gene. The transcription efficiency of the gene and the translation efficiency of the gene can be improved by, for example, modifying an expression control sequence of the gene. The term "expression control sequence" collectively refers to sites that affect the expression of a gene, such as promoter. Expression control sequences can be identified by using a promoter search vector or gene analysis software such as GENETYX.

The transcription efficiency of a gene can be improved by, for example, replacing the promoter of the gene on a chromosome with a stronger promoter. The term "stronger promoter" refers to a promoter providing an improved transcription of a gene compared with an inherently existing wild-type promoter of the gene. As the stronger promoter, for example, a natural high expression promoter can be used. Furthermore, as the stronger promoter, for example, a highly-active type of an existing promoter may also be obtained and use. A highly-active type of an existing promoter may be obtained by, for example, using various reporter genes.

The translation efficiency of a gene can also be improved by, for example, modifying codons. For example, the translation efficiency of the gene can be improved by replacing a rare codon present in the gene with a synonymous codon more frequently used. That is, the gene to be introduced may be modified, for example, so as to contain optimal codons according to the frequencies of codons observed in a host to be used. Codons can be replaced by, for example, the site-specific mutation method. Alternatively, a gene fragment in which objective codons are replaced may be totally synthesized. Frequencies of codons in various organisms are disclosed in the "Codon Usage Database" (http://www.kazusa.or.jp/codon; Nakamura, Y. et al, Nucl. Acids Res., 28, 292 (2000)).

Furthermore, the expression of a gene can also be increased by amplifying a regulator that increases the expression of the gene, or deleting or attenuating a regulator that reduces the expression of the gene.

Such methods for increasing the gene expression as mentioned above may be used independently or in any appropriate combination.

Furthermore, the modification that increases the activity of a protein can also be attained by, for example, enhancing the specific activity of the protein. A protein showing an enhanced specific activity can be obtained by, for example, searching various organisms. Furthermore, a highly-active type of an existing protein may also be obtained by introducing a mutation into the existing protein. The mutation to be introduced may be, for example, substitution, deletion, insertion, or addition of one or several amino acid residues at one or several position of the protein. The mutation can be introduced by, for example, such a site-specific mutation method as mentioned above. The mutation may also be introduced by, for example, a mutagenesis treatment. Examples of the mutagenesis treatment include irradiation of X-ray, irradiation of ultraviolet, and a treatment with a mutation agent such as N-methyl-N'-nitro-N-nitrosoguanidine (MNNG), ethyl methanesulfonate (EMS), and methyl methanesulfonate (MMS). Furthermore, a random mutation may be induced by directly treating DNA in vitro with hydroxylamine. Enhancement of the specific activity may be independently used, or may be used in any appropriate combination with such methods for enhancing gene expression as mentioned above.

Transformation can be carried out by, for example, a method generally used for transformation of Labyrinthulea microorganisms. Examples of such a method include electroporation.

An increase in the activity of a protein can be confirmed by measuring the activity of the protein.

An increase in the activity of a protein can also be confirmed by confirming an increase in the expression of a gene encoding the protein. An increase in the expression of a gene can be confirmed by confirming an increase in the transcription amount of the gene, or by confirming an increase in the amount of a protein expressed from the gene.

An increase of the transcription amount of a gene can be confirmed by comparing the amount of mRNA transcribed from the gene with that of a non-modified strain such as a wild-type strain or parent strain. Examples of the method for evaluating the amount of mRNA include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA (such as the number of molecules of the mRNA per cell) may be increased to, for example, 1.5 times or more, 2 times or more, or 3 times or more of that of a non-modified strain.

An increase in the amount of a protein can be confirmed by Western blotting using antibodies (Molecular Cloning (Cold Spring Harbor Laboratory Press, Cold Spring Harbor (USA), 2001)). The amount of the protein (such as the number of molecules of the protein per cell) may be increased to, for example, 1.5 times or more, 2 times or more, or 3 times or more of that of a non-modified strain.

The aforementioned methods for increasing the activity of a protein can be used for enhancement of the activities of any proteins and enhancement of the expression of any genes.

### <2> Method for producing sterol

The method of the present invention is a method for producing a sterol comprising culturing the microorganism of the present invention in a culture medium, and collecting the sterol from cells generated by the culturing. In the present invention, a single kind of sterol may be produced, or two or more kinds of sterols may be produced. Examples of the modification possessed by the microorganism of the present invention and the sterol to be produced are as described above.

The culture medium to be used is not particularly limited, so long as the microorganism of the present invention can proliferate in it, and a sterol can be produced. As the culture medium, for example, a usual culture medium used for culturing heterotrophic microorganisms such as Labyrinthulea microorganisms can be used. The culture medium may contain carbon source, nitrogen source, phosphorus source, and sulfur source, as well as components selected from other various organic components and inorganic components as required. Specific examples of the culture medium include GY medium prepared with 0 to 1 × artificial seawater.

Specific examples of the carbon source include, for example, saccharides such as glucose, fructose, sucrose, lactose, galactose, xylose, arabinose, blackstrap molasses, hydrolysates of starches, and hydrolysates of biomass, organic acids such as acetic acid and citric acid, alcohols such as ethanol, glycerol, and crude glycerol, and aliphatic acids. As the carbon source, a single kind of carbon source may be used, or two or more kinds of carbon sources may be used in combination.

Specific examples of the nitrogen source include, for example, ammonium salts such as ammonium sulfate, ammonium chloride, and ammonium phosphate, organic nitrogen sources such as peptone, yeast extract, meat extract, and soybean protein decomposition products, ammonia, and urea. Ammonia gas or aqueous ammonia used for adjusting pH may also be used as the nitrogen source. As the nitrogen source, a single kind of nitrogen source may be used, or two or more kinds of nitrogen sources may be used in combination.

Specific examples of the phosphate source include, for example, phosphoric acid salts such as potassium dihydrogenphosphate and dipotassium hydrogenphosphate, and phosphoric acid polymers such as pyrophosphoric acid. As the phosphate source, a single kind of phosphate source may be used, or two or more kinds of phosphate sources may be used in combination.

Specific examples of the sulfur source include, for example, inorganic sulfur compounds such as sulfates, thiosulfates, and sulfites, and sulfur-containing amino acids such as cysteine, cystine, and glutathione. As the sulfur source, a single kind of sulfur source may be used, or two or more kinds of sulfur sources may be used in combination.

Specific examples of other various organic components and inorganic components include, for example, inorganic salts such as sodium chloride and potassium chloride; trace metals such as iron, manganese, magnesium, and calcium; vitamins such as vitamin B1, vitamin B2, vitamin B6, nicotinic acid, nicotinamide, and vitamin B12; amino acids; nucleic acids; and organic components containing those such as peptone, casamino acid, yeast extract, and soybean protein decomposition product. As other various organic components and inorganic components, a single kind of component may be used, or two or more kinds of components may be used in combination.

The culture conditions are not particularly limited so long as the microorganism of the present invention can proliferate, and a sterol can be produced. The culture can be performed, for example, under usual conditions used for culturing heterotrophic microorganisms such as Labyrinthulea microorganisms.

The culture can be performed by using a liquid medium. At the time of the culture, the microorganism of the present invention cultured on a solid medium such as agar medium may be directly inoculated into a liquid medium, or the microorganism of the present invention cultured in a liquid medium as seed culture may be inoculated into a liquid medium for main culture. That is, the culture may be performed separately as seed culture and main culture. In such a case, the culture conditions of the seed culture and the main culture may be or may not be the same. The amount of the microorganism of the present invention contained in the culture medium at the time of the start of the culture is not particularly limited. The main culture may be performed by, for example, inoculating a seed culture broth to a culture medium for main culture at an amount of 1 to 50%(v/v).

The culture can be performed as batch culture, fed-batch culture, continuous culture, or a combination of these. The culture medium used at the time of the start of the culture is also referred to as "starting medium". The culture medium supplied to a culture system (fermentation tank) in fed-batch culture or continuous culture is also referred to as "feed medium". Furthermore, to supply a feed medium to a culture system in fed-batch culture or continuous culture is also referred to as to "feed". Furthermore, when the culture is performed separately as seed culture and main culture, for example, both the seed culture and the main culture may be performed as batch culture. Alternatively, for example, the seed culture may be performed as batch culture, and the main culture may be performed as fed-batch culture or continuous culture.

The culture can be performed, for example, under an aerobic condition. The term "aerobic condition" refers to a condition where the dissolved oxygen concentration in the liquid medium is not lower than 0.33 ppm, which is the detection limit for the detection with an oxygen membrane electrode, or may preferably to a condition where the dissolved oxygen concentration in the liquid medium is not lower than 1.5 ppm. The oxygen concentration can be controlled to, for example, 5 to 50%, preferably about 10%, of the saturated oxygen concentration. Specifically, the culture under an aerobic condition can be performed by aeration culture, shaking culture, stirring culture, or a combination thereof. The pH of the culture medium may be, for example, 3 to 10, preferably 4.0 to 9.5. During the culture, the pH of the culture medium can be adjusted as required. The pH of the culture medium can be adjusted by using various alkaline and acidic substances such as ammonia gas, aqueous ammonia, sodium carbonate, sodium bicarbonate, potassium carbonate, potassium bicarbonate, magnesium carbonate, sodium hydroxide, calcium hydroxide, magnesium hydroxide, hydrochloric acid, and sulfuric acid. The culture temperature may be, for example, 20 to 35°C, preferably 25 to 35°C. The culture period may be, for example, 10 to 120 hours. The culture may be continued, for example, until the carbon source contained in the culture medium is consumed, or until the microorganism of the present invention loses the activity. By culturing the microorganism of the present invention under such conditions as described above, cells containing a sterol are generated.

The sterol can be collected from the cells as required. That is, the sterol can be extracted from the cells and collected. The cells may be subject to extraction of the sterol while being present in a culture broth, or after being collected from the culture broth. The cells, such as a culture broth containing cells and cells collected from the culture broth, may also be subject to extraction of the sterol after being subject to a treatment such as dilution, concentration, freezing, thawing, and drying, as required. These treatments can be independently carried out, or can be carried out in an appropriate combination. These treatments can be appropriately selected according to various conditions such as the type of methods for extracting the sterol.

Methods for collecting cells from a culture broth are not particularly limited, and known methods can be used (Grima, E. M. et al. 2003. Biotechnol. Advances 20: 491-515). Specifically, for example, cells can be collected from a culture broth by a means such as natural sedimentation, centrifugation, and filtration. In addition, at this time, a flocculant may be used. The collected cells can be washed using an appropriate medium as required. The collected cells can also be re-suspended using an appropriate medium as required. Examples of media that can be used for washing or suspending include, for example, aqueous media (aqueous solvents) such as water and aqueous buffer, organic media (organic solvents) such as methanol, and mixtures thereof. The medium can be appropriately selected according to various conditions such as the type of methods for extracting the sterol.

Methods for extracting the sterol are not particularly limited, and known methods can be used. Examples of such methods include, for example, methods for extracting lipids from cells of microorganisms such as common algae. Specific examples of such methods include, for example, organic solvent treatment, ultrasonic treatment, bead crushing treatment, acid treatment, alkali treatment, enzyme treatment, hydrothermal treatment, supercritical treatment, microwave treatment, electromagnetic field treatment, and compression treatment. These treatments can be independently used, or can be used in an appropriate combination.

The organic solvent to be used for organic solvent treatment is not particularly limited, so long as the sterol can be extracted from cells using that organic solvent. Examples of the organic solvent include, for example, alcohols such as methanol, ethanol, 2-propanol, butanol, pentanol, hexanol, heptanol, and octanol; ketones such as acetone; ethers such as dimethyl ether and diethyl ether; esters such as methyl acetate and ethyl acetate; alkanes such as n-hexane; and chloroform. Examples of methods for extracting lipids using an organic solvent include Bligh-Dyer method and Folch method. As the organic solvent, a single kind of organic solvent may be used, or two or more kinds of organic solvents may be used in combination.

The pH of alkali treatment is not particularly limited, so long as the sterol can be extracted from cells at that pH. The pH of alkali treatment may be typically pH 8.5 or higher, preferably pH 10.5 or higher, more preferably pH 11.5 or higher, and may be pH 14 or lower. The temperature of alkali treatment may be typically 30°C or higher, preferably 50°C or higher, more preferably 70°C or higher. The temperature of alkali treatment may preferably be 120°C or lower. The time of alkali treatment may be typically 10 minutes or longer, preferably 30 minutes or longer, more preferably 50 minutes or longer. The period of alkali treatment may preferably be 150 minutes or shorter. For alkali treatment, alkaline substances such as NaOH and KOH can be used.

The extracted sterol can be collected by known methods used for separation and purification of compounds. Examples of such methods include, for example, ionexchange resin method and membrane separation method. These methods can be independently used, or can be used in an appropriate combination.

The collected sterol may contain such components as cells, culture medium components, moisture, components used for extraction treatment, and by-product metabolites of the microorganism of the present invention, in addition to the sterol. The collected sterol may be purified to a desired extent. The purity of the sterol may be, for example, 1%(w/w) or higher, 2%(w/w) or higher, 5%(w/w) or higher, 10%(w/w) or higher, 30%(w/w) or higher, 50%(w/w) or higher, 70%(w/w) or higher, 90%(w/w) or higher, or 95%(w/w) or higher.

The type and amount of sterol can be determined by known methods used for detection or quantification of compounds. Examples of such methods include, for example, HPLC, LC/MS, GC/MS, and NMR These methods can be independently used, or can be used in an appropriate combination.

The collected sterol may be, for example, a free sterol, a derivative thereof (such as sterol ester, steryl glucoside, and acyl steryl glucoside), or a mixture thereof. When the sterol is present in a form of such a derivative, a free sterol can also be obtained by hydrolysis. Hydrolysis can be carried out in a conventional manner. For example, hydrolysis can be carried out enzymatically by using esterase for sterol esters or glycosidase for steryl glucosides and acyl steryl glucosides.

The sterol can be used for various use purposes. The use purpose of sterol is not particularly limited. The sterol can be used as it is solely, after being blended with another ingredient, or as a raw material of another ingredient. Examples of the use purpose of sterol include food additives, feed additives, health foods, pharmaceuticals, chemicals, cosmetic ingredients, and raw materials thereof. Examples of the feed include livestock feeds and aquatic feeds.

### Examples

Hereinafter, the present invention will be more specifically explained with reference to non-limiting examples.

### Example 1: Obtaining a sterol-accumulating strain of Labyrinthulea

A sample of leaf rot containing river water of Okinawa was collected. The sample was added with an appropriate amount of pine pollen (collected in Miyazaki Prefecture), and left to stand at room temperature for 8 days. A 100 µl-aliquot of the sample after being left to stand was seeded on plates of a culture medium, wherein the culture medium was 0.1 × Reichmani preservation medium (Nissui) prepared with 0.5 × artificial seawater, to which penicillin G calcium and streptomycin sulfate were each added to a final concentration of 50 mg/L, and to which vitamin B 1 (Nacalai), vitamin B2 (Nacalai), and vitamin B12 (Nacalai) were added to a final concentration of 2 mg/L, 0.01 mg/L, and 0.01 mg/L, respectively. The plates were each cultured at 28°C for 4 days, and the obtained colonies were streaked on the same medium to purify the colonies. One of thus-obtained strains was named AJ7869 strain (FERM BP-22342). Furthermore, the AJ7868 strain (FERM BP-22290) was obtained by the same procedure (WO2017/065293). Furthermore, the AJ7879 strain (FERM BP-22367) was obtained in the same procedure. The compositions of 1 × Reichmani preservation medium (Nissui) and 1 × artificial seawater are as follows.

### <Composition of 1 × Reichmani preservation medium (Nissui)>

| | |
|---|---|
| Yeast extract | 8.5 g |
| Peptone | 8.5 g |
| Glucose | 11 g |
| Potassium dihydrogen phosphate | 2.0 g |
| Tomato juice powder | 3.7 g |
| Polysorbate | 80 1.0 g |
| Agar | 15 g |
| Ultra pure water | 1000 ml |

### <Composition of 1 × artificial seawater>

| | |
|---|---|
| NaCl | 30 g |
| KCl | 0.7 g |
| MgCl₂ · 6H₂O | 10.8 g |
| MgSO₄ · 7H₂O | 5.4 g |
| CaCl₂ · 2H₂O | 1 g |
| Ultra pure water | 1000 ml |

### Example 2: 18S rDNA analysis of sterol accumulation strains

With respect to the AJ7869 strain isolated as described above, the nucleotide sequence of the 18S rDNA region was determined using universal primers for amplifying Labyrinthulea 18S rDNA region (SEQ ID NOS: 1 and 2). In addition, the nucleotide sequence of the 18S rDNA region was determined for AJ7868 and AJ7879 strains on the basis of analysis with Miseq manufactured by Illumina. The nucleotide sequence of the 18S rDNA region of the AJ7869 strain is shown in SEQ ID NO: 3. The nucleotide sequence of the 18S rDNA region of AJ7868 strain is shown in SEQ ID NO: 43. The nucleotide sequence of the 18S rDNA region of the AJ7879 strain is shown in SEQ ID NO: 44. The 18S rDNA of the AJ7869, AJ7868, and AJ7879 strains showed a high identity of 99%, 98%, and 99% to the nucleotide sequence of the 18S rDNA of Aurantiochytrium sp. BURABG162 (SEQ ID NO: 4), Aurantiochytrium sp. SKE217 (SEQ ID NO: 5), and Aurantiochytrium sp. SKE218 (SEQ ID NO: 6), respectively. Those three strains have been reported to belong to Aurantiochytrium sp.1 in a report by Ueda et al. (Mayumi Ueda, et al., Seasonal dynamics of culturable thraustochytrids (Labyrinthulomycetes, Stramenopile) in estuarine and coastal waters., Aquatic Microbial Ecology. 2015 ;74:187-204). As a result, since the AJ7869, AJ7868, and AJ7879 strains were found to be related to Aurantiochytrium sp.1, these strains were named the Aurantiochytrium sp.1 AJ7869, AJ7868, and AJ7879 strains, respectively.

### Example 3: Construction of smtl gene-deficient strain and culture evaluation (1)

A deficient strain of a smtl gene (SEQ ID NO: 7) was constructed from the AJ7869 strain as a parent strain by the following procedure.

PCR was performed using the genomic DNA of the AJ7869 strain as a template and the primers of SEQ ID NOS: 8 and 9, to amplify a DNA fragment containing an upstream region of the smtl gene. PCR was performed using the genomic DNA of the AJ7869 strain as a template and the primers of SEQ ID NOS: 10 and 11, to amplify a DNA fragment containing a downstream region of the smtl gene. PCR was performed using an expression cassette for a blasticidin S resistance gene (Keishi Sakaguchi, et al., Versatile Transformation system that is Applicable to both multiple transgene expression and gene targeting for thraustochytrids., Applied and Environmental Microbiology. 2012 ;78(9):3193-3202.) and primers of SEQ ID NOS: 12 and 13, to amplify a DNA fragment containing the blasticidin S resistance gene. PCR was performed using the plasmid pUC19 as a template and primers of SEQ ID NOS: 14 and 15, to amplify a linear DNA fragment of pUC19. These DNA fragments were mutually ligated using In-fusion (Takara), and the resulting plasmid was named pUC19smt1-bla. PCR was performed using pUC19smt1-bla as a template and primers of SEQ ID NOS: 16 and 17, to obtain a DNA fragment containing the upstream and downstream regions of the smtl gene and the blasticidin S resistance gene therebetween. This DNA fragment was introduced into the AJ7869 strain by electroporation (Keishi Sakaguchi, et al., Versatile Transformation system that is Applicable to both multiple transgene expression and gene targeting for thraustochytrids., Applied and Environmental Microbiology. 2012 ;78(9):3193-3202), to obtain a blasticidin S resistant strain. The obtained strain was named the AJ7870 strain. This strain is a smtl gene-deficient strain in which the smtl gene has been replaced with the blasticidin S resistance gene.

The AJ7869 and AJ7870 strains were each inoculated to a plate of a culture medium, wherein the culture medium was 0.2 × Reichmani preservation medium (Nissui) prepared with 0.5 × artificial seawater, to which vitamin B1 (Nacalai), vitamin B2 (Nacalai), and vitamin B12 (Nacalai) were added to a final concentration of 2 mg/L, 0.01 mg/L, and 0.01 mg/L, respectively, and cultured at 28.5°C for 60 hours. Five platinum loops of cells on the plate medium were scraped and inoculated into a 500 ml-volume Erlenmeyer flask with a baffle containing 50 mL of GY medium prepared with 0.25 × artificial seawater having the following composition, and cultured at a culture temperature of 28.5°C with stirring at 120 rpm (rotary) for 24 hours. A 1.5 ml-aliquot of the obtained culture broth was inoculated into a 500 ml-volume Erlenmeyer flask with a baffle containing 48.5 ml of GTY medium prepared with 0.25 × artificial seawater, and cultured at a culture temperature of 28.5°C with stirring at 120 rpm (rotary) for 48 hours.

### <Composition of GY medium>

| (Group A) | |
|---|---|
| Glucose | 30 g/L |

| (Group B) | |
|---|---|
| Yeast extract | 10 g/L |

| (Group C) | |
|---|---|
| Vitamin B1 | 2 mg/L |
| Vitamin B2 | 0.01 mg/L |
| Vitamin B12 | 0.01 mg/L |

| (Group D) | |
|---|---|
| PIPES | 13.6 g/L |

The group B was adjusted to pH 6.5 using HCl and autoclaved at 120°C for 10 minutes, and the group A was autoclaved at 120°C for 10 minutes without pH adjustment. The group D was adjusted to 6.5 using NaOH and filtrated with a filter, and the group C was filtrated with a filter without pH adjustment. After cooling the groups A and B to room temperature, the four groups were mixed.

### <Composition of GTY medium>

| (Group A) | |
|---|---|
| Glucose | 50 g/L |

| (Group B) | |
|---|---|
| Tripton | 10 g/L |
| Yeast extract | 5 g/L |

| (Group C) | |
|---|---|
| Vitamin B1 | 2 mg/L |
| Vitamin B2 | 0.01 mg/L |
| Vitamin B12 | 0.01 mg/L |

| (Group D) | |
|---|---|
| PIPES | 13.6 g/L |

The group B was adjusted to pH 6.5 using HCl and autoclaved at 120°C for 10 minutes, and the group A was autoclaved at 120°C for 10 minutes without pH adjustment. The group D was adjusted to 6.5 using NaOH and filtrated with a filter, and the group C was filtrated with a filter without pH adjustment. After cooling the groups A and B to room temperature, the four groups were mixed.

After completion of the culture, 0.5 ml of the culture solution was centrifuged, and lipids were extracted by the Bligh-Dyer method from the obtained precipitate, to obtain a lipid extract. In order to hydrolyze sterol esters in the lipid extract, a fatty acid methylation kit (Nacalai) capable of hydrolyzing sterol esters while methylating fatty acids was used. The obtained hexane layer was applied to gas chromatography, to quantify each sterol. The dry cell weight (DCW) was calculated by centrifuging 0.5 ml of the culture solution, drying the obtained precipitate at 50°C for 3 days, and subtracting the weight of the Eppendorf tube from the measured weight. Results are shown in Table 1. In the AJ7870 strain, accumulation of stigmasterol disappeared, and the accumulation amount of cholesterol was increased to an amount about 2.1 times as much as that of the AJ7869 strain. Therefore, it was shown that deficiency of the smtl gene is effective for cholesterol accumulation.

**Table 1: Cultivation results of the Aurantiochytrium sp.1 strain AJ7869 and a modified strain thereof**

| | AJ7869 strain (Wild-type strain) | AJ7870 strain (Δsmt1) |
|---|---|---|
| DCW (g/L) | 14.9 | 14.6 |
| Cholesterol/DCW(%) | 1.3 | 2.7 |
| Stigmasterol/DCW(%) | 1.2 | 0 |

### Example 4: Construction of dhcr7 gene-deficient strains and culture evaluation

Deficient strains of a dhcr7 gene (SEQ ID NO: 18) were constructed from the AJ7869 and AJ7870 strains as parent strains by the following procedure.

PCR was performed using the genomic DNA of the AJ7869 strain as a template and the primers of SEQ ID NOS: 19 and 20, to amplify a DNA fragment containing an upstream region of the dhcr7 gene. PCR was performed using the genomic DNA of the AJ7869 strain as a template and the primers of SEQ ID NOS: 21 and 22, to amplify a DNA fragment containing a downstream region of the dhcr7 gene. PCR was performed using an expression cassette for a zeocin resistance gene (Keishi Sakaguchi, et al., Versatile Transformation system that is Applicable to both multiple transgene expression and gene targeting for thraustochytrids., Applied and Environmental Microbiology. 2012 ;78(9):3193-3202.) and primers of SEQ ID NOS: 23 and 24, to amplify a DNA fragment containing the zeocin resistance gene. PCR was performed using the plasmid pUC19 as a template and primers of SEQ ID NOS: 25 and 26, to amplify a linear DNA fragment of pUC19. These DNA fragments were mutually ligated using In-fusion (Takara), and the resulting plasmid was named pUC19dhcr7-zeo. PCR was performed using pUC19dhcr7-zeo as a template and primers of SEQ ID NOS: 27 and 28, to obtain a DNA fragment containing the upstream and downstream regions of the dhcr7 gene and the zeocin resistance gene therebetween. This DNA fragment was introduced into the AJ7869 and AJ7870 strains by electroporation (Keishi Sakaguchi, et al., Versatile Transformation system that is Applicable to both multiple transgene expression and gene targeting for thraustochytrids., Applied and Environmental Microbiology. 2012 ;78(9):3193-3202), to obtain zeocin resistant strains. The obtained strains were named the AJ7872 and AJ7871 strains, respectively. Each of these strains is a dhcr7 gene-deficient strain in which the dhcr7 gene has been replaced with the zeocin resistance gene.

The AJ7869, AJ7870, AJ7872, and AJ7871 strains were each inoculated to a plate of a culture medium, wherein the culture medium was 0.2 × Reichmani preservation medium (Nissui) prepared with 0.5 × artificial seawater, to which vitamin B1 (Nacalai), vitamin B2 (Nacalai), and vitamin B12 (Nacalai) were added to a final concentration of 2 mg/L, 0.01 mg/L, and 0.01 mg/L, respectively, and cultured at 28.5°C for 60 hours. Five platinum loops of cells on the plate medium were scraped and inoculated into a 500 ml-volume Erlenmeyer flask with a baffle containing 50 mL of GY medium prepared with 0.25 × artificial seawater having the following composition, and cultured at a culture temperature of 28.5°C with stirring at 120 rpm (rotary) for 24 hours. A 1.5 ml-aliquot of the obtained culture broth was inoculated into a 500 ml-volume Erlenmeyer flask with a baffle containing 48.5 ml of GTY medium prepared with 0.25 × artificial seawater, and cultured at a culture temperature of 28.5°C with stirring at 120 rpm (rotary) for 48 hours.

After completion of the culture, 0.5 ml of the culture solution was centrifuged, and lipids were extracted by the Bligh-Dyer method from the obtained precipitate, to obtain a lipid extract. In order to hydrolyze sterol esters in the lipid extract, a fatty acid methylation kit (Nacalai) capable of hydrolyzing sterol esters while methylating fatty acids was used. The obtained hexane layer was applied to gas chromatography, to quantify each sterol. The dry cell weight (DCW) was calculated by centrifuging 0.5 ml of the culture solution, drying the obtained precipitate at 50°C for 3 days, and subtracting the weight of the Eppendorf tube from the measured weight. Results are shown in Table 2. In the AJ7870, AJ7872, and AJ7871 strains, accumulation of 7-dehydrocholesterol (provitamin D3), which was not observed in the AJ7869 strain, was observed. In addition, in the AJ7871 strain, the accumulated amount of 7-dehydrocholesterol was increased as compared with the AJ7870 and AJ7872 strains. Therefore, it was shown that deficiency of the smtl gene and deficiency of the dhcr7 gene are each effective for 7-dehydrocholesterol accumulation. In addition, accumulation of ergosterol (provitamin D2) was observed in the AJ7872 strain, while it was not observed in the AJ7869 strain. Furthermore, in the AJ7872 strain, the accumulation amount of provitamin D6 was increased to an amount 1.4 times as much as that of the AJ7869 strain (not shown in Table). Therefore, it was shown that deficiency of the dhcr7 gene is effective for accumulation of provitamins D.

**Table 2: Cultivation results of the Aurantiochytrium sp.1 strain AJ7869 and modified strains thereof**

| | AJ7869 strain (Wild-type strain) | AJ7870 strain (Δsmt1) | AJ7872 strain (Δdhcr7) | AJ7871 strain (Δsmt1Δdhcr7) |
|---|---|---|---|---|
| DCW (g/L) | 16.2 | 15.8 | 15.8 | 16.2 |
| 7-dehydro cholesterol (g/L) | 0 | 0.07 | 0.20 | 0.25 |
| Cholesterol (g/L) | 0.18 | 0.38 | 0 | 0 |
| Ergosterol (g/L) | 0 | 0 | 0.13 | 0 |

### Example 5: Construction of dhcr24 gene-deficient strain and culture evaluation

A deficient strain of a dhcr24 gene (SEQ ID NO: 29) was constructed from the AJ7869 strain as a parent strain by the following procedure.

PCR was performed using the genomic DNA of the AJ7869 strain as a template and the primers of SEQ ID NOS: 30 and 31, to amplify a DNA fragment containing an upstream region of the dhcr24 gene. PCR was performed using the genomic DNA of the AJ7869 strain as a template and the primers of SEQ ID NOS: 32 and 33, to amplify a DNA fragment containing a downstream region of the dhcr24 gene. PCR was performed using an expression cassette for a zeocin resistance gene (Keishi Sakaguchi, et al., Versatile Transformation system that is Applicable to both multiple transgene expression and gene targeting for thraustochytrids., Applied and Environmental Microbiology. 2012 ;78(9):3193-3202.) and primers of SEQ ID NOS: 34 and 35, to amplify a DNA fragment containing the zeocin resistance gene. PCR was performed using the plasmid pUC19 as a template and primers of SEQ ID NOS: 36 and 37, to amplify a linear DNA fragment of pUC19. These DNA fragments were subject to cloning using In-fusion (Takara), and the resulting plasmid was named pUC19dhcr24-zeo. PCR was performed using pUC19dhcr24-zeo as a template and primers of SEQ ID NOS: 30 and 33, to obtain a DNA fragment containing the upstream and downstream regions of the dhcr24 gene and the zeocin resistance gene therebetween. This DNA fragment was introduced into the AJ7869 strain by electroporation (Keishi Sakaguchi, et al., Versatile Transformation system that is Applicable to both multiple transgene expression and gene targeting for thraustochytrids., Applied and Environmental Microbiology. 2012 ;78(9):3193-3202), to obtain a zeocin resistant strain. The obtained strain was named the AJ7873 strain. This strain is a dhcr24 gene-deficient strain in which the dhcr24 gene has been replaced with the zeocin resistance gene.

The AJ7869 and AJ7873 strains were each inoculated to a plate of a culture medium, wherein the culture medium was 0.2 × Reichmani preservation medium (Nissui) prepared with 0.5 × artificial seawater, to which vitamin B1 (Nacalai), vitamin B2 (Nacalai), and vitamin B12 (Nacalai) were added to a final concentration of 2 mg/L, 0.01 mg/L, and 0.01 mg/L, respectively, and cultured at 28.5°C for 60 hours. Five platinum loops of cells on the plate medium were scraped and inoculated into a 500 ml-volume Erlenmeyer flask with a baffle containing 50 mL of GY medium prepared with 0.25 × artificial seawater having the following composition, and cultured at a culture temperature of 28.5°C with stirring at 120 rpm (rotary) for 24 hours. A 1.5 ml-aliquot of the obtained culture broth was inoculated into a 500 ml-volume Erlenmeyer flask with a baffle containing 48.5 ml of GTY medium prepared with 0.25 × artificial seawater, and cultured at a culture temperature of 28.5°C with stirring at 120 rpm (rotary) for 48 hours.

After completion of the culture, 0.5 ml of the culture solution was centrifuged, and lipids were extracted by the Bligh-Dyer method from the obtained precipitate, to obtain a lipid extract. In order to hydrolyze sterol esters in the lipid extract, a fatty acid methylation kit (Nacalai) capable of hydrolyzing sterol esters while methylating fatty acids was used. The obtained hexane layer was applied to gas chromatography, to quantify each sterol. The dry cell weight (DCW) was calculated by centrifuging 0.5 ml of the culture solution, drying the obtained precipitate at 50°C for 3 days, and subtracting the weight of the Eppendorf tube from the measured weight. Results are shown in Table 3. In the AJ7873 strain, accumulation of cholesterol disappeared, and the accumulation amounts of stigmasterol and brassicasterol were increased to amounts 1.4 times and 2.2 times as much as those of the AJ7869 strain, respectively. Therefore, it was shown that deficiency of the dhcr24 gene is effective for accumulation of sterols having methyl or ethyl at position C24.

**Table 3: Cultivation results of the Aurantiochytrium sp.1 strain AJ7869 and a modified strain thereof**

| | AJ7869 strain (Wild-type strain) | AJ7873 strain (Δdhcr24) |
|---|---|---|
| DCW (g/L) | 15.4 | 14 |
| Cholesterol (g/L) | 0.19 | 0 |
| Stigmasterol (g/L) | 0.17 | 0.24 |
| Brassicasterol (g/L) | 0.05 | 0.11 |

### Example 6: Construction of smtl gene-deficient strains and culture evaluation (2)

Deficient strains of a smtl gene (SEQ ID NO: 55) were constructed from the AJ7879 and AJ7868 strains as parent strains by the following procedure.

A DNA fragment containing the upstream and downstream regions of the smtl gene and the blasticidin S resistance gene therebetween was obtained according to the procedure described in Example 3.

PCR was performed using the genomic DNA of the AJ7869 strain as a template and the primers of SEQ ID NOS: 8 and 9, to amplify a DNA fragment containing an upstream region of the smtl gene. PCR was performed using the genomic DNA of the AJ7869 strain as a template and the primers of SEQ ID NOS: 10 and 11, to amplify a DNA fragment containing a downstream region of the smtl gene. PCR was performed using an expression cassette for a neomycin resistance gene (Keishi Sakaguchi, et al., Versatile Transformation system that is Applicable to both multiple transgene expression and gene targeting for thraustochytrids., Applied and Environmental Microbiology. 2012 ;78(9):3193-3202.) and primers of SEQ ID NOS: 12 and 13, to amplify a DNA fragment containing the neomycin resistance gene. PCR was performed using the plasmid pUC19 as a template and primers of SEQ ID NOS: 14 and 15, to amplify a linear DNA fragment of pUC19. These DNA fragments were mutually ligated using In-fusion (Takara), and the resulting plasmid was named pUC19smt1-neo. PCR was performed using pUC19smt1-neo as a template and primers of SEQ ID NOS: 16 and 17, to obtain a DNA fragment containing the upstream and downstream regions of the smtl gene and the neomycin resistance gene therebetween.

The DNA fragment containing the upstream and downstream regions of the smtl gene and the blasticidin S resistance gene therebetween was introduced into the AJ7879 strain by electroporation (Keishi Sakaguchi, et al., Versatile Transformation system that is Applicable to both multiple transgene expression and gene targeting for thraustochytrids., Applied and Environmental Microbiology. 2012 ;78(9):3193-3202), to obtain a blasticidin S resistant strain. The obtained strain was named the AJ7880 strain. This strain is a smtl gene-deficient strain in which the smtl gene has been replaced with the blasticidin S resistance gene.

The DNA fragment containing the upstream and downstream regions of the smtl gene and the blasticidin S resistance gene therebetween was introduced into the AJ7868 strain by electroporation (Keishi Sakaguchi, et al., Versatile Transformation system that is Applicable to both multiple transgene expression and gene targeting for thraustochytrids., Applied and Environmental Microbiology. 2012 ;78(9):3193-3202), to obtain a blasticidin S resistant strain. The obtained strain was named the AJ7868 blasticidin S resistant strain. This strain is a smtl gene-deficient strain in which one of two smtl genes present on the chromosome has been replaced with the blasticidin S resistance gene. This strain was further introduced with the DNA fragment containing the upstream and downstream regions of the smtl gene and the neomycin resistance gene therebetween by electroporation, to obtain a neomycin resistant strain. The obtained strain was named the AJ7878 strain. This strain is a smtl gene-deficient strain in which two smtl genes present on the chromosome has been replaced respectively with the blasticidin S resistance gene and the neomycin resistance gene.

The AJ7879, AJ7880, AJ7868, and AJ7878 strains were each inoculated to a plate of a culture medium, wherein the culture medium was 0.2 × Reichmani preservation medium (Nissui) prepared with 0.5 × artificial seawater, to which vitamin B1 (Nacalai), vitamin B2 (Nacalai), and vitamin B12 (Nacalai) were added to a final concentration of 2 mg/L, 0.01 mg/L, and 0.01 mg/L, respectively, and cultured at 28.5°C for 60 hours. Five platinum loops of cells on the plate medium were scraped and inoculated into a 500 ml-volume Erlenmeyer flask with a baffle containing 50 mL of GY medium prepared with 0.25 × artificial seawater having the following composition, and cultured at a culture temperature of 28.5°C with stirring at 120 rpm (rotary) for 24 hours. A 1.5 ml-aliquot of the obtained culture broth was inoculated into a 500 ml-volume Erlenmeyer flask with a baffle containing 48.5 ml of GTY medium prepared with 0.25 × artificial seawater, and cultured at a culture temperature of 28.5°C with stirring at 120 rpm (rotary) for 48 hours.

After completion of the culture, 0.5 ml of the culture solution was centrifuged, and lipids were extracted by the Bligh-Dyer method from the obtained precipitate, to obtain a lipid extract. In order to hydrolyze sterol esters in the lipid extract, a fatty acid methylation kit (Nacalai) capable of hydrolyzing sterol esters while methylating fatty acids was used. The obtained hexane layer was applied to gas chromatography, to quantify each sterol. The dry cell weight (DCW) was calculated by centrifuging 0.5 ml of the culture solution, drying the obtained precipitate at 50°C for 3 days, and subtracting the weight of the Eppendorf tube from the measured weight. Results are shown in Table 4. In the AJ7880 strain, accumulation of stigmasterol disappeared, and the accumulation amount of cholesterol was increased to an amount about 1.9 times as much as that of the AJ7879 strain. Also, in the AJ7878 strain, accumulation of stigmasterol disappeared, and the accumulation amount of cholesterol was increased to an amount about 2.2 times as much as that of the AJ7868 strain. Therefore, it was shown that, also for other strains Aurantiochytrium sp.1, deficiency of the smtl gene is effective for cholesterol accumulation.

**Table 4: Cultivation results of the Aurantiochytrium sp.1 wild-type strains and modified strains thereof**

| | AJ7879 strain (Wild-type strain) | AJ7880 strain (Δsmt1) | AJ7868 strain (Wild-type strain) | AJ7878 strain (Δsmt1) |
|---|---|---|---|---|
| DCW (g/L) | 17.5 | 17.3 | 16.0 | 16.5 |
| Cholesterol /DCW(%) | 1.2 | 2.3 | 1.3 | 2.8 |
| Stigmasterol /DCW(%) | 1.1 | 0 | 1.3 | 0 |

### Example 7: Construction of double expression enhanced strain of dhcr24 and dhcr7 genes and culture evaluation

A double expression enhanced strain of a dhcr24 gene (SEQ ID NO: 29) and a dhcr7 gene (SEQ ID NO: 18) was constructed from the AJ7870 strain as a parent strain by the following procedure.

PCR was performed the primers of SEQ ID NOS: 47 and 48, in combination with a plasmid containing an expression cassette for neomycin resistance gene-dhcr7 gene (SEQ ID NO: 45), to amplify a DNA fragment containing the expression cassette for neomycin resistance gene-dhcr7 gene, and in combination with a plasmid containing an expression cassette for zeocin resistance gene-dhcr24 gene (SEQ ID NO: 46), to amplify a DNA fragment containing the expression cassette for neomycin resistance gene-dhcr24 gene. These DNA fragments were introduced into the AJ7870 strain by electroporation (Keishi Sakaguchi, et al., Versatile Transformation system that is Applicable to both multiple transgene expression and gene targeting for thraustochytrids., Applied and Environmental Microbiology. 2012 ;78(9):3193-3202), to obtain a strain resistant to both neomycin and zeocin. The obtained strain was named the AJ7882 strain. This strain is a double expression enhanced strain of the dhcr24 and dhcr7 genes in which the dhcr24 and dhcr7 genes were randomly introduced into the chromosome.

### Example 8: Construction of cyp710a gene-deficient strain and culture evaluation

A deficient strain of a cyp710a gene (SEQ ID NO: 41) encoding sterol C-22 desaturase was constructed from the AJ7882 strain (a double expression enhanced strain of the dhcr24 and dhcr7 genes) as a parent strain by the following procedure.

PCR was performed using the genomic DNA of the AJ7869 strain as a template and the primers of SEQ ID NOS: 49 and 50, to amplify a DNA fragment containing an upstream region of the cyp710a gene. PCR was performed using an expression cassette for a hygromycin resistance gene (pUC19 pUBI-HygR-SV40; Keishi Sakaguchi, et al., Versatile Transformation system that is Applicable to both multiple transgene expression and gene targeting for thraustochytrids., Applied and Environmental Microbiology. 2012 ;78(9):3193-3202.) and primers of SEQ ID NOS: 51 and 52, to amplify a DNA fragment containing the blasticidin S resistance gene. PCR was performed using the genomic DNA of the AJ7869 strain as a template and the primers of SEQ ID NOS: 53 and 54, to amplify a DNA fragment containing a downstream region of the cyp710a gene. Crossover PCR was performed using these DNA fragments as templates and primers of SEQ ID NOS: 49 and 54, to obtain a DNA fragment containing the upstream and downstream regions of the cyp710a gene and the hygromycin resistance gene therebetween. This DNA fragment was introduced into the AJ7882 strain by electroporation (Keishi Sakaguchi, et al., Versatile Transformation system that is Applicable to both multiple transgene expression and gene targeting for thraustochytrids., Applied and Environmental Microbiology. 2012 ;78(9):3193-3202), to obtain a hygromycin resistant strain. The obtained strain was named the AJ7881 strain. This strain is a cyp710a gene-deficient strain in which the cyp710a gene has been replaced with the hygromycin resistance gene.

The AJ7870, AJ7881, and AJ7882 strains were each inoculated to a plate of a culture medium, wherein the culture medium was 0.2 × Reichmani preservation medium (Nissui) prepared with 0.5 × artificial seawater, to which vitamin B 1 (Nacalai), vitamin B2 (Nacalai), and vitamin B12 (Nacalai) were added to a final concentration of 2 mg/L, 0.01 mg/L, and 0.01 mg/L, respectively, and cultured at 28.5°C for 60 hours. Five platinum loops of cells on the plate medium were scraped and inoculated into a 500 ml-volume Erlenmeyer flask with a baffle containing 50 mL of GY medium prepared with 0.25 × artificial seawater having the following composition, and cultured at a culture temperature of 28.5°C with stirring at 120 rpm (rotary) for 24 hours. A 1.5 ml-aliquot of the obtained culture broth was inoculated into a 500 ml-volume Erlenmeyer flask with a baffle containing 48.5 ml of GTY medium prepared with 0.25 × artificial seawater, and cultured at a culture temperature of 28.5°C with stirring at 120 rpm (rotary) for 48 hours.

After completion of the culture, 0.5 ml of the culture solution was centrifuged, and lipids were extracted by the Bligh-Dyer method from the obtained precipitate, to obtain a lipid extract. In order to hydrolyze sterol esters in the lipid extract, a fatty acid methylation kit (Nacalai) capable of hydrolyzing sterol esters while methylating fatty acids was used. The obtained hexane layer was applied to gas chromatography, to quantify each sterol. The dry cell weight (DCW) was calculated by centrifuging 0.5 ml of the culture solution, drying the obtained precipitate at 50°C for 3 days, and subtracting the weight of the Eppendorf tube from the measured weight. Results are shown in Table 5. In the AJ7882 strain (Δsmt1/dhcr24+dhcr7 double expression enhanced strain), the accumulation amount of cholesterol was increased to an amount about 1.3 times as much as that of the AJ7870 strain (Δsmt1). Furthermore, in the AJ7881 strain (Δsmt1Δcyp710a/dhcr24+dhcr7 double expression enhanced strain), the accumulation amount of cholesterol was increased to an amount about 1.2 times as much as that of the AJ7882 strain (Δsmt1/dhcr24+dhcr7 double expression enhanced strain). Therefore, it was shown that enhancement of cholesterol biosynthesis pathway such as dhcr7 and dhcr24 and suppression of by-production of C22 unsaturated sterols by disruption of the cyp710a gene are effective for cholesterol accumulation in a smt1-gene deficient strain.

**Table 5: Cultivation results of modified strains of the Aurantiochytrium sp.1 strain AJ7869**

| | AJ7870 strain (Δsmt1) | AJ7882 strain (Δsmt1 /dhcr24+dhcr7 double expression enhanced strain) | AJ7881 strain (Δsmt1Δcyp710a /dhcr24+dhcr7 double expression enhanced strain) |
|---|---|---|---|
| DCW (g/L) | 16.6 | 15.7 | 15 |
| Cholesterol (g/L) | 0.34 | 0.41 | 0.46 |
| Cholesterol /DCW(%) | 2.0 | 2.6 | 3.1 |

### Industrial Applicability

According to the present invention, a sterol-producing ability of a Labyrinthulea microorganism can be improved, and a sterol can be efficiently produced.

### <Explanation of Sequence Listing>

### SEQ ID NOS:

1-2: Primers
3: Nucleotide sequence of 18S rDNA region of Aurantiochytrium sp.1 AJ7869 strain
4: Nucleotide sequence of 18S rDNA region of Aurantiochytrium sp.1 BURABG162 strain
5: Nucleotide sequence of 18S rDNA region of Aurantiochytrium sp.1 SKE217 strain
6: Nucleotide sequence of 18S rDNA region of Aurantiochytrium sp.1 SKE218 strain
7: Nucleotide sequence of SMT1 gene of Aurantiochytrium sp.1 AJ7869 strain
8-17: Primers
18: Nucleotide sequence of DHCR7 gene of Aurantiochytrium sp.1 AJ7869 strain
19-28: Primers
29: Nucleotide sequence of DHCR24 gene of Aurantiochytrium sp.1 AJ7869 strain
30-37: Primers
38: Amino acid sequence of SMT1 of Aurantiochytrium sp.1 AJ7869 strain
39: Amino acid sequence of DHCR7 of Aurantiochytrium sp.1 AJ7869 strain
40: Amino acid sequence of DHCR24 of Aurantiochytrium sp.1 AJ7869 strain
41: Nucleotide sequence of cyp710a gene of Aurantiochytrium sp.1 AJ7869 strain
42: Amino acid sequence of CYP710A of Aurantiochytrium sp.1 AJ7869 strain
43: Nucleotide sequence of 18S rDNA region of Aurantiochytrium sp.1 AJ7868 strain
44: Nucleotide sequence of 18S rDNA region of Aurantiochytrium sp.1 AJ7879 strain
45: Nucleotide sequence of an expression cassette for neomycin resistance gene-dhcr7 gene
46: Nucleotide sequence of an expression cassette for zeocin resistance gene-dhcr24 gene
47-54: Primers
55: Nucleotide sequence of SMT1 gene of Aurantiochytrium sp.1 AJ7868 and AJ7879 strains
56: Amino acid sequence of SMT1 of Aurantiochytrium sp.1 AJ7868 and AJ7879 strains

## Claims

1. A Labyrinthulea microorganism,
which has a sterol-producing ability,
which is Aurantiochytrium sp. 1,
which has been modified so that the activity of 24-dehydrocholesterol reductase is reduced as compared with a non-modified strain, and
wherein the sterol is at least one sterol selected from plant sterols and fungal sterols.

2. The microorganism according to claim 1, wherein the activity of the 24-dehydrocholesterol reductase is reduced by reducing the expression of a gene encoding the 24-dehydrocholesterol reductase or disrupting the gene.

3. The microorganism according to claim 1 or 2, wherein the activity of the 24-dehydrocholesterol reductase is reduced by deletion of a part or the whole of the amino acid sequence of the 24-dehydrocholesterol reductase.

4. The microorganism according to any one of claims 1 to 3, wherein the sterol is stigmasterol and/or brassicasterol.

5. A Labyrinthulea microorganism,
which has a sterol-producing ability,
which is Aurantiochytrium sp. 1,
which has been modified so that the activity of 7-dehydrocholesterol reductase is reduced as compared with a non-modified strain, and
wherein the sterol is 7-dehydrocholesterol and/or ergosterol.

6. The microorganism according to claim 5, wherein the activity of the 7-dehydrocholesterol reductase is reduced by reducing the expression of a gene encoding the 7-dehydrocholesterol reductase or disrupting the gene.

7. The microorganism according to claim 5 or 6, wherein the activity of the 7-dehydrocholesterol reductase is reduced by deletion of a part or the whole of the amino acid sequence of the 7-dehydrocholesterol reductase.

8. The microorganism according to any one of claims 5 to 7, which has been further modified so that the activity of sterol 24-C-methyltransferase is reduced as compared with a non-modified strain.

9. A Labyrinthulea microorganism,
which has a sterol-producing ability,
which is Aurantiochytrium sp.1,
which has been modified so that the activity of sterol 24-C-methyltransferase is reduced as compared with a non-modified strain, and
wherein the sterol is at least one sterol selected from animal sterols.

10. The microorganism according to claim 8 or 9, wherein the sterol 24-C-methyltransferase is a protein defined in (a), (b), or (c) mentioned below:
(a) a protein comprising the amino acid sequence of SEQ ID NO: 38 or 56;
(b) a protein comprising the amino acid sequence of SEQ ID NO: 38 or 56, but which includes substitution, deletion, insertion, and/or addition of 1 to 10 amino acid residues, and having a sterol 24-C-methyltransferase activity;
(c) a protein comprising an amino acid sequence showing an identity of 90% or higher to the amino acid sequence of SEQ ID NO: 38 or 56, and having a sterol 24-C-methyltransferase activity.

11. The microorganism according to any one of claims 8 to 10, wherein the activity of the 24-dehydrocholesterol reductase is reduced by reducing the expression of a gene encoding the 24-dehydrocholesterol reductase or disrupting the gene.

12. The microorganism according to any one of claims 8 to 11, wherein the activity of the 24-dehydrocholesterol reductase is reduced by deletion of a part or the whole of the amino acid sequence of the 24-dehydrocholesterol reductase.

13. The microorganism according to any one of claims 9 to 12, wherein the sterol is cholesterol and/or 7-dehydrocholesterol.

14. The microorganism according to any one of claims 9 to 13, wherein the sterol is cholesterol.

15. The microorganism according to any one of claims 9 to 14, which further has at least one characteristic selected from (A), (B), and (C) mentioned below:
(A) the microorganism has been modified so that the activity of sterol C-22 desaturase is reduced as compared with a non-modified strain;
(B) the microorganism has been modified so that the activity of 24-dehydrocholesterol reductase is increased as compared with a non-modified strain;
(C) the microorganism has been modified so that the activity of 7-dehydrocholesterol reductase is increased as compared with a non-modified strain.

16. The microorganism according to claim 15, wherein the sterol C-22 desaturase is a protein defined in (a), (b), or (c) mentioned below:
(a) a protein comprising the amino acid sequence of SEQ ID NO: 42;
(b) a protein comprising the amino acid sequence of SEQ ID NO: 42, but which includes substitution, deletion, insertion, and/or addition of 1 to 10 amino acid residues, and having a sterol C-22 desaturase activity;
(c) a protein comprising an amino acid sequence showing an identity of 90% or higher to the amino acid sequence of SEQ ID NO: 42, and having a sterol C-22 desaturase activity.

17. The microorganism according to any one of claims 1 to 4, 15, and 16, wherein the 24-dehydrocholesterol reductase is a protein defined in (a), (b), or (c) mentioned below:
(a) a protein comprising the amino acid sequence of SEQ ID NO: 40;
(b) a protein comprising the amino acid sequence of SEQ ID NO: 40, but which includes substitution, deletion, insertion, and/or addition of 1 to 10 amino acid residues, and having a 24-dehydrocholesterol reductase activity;
(c) a protein comprising an amino acid sequence showing an identity of 90% or higher to the amino acid sequence of SEQ ID NO: 40, and having a 24-dehydrocholesterol reductase activity.

18. The microorganism according to any one of claims 5 to 8 and 15 to 17, wherein the 7-dehydrocholesterol reductase is a protein defined in (a), (b), or (c) mentioned below:
(a) a protein comprising the amino acid sequence of SEQ ID NO: 39;
(b) a protein comprising the amino acid sequence of SEQ ID NO: 39, but which includes substitution, deletion, insertion, and/or addition of 1 to 10 amino acid residues, and having a 7-dehydrocholesterol reductase activity;
(c) a protein comprising an amino acid sequence showing an identity of 90% or higher to the amino acid sequence of SEQ ID NO: 39, and having a 7-dehydrocholesterol reductase activity.

19. The microorganism according to any one of claims 1 to 18, wherein the nucleotide sequence of the 18S rDNA of the microorganism has an identity of 97% or higher to the nucleotide sequence of SEQ ID NO: 3.

20. The microorganism according to any one of claims 1 to 19, which is a modified strain derived from Aurantiochytrium sp.1 strain AJ7869 (FERM BP-22342), AJ7868 (FERM BP-22290), or AJ7879 (FERM BP-22367).

21. A method for producing a sterol, comprising:
culturing the microorganism according to any one of claims 1 to 20 in a culture medium; and
collecting the sterol from cells generated by the culturing.

22. The method according to claim 21, wherein the sterol is a free sterol, a sterol ester, a steryl glucoside, an acyl steryl glucoside, or a mixture thereof.


SEQUENCE LISTING

<110>  Ajinomoto Co., Inc.

<120>  Method for producing sterol

<130>  E042-18346

<150>  JP2017-202347
<151>  2017-10-19

<160>  56

<170>  PatentIn version 3.5

<210>  1
<211>  21
<212>  DNA
<213>  Artificial Sequence

<220>
<223>  primer

<400>  1
aacctggttg atcctgccag t                                                21


<210>  2
<211>  21
<212>  DNA
<213>  Artificial Sequence

<220>
<223>  primer

<400>  2
tgatccttct gcaggttcac c                                                21


<210>  3
<211>  1761
<212>  DNA
<213>  Aurantiochytrium sp.1

<400>  3
cagtagccct acgctcgtct caaagactaa gccatgcatg tgtaagtata agcgaattat      60
actgtgaaac tgcgaacggc tcattatatc agttataatc ccttcggtag ttcctttaga     120
cggatacctg cagtaattct ggaattaata cgtgctgtac gggcccgact ttcggggagg     180
gccgcactta ttaggtctaa gccaacgttc ttggtgagtc atgataattg agcagatcgc     240
ttttcggagc gatgaatcgt ttgagtttct gccccatcag ttgtcgacgg tagggtattg     300
gcctacggtg actataacgg gtgacgggga gttagggctc gactccggag agggagcctg     360
agagacggct accacatcca aggaaggcag caggcgcgta aattacccaa tgtggactcc     420
acgaggtagt gacgagaaat atcaatgcgg ggcgcttcgc gtcttgctat tggaatgaga     480
gcaatgtaaa accctcatcg aggatcaact ggagggcaag tctggtgcca gcagccgcgg     540
taattccagc tccagaagcg tatgctaaag ttgttgcagt taaaaagctc gtagttgaat     600
ttctggtgtg ggagcccagg cctcggtgcg aatgcgcctt gtattgcctt gcggctcctt     660
tgccatcctc gtttttcttt tgaagaacgt cattcactgt aatcaaagca gagtgttcca     720
agcaggccgt agggccggta tgtttattat gggatgatca gataggactc gggtgctatt     780
ttgttggttt gcacatctga gtaatgatta ataggaacag tcgggggtat ccgtatttag     840
gagctagagg tgaaattctt ggatttccga aagacgaact acagcgaagg catttaccaa     900
gcatgttttc attaatcaag aacgaaagtc tggggatcga agatgattag ataccatcgt     960
agtctagacc gtaaacgatg ccgacttgcg attgcgggtg gcttgtattg ggcctccgca    1020
gcagcacatg agaaatcaaa gtctttgggt tccgggggga gtatggtcgc aaggctgaaa    1080

cttaaaggaa ttgacggaag ggcaccacca ggagtggagc ctgcggctta atttgactca 1140
acacgggaaa acttaccagg tccagacata ggtaggattg acagattgag agctctttct 1200
tgattctatg ggtggtggtg catggccgtt cttagttggt ggagtgattt gtctggttaa 1260
ttccgttaac gaacgagacc tcggcctact aaatagccgg gcgtatggcg acatatgtgt 1320
ttgtggcttc ttagagggac atgttcggtt tacgagcagg aagttcgagg caataacagg 1380
tctgtgatgc ccttagatgt tctgggccgc acgcgcgcta cactgatggg ttcagcgggt 1440
ctttgttgtg tttactcaca gcgttgcttt gtcggaaggc atggctaatc ctttgaacgc 1500
ccatcgtgct ggggctagat ttttgcaatt attaatctcc aacgaggaat tcctagtaaa 1560
cgcaagtcat cagcttgcat tgaatacgtc cctgcccttt gtacacaccg cccgtcgcac 1620
ctaccgattg aacggtccga tgaaaccatg ggactacctt ttgagcgttt gttcgcgatg 1680
gaggtgggaa ctcgggtgaa tcttattgtt tagaggaagg tgaagtcgta acaaggtttc 1740
cgtaggtgaa cctgcggaag g 1761

<210> 4
<211> 1731
<212> DNA
<213> Aurantiochytrium sp.1

<400> 4
tcgctcgtct caaagactaa gccatgcatg tgtaagtata agcgaattat actgtgaaac 60
tgcgaacggc tcattatatc agttataatc ccttcggtag ttcctttaga cggatacctg 120
cagtaattct ggaattaata cgtgctgtac gggcccgact ttcggggagg gccgcactta 180
ttaggtctaa gccaacgttc ttggtgagtc atgataattg agcagatcgc ttttcggagc 240
gatgaatcgt ttgagtttct gccccatcag ttgtcgacgg tagggtattg gcctacggtg 300
actataacgg gtgacgggga gttagggctc gactccggag agggagcctg agagacggct 360
accacatcca aggaaggcag caggcgcgta aattacccaa tgtggactcc acgaggtagt 420
gacgagaaat atcaatgcgg ggcgcttcgc gtcttgctat tggaatgaga gcaatgtaaa 480
accctcatcg aggatcaact ggagggcaag tctggtgcca gcagccgcgg taattccagc 540
tccagaagcg tatgctaaag ttgttgcagt taaaaagctc gtagttgaat ttctggtgtg 600
ggagcccagg cctcggtgcg aatgcgcctt gtcttgcctt gcggctcctt tgccatcctc 660
gtttttcttt tgaaggacgt cattcactgt aatcaaagca gagtgttcca agcaggccgt 720
agggccggta tgtttattat gggatgatca gataggactc gggtgctatt ttgttggttt 780
gcacatctga gtaatgatta ataggaacag tcgggggtat ccgtatttag gagctagagg 840
tgaaattctt ggatttccga aagacgaact acagcgaagg catttaccaa gcatgttttc 900
attaatcaag aacgaaagtc tggggatcga agatgattag ataccatcgt agtctagacc 960
gtaaacgatg ccgacttgcg attgcgggtg gcttgtattg ggcctccgca gcagcacatg 1020
agaaatcaaa gtctttgggt tccgggggga gtatggtcgc aaggctgaaa cttaaaggaa 1080
ttgacggaag ggcaccacca ggagtggagc ctgcggctta atttgactca acacgggaaa 1140
acttaccagg tccagacata ggtaggattg acagattgag agctctttct tgattctatg 1200
ggtggtggtg catggccgtt cttagttggt ggagtgattt gtctggttaa ttccgttaac 1260
gaacgagacc tcggcctact aaatagccgg gcgtatggcg acatatgtgt ttgtggcttc 1320
ttagagggac atgttcggtt tacgagcagg aagttcgagg caataacagg tctgtgatgc 1380
ccttagatgt tctgggccgc acgcgcgcta cactgatggg ttcagcgggt gatttatggg 1440
tttttcactg aagttgcttt gtcggaaggc atggctaatc ctttgaacgc ccatcgtgct 1500
ggggctagat ttttgcaatt attaatctcc aacgaggaat tcctagtaaa cgcaagtcat 1560
cagcttgcat tgaatacgtc cctgcccttt gtacacaccg cccgtcgcac ctaccgattg 1620
aacggtccga tgaaaccatg ggactacctt ttgagcgttt gttcgcgatg gaggtgggaa 1680
ctcgggtgaa tcttattgtt tagaggaagg tgaagtcgta acaaggtttc c 1731

<210> 5
<211> 1764
<212> DNA
<213> Aurantiochytrium sp.1

<220>
<221> misc_feature
<222> (532)..(532)
<223> n is a, c, g, or t

<220>

<221>  misc_feature
<222>  (535)..(535)
<223>  n is a, c, g, or t

<220>
<221>  misc_feature
<222>  (630)..(630)
<223>  n is a, c, g, or t

<220>
<221>  misc_feature
<222>  (687)..(687)
<223>  n is a, c, g, or t

<220>
<221>  misc_feature
<222>  (1000)..(1001)
<223>  n is a, c, g, or t

<220>
<221>  misc_feature
<222>  (1397)..(1397)
<223>  n is a, c, g, or t

<220>
<221>  misc_feature
<222>  (1722)..(1722)
<223>  n is a, c, g, or t

<220>
<221>  misc_feature
<222>  (1751)..(1751)
<223>  n is a, c, g, or t

<220>
<221>  misc_feature
<222>  (1755)..(1755)
<223>  n is a, c, g, or t

<220>
<221>  misc_feature
<222>  (1762)..(1762)
<223>  n is a, c, g, or t

<400>  5
ccagtagccc tacgctcgtc tcaaagacta agccatgcat gtgtaagtat aagcgaatta      60
tactgtgaaa ctgcgaacgg ctcattatat cagttataat cccttcggta gttcctttac     120
acggatacct gcagtaattc tggaattaat acgtgctgta cgggcccgac tttcggggag     180
ggccgcactt attaggtcta agccaacgtt cttggtgagt catgataatt gagcagatcg     240
cttttcggag cgatgaatcg tttgagtttc tgccccatca gttgtcgacg gtagggtatt     300
ggcctacggt gactataacg ggtgacgggg agttagggct cgactccgga gagggagcct     360
gagagacggc taccacatcc aaggaaggca gcaggcgcgt aaattaccca atgtggactc     420
cacgaggtag tgacgagaaa tatcaatgcg gggcgcttcg cgtcttgcta ttggaatgag     480
agcaatgtaa aaccctcatc gaggatcaac tggagggcaa gtctggtgcc ancanccgcg     540
gtaattccag ctccagaagc gtatgctaaa gttgttgcag ttaaaaagct cgtagttgaa     600
tttctggtgt gggagcccag gcctcggtgn gaatgcgcct tgtattgcct tgcggctccc     660
ttgccatcct cgtttttctt ttgaagnacg tcattcactg taatcaaagc agagtgttcc     720
aagcaggccg tagggccggt atgtttatta tgggatgatc agataggact cgggtgctat     780
tttgttggtt tgcacatctg agtaatgatt aataggaaca gtcgggggta tccgtattta     840
ggagctagag gtgaaattct tggatttccg aaagacgaac tacagcgaag gcatttacca     900
agcatgtttt cattaatcaa gaacgaaagt ctggggatcg aagatgatta gataccatcg     960
tagtctagac cgtaaacgat gccgacttgc gattgcgggn ngcttgtatt gggcttccgc    1020
agcagcacat gagaaatcaa agtctttggg ttccgggggg agtatggtcg caaggctgaa    1080

acttaaagga attgacggaa gggcaccacc aggagtggag cctgcggctt aatttgactc 1140
aacacgggaa aacttaccag gtccagacat aggtaggatt gacagattga gagctctttc 1200
ttgattctat gggtggtggt gcatggccgt tcttagttgg tggagtgatt tgtctggtta 1260
attccgttaa cgaacgagac ctcggcctac taaatagccg ggcgtatggc gacatatgtg 1320
tttgtggctt cttagaggga catgttcggt ttacgagcag gaagttcgag gcaataacag 1380
gtctgtgatg cccttanatg ttctgggccg cacgcgcgct acactgatgg gttcagcggg 1440
tctttgttgt gtttactcac agcgttgctt tgtcggaagg catggctaat cctttgaacg 1500
cccatcgtgc tggggctaaa tttttgcaat tattaatctc caacgaggaa ttcctagtaa 1560
acgcaagtca tcagcttgca ttgaatacgt ccctgccctt tgtacacacc gcccgtcgca 1620
cctaccgatt gaacggtccg atgaaaccat gggactacct tttgagcgtt tgttcccaat 1680
ggagggggga actcgggtga atcttattgt ttaaaggaag gngaagtcgt aacaaggttt 1740
ccgtaggtga ncctngcgga angg 1764

<210> 6
<211> 1711
<212> DNA
<213> Aurantiochytrium sp.1

<220>
<221> misc_feature
<222> (1451)..(1451)
<223> n is a, c, g, or t

<400> 6
ctgccagtag ccctacgctc gtctcaaaga ctaagccatg catgtgtaag tataagcgaa 60
ttatactgtg aaactgcgaa cggctcatta tatcagttat aatcccttcg gtagttcctt 120
tacacggata cctgcagtaa ttctggaatt aatacgtgct gtacgggccc gactttcggg 180
gagggccgca cttattaggt ctaagccaac gttcttggtg agtcatgata attgagcaga 240
tcgcttttcg gagcgatgaa tcgtttgagt ttctgcccca tcagttgtcg acggtagggt 300
attggcctac ggtgactata acgggtgacg gggagttagg gctcgactcc ggagagggag 360
cctgagagac ggctaccaca tccaaggaag gcagcaggcg cgtaaattac ccaatgtgga 420
ctccacgagg tagtgacgag aaatatcaat gcggggcgct tcgcgtcttg ctattggaat 480
gagagcaatg taaaaccctc atcgaggatc aactggaggg caagtctggt gccagcagcc 540
gcggtaattc cagctccaga agcgtatgct aaagttgttg cagttaaaaa gctcgtagtt 600
gaatttctgg tgtgggagcc caggcctcgg tgcgaatgcg ccttgtattg ccttgcggct 660
cctttgccat cctcgttttt cttttgaaga acgtcattca ctgtaatcaa agcagagtgt 720
tccaagcagg ccgtagggcc ggtatgttta ttatgggatg atcagatagg actcgggtgc 780
tattttgttg gtttgcacat ctgagtaatg attaatagga acagtcgggg gtatccgtat 840
ttaggagcta gaggtgaaat tcttggattt ccgaaagacg aactacagcg aaggcattta 900
ccaagcatgt tttcattaat caagaacgaa agtctgggga tcgaagatga ttagatacca 960
tcgtagtcta gaccgtaaac gatgccgact tgcgattgcg ggtggcttgt attgggcttc 1020
cgcagcagca catgagaaat caaagtcttt gggttccggg gggagtatgg tcgcaaggct 1080
gaaacttaaa ggaattgacg gaagggcacc accaggagtg gagcctgcgg cttaatttga 1140
ctcaacacgg gaaaacttac caggtccaga cataggtagg atgacagatg agagctcttt 1200
cttgattcta tgggtggtgg tgcatggccg ttcttagttg gtggagtgat ttgtctggtt 1260
aattccgtta acgaacgaga cctcggccta ctaaatagcc gggcgtatgg cgacatatgt 1320
gtttgtggct tcttagaggg acatgttcgg tttacgagca ggaagttcga ggcaataaca 1380
ggtctgtgat gcccttaaat gttctgggcc gcacgcgcgc tacactgatg ggttcagcgg 1440
gtctttgttg ngtttactca cagcgttgct ttgtcggaag gcatggctaa tcctttgaac 1500
gcccatcgtg ctggggctag atttttgcaa ttattaatct ccaacgagga attcctagta 1560
aacgcaagtc atcagcttgc attgaatacg tccctgccct ttgtacacac cgcccgtcgc 1620
acctaccgat tgaacggtcc gatgaaacca tgggactacc ttttgagcgt ttgttcgcga 1680
tggaggtggg aactcgggtg aatcttattg t 1711

<210> 7
<211> 1011
<212> DNA
<213> Aurantiochytrium sp.1

<400> 7

atggtcgttc tctgggagaa gggcgagaac cactcgtcgg accagttcgt tggcgccatc 60
aaccatgcca tgaagaacac cgtggacaac gaggacaagg acaagaacta cgcacccgtg 120
gtcaagagct tctacgcctc ggtcgccacc agcctctacg agtacggctg gggcaagtcc 180
ttccacttcg ccccgcgcaa caagggcgag ggcttcaagg agtccctcct ccgccaggag 240
cactggctcg ccgcccagct cggcatccgc cccggccagg atgtctgtga cctcggctgc 300
ggtgtctgcg gccccctcgt caacatcgcc aagttctccc gcgccaacat caccggcgtg 360
accatctccg acttccagat cgcccgcggc aacgcctgga tcgcccagaa cggcctctcc 420
aaccgctgca aggccgtcga gggtgacttc cacgagctcc ccttcgagga caactccttc 480
gactgcggct acgactgcga ggccaccgcc cactccacca agctcaacac cttcttcgcc 540
gaggtcgagc gcgtcctccg ccccggcggt gtcttcgccg gcttcgcctg ggtcaccctc 600
cccaagcaca acccggagga ccccgaggag gtcaagattc tcgaggacct tgcctttggc 660
aacgccattg ccgtcctcca cagctttgac gactacgtca acgccatcaa ggccaacccc 720
ggtctcgagc tcgtcgaggc ctacgacgcc aacaagctcg gcgacgacat ggagtggcac 780
gagcccctca agcctggctt ctctatcgac ggcatcctca tgtcctgggc tggccgccag 840
ctcaccgcga acctcgcccg cgtcaccgag gccatcggcc ttgcccccaa gggcacctac 900
aacgcctccc aggtcctcga ggttgctgcc cgctccctcg tcgacgccgg cgaccgcggt 960
atctacaccc ccatctacta ctacaaggtc cgcaaggtcg ccaagaacta a 1011

<210> 8
<211> 40
<212> DNA
<213> Artificial Sequence

<220>
<223> primer

<400> 8
ttgggtaacg ccaggggagg ctgcctgcgc tggaatgcga 40

<210> 9
<211> 43
<212> DNA
<213> Artificial Sequence

<220>
<223> primer

<400> 9
cgtgactggg aaaacttgtc ctcgttgtcc acggtgttct tca 43

<210> 10
<211> 41
<212> DNA
<213> Artificial Sequence

<220>
<223> primer

<400> 10
catagctgtt tcctgacaag aactacgcac ccgtggtcaa g 41

<210> 11
<211> 41
<212> DNA
<213> Artificial Sequence

<220>
<223> primer

<400>  11
gataacaatt tcacattagt tcttggcgac cttgcggacc t                          41


<210>  12
<211>  17
<212>  DNA
<213>  Artificial Sequence

<220>
<223>  primer

<400>  12
gttttcccag tcacgac                                                     17


<210>  13
<211>  17
<212>  DNA
<213>  Artificial Sequence

<220>
<223>  primer

<400>  13
caggaaacag ctatgac                                                     17


<210>  14
<211>  25
<212>  DNA
<213>  Artificial Sequence

<220>
<223>  primer

<400>  14
cctggcgtta cccaacttaa tcgcc                                            25


<210>  15
<211>  28
<212>  DNA
<213>  Artificial Sequence

<220>
<223>  primer

<400>  15
tgtgaaattg ttatccgctc acaattcc                                         28


<210>  16
<211>  25
<212>  DNA
<213>  Artificial Sequence

<220>
<223>  primer

<400>  16
ggaggctgcc tgcgctggaa tgcga                                            25

<210>  17
<211>  26
<212>  DNA
<213>  Artificial Sequence

<220>
<223>  primer

<400>  17
ttagttcttg gcgaccttgc ggacct                                          26


<210>  18
<211>  1671
<212>  DNA
<213>  Aurantiochytrium sp.1

<400>  18
ttgtacgagg cgcgtgcccg gggcagaagg cgcaagcagg caggcaaacg cgatctccga      60
ccgcgtgcgc atcgtgcgtg cgagcgtggg cgcgagagac ctcctcgccg ggcggcaaat     120
ccgcaacgag gccagccccg agcagcgagg gcagcagcga gggagggagg agcagcggcg     180
acggagagac agccagagaa acagacgaga aagcagacgc ggaaggcacg cgcgcgaagc     240
aggatggcgg ccggaaagca gggaggcgcc aaggccaagg gcctcgcgcc cgcgggcagc     300
gccgcagcag ccgccaagaa gaagtcggaa atgtggagcg gagcgtccac ctccgagagc     360
gtgggcatcg gccccgaggc cctgcgcgcc acgctcatgc cggttctgct cgtactttcc     420
acgccgctca ccagcatcat cctgagccga agtgtcgtca ccaccaagca cgactcctcg     480
ttcctcgaaa atgcatggga cgtcatcgag gagatcctcg aaaagggcat cgtctacacg     540
tacacccgcg acgccttcaa ccccttcgtg tggaagatga tcggcaccta catggtcgtc     600
cagctcctgc tcatgcgatt catgcccgga cccacgtacg agggcccgca aagccccatg     660
ggcaaccgcc ccatctacaa ggacaactgc tttgcctgct acgtggcctc ctttgccctc     720
tacgggctcg gcgtgtactc tggcctcttc aacggtggca tcgcctttga ctactttcag     780
gaattcattg cctccatgaa catcttcgcc ctcatctttg tggctatcct ctacgtcaag     840
ggcgccgtgg ctccctccag ctccgactcg ggcctctcgg gtaacatcgc ctttgactac     900
tactggggca ccgagctgta cccgcgcatc ctcggctggg acgtcaaggt ctttaccaac     960
tgccgctatg gcatgactgg ctgggccctg ctctgcacct cgttcgccta cgcgcagtac    1020
gagcgctttg gcgaggtcag caactcaatc ctcattgccg ccgctctcca ggtcatctac    1080
ctggccaagt tccacatttg ggagcgtggc tacatgttca ccattgacat catgcacgac    1140
cgcgccggct actacatttg ctggggctgc ctcgtctggg tccccagcct ctacgccgca    1200
cccacggcct ttctcgtgct ccacccgtac aactttccca cttgggccgc cgcggcgcag    1260
ttcgtctttt gcctgctctc catcttcctc aactacgacg tcgaccgtca gcgccaggaa    1320
tttcgcgcca aggacggcaa gatgaagatc tggggcaagg acgccgagta cctcgtcgcc    1380
gagtacacga cgagcgacgg caagaagcac tcctccatgc ttttgcactc gggctggtgg    1440
ggtcaggctc gcaagatcaa ctactttttc gagctttgcg ctgccttttc ctggtctgtc    1500
attgttgccc acccctttca ccttctggca tgggtctact ttgcctttct ctttattctc    1560
ctcatcgacc gtgcctggcg tgacgacgcg cgctgtgcat ccaagtatgg cgaaaagtgg    1620
gacgagtaca aggtcaaggt cccctacctg atcatcccgg gtgtctacta a             1671


<210>  19
<211>  38
<212>  DNA
<213>  Artificial Sequence

<220>
<223>  primer

<400>  19
ttgggtaacg ccaggggtgc gccgggccgc tgttcttc                             38


<210>  20
<211>  39

<212>  DNA
<213>  Artificial Sequence

<220>
<223>  primer

<400>  20
cgtgactggg aaaacgaagg cgtcgcgggt gtacgtgta                              39


<210>  21
<211>  42
<212>  DNA
<213>  Artificial Sequence

<220>
<223>  primer

<400>  21
catagctgtt tcctggtatt gtgtctagaa ttttcactcg gt                          42


<210>  22
<211>  39
<212>  DNA
<213>  Artificial Sequence

<220>
<223>  primer

<400>  22
gataacaatt tcacaggcgc aaagatgcgg tcatcatgt                              39


<210>  23
<211>  17
<212>  DNA
<213>  Artificial Sequence

<220>
<223>  primer

<400>  23
gttttcccag tcacgac                                                      17


<210>  24
<211>  17
<212>  DNA
<213>  Artificial Sequence

<220>
<223>  primer

<400>  24
caggaaacag ctatgac                                                      17


<210>  25
<211>  25
<212>  DNA
<213>  Artificial Sequence

<220>
<223>  primer

<400>  25
cctggcgtta cccaacttaa tcgcc                                          25


<210>  26
<211>  28
<212>  DNA
<213>  Artificial Sequence

<220>
<223>  primer

<400>  26
tgtgaaattg ttatccgctc acaattcc                                       28


<210>  27
<211>  23
<212>  DNA
<213>  Artificial Sequence

<220>
<223>  primer

<400>  27
ggtgcgccgg gccgctgttc ttc                                            23


<210>  28
<211>  24
<212>  DNA
<213>  Artificial Sequence

<220>
<223>  primer

<400>  28
ggcgcaaaga tgcggtcatc atgt                                           24


<210>  29
<211>  1572
<212>  DNA
<213>  Aurantiochytrium sp.1

<400>  29
atgacggagc agcggggcat cttcgtgaca gtcttcctga tgcccgtgag cgtggtctgg      60
gacatcatgt tcgcactgcg ctcgtggctc atcatggcgc tgtactccgc gccagagctg     120
cacaaacagc gtgtgcagga agcctccgct caggtccgtg acgccacgac caacgccccg     180
cccggcacca agctgtgcac cgcgcgcggg gggtggctct ccatctcgct cagtctgcgg     240
ccgtacaaaa aggtggccac caacatctcc attaacatgt acgacatcct cgagcttaac     300
gaaaccgaaa gctatgtcca tgtcgagccc atggtcaaca tgggccagct tagccactac     360
ctcatccctc gcggctacac catcccggtg ctccccgaaa tggacgacct caccgtcgga     420
ggtctcttca tgggcaccgg catcgaggcc agctcgcaca aatttggtct ctttgacgag     480
ttcgtcatcg aggccgaggt catcctcgcc tccggcgagg tcgtcacttg cagcaagacc     540
cagaaccgcg acctcttcga cgcgctcccc tggtcgtacg gcacgctcgg cttcctcgtc     600
tcctgcaaga tcaaggtcat cccttgcaag ccctttatcc gcatcgagta ccgcggctgc     660
cacaacaaaa agcagggcct cgagcttttc accaaggcct cctgcatgga catcccgccc     720
gactttgtcg agtgcatcgc ctactcatcc gagacaatgg tagttatgac ctccgatatg     780
gtcgacgaag atgccgtcga aaaggacaag atcaacaaga tcggccgctg gtacaaaccc     840

tggtacttca agcacgtcga gaagatgctc gagatctcct cgcccgaaaa gccccacgtc 900
gagtacatcc ccatccgcca gttcttccac cgctggacct cggccatctt ctgggagctc 960
gagcagatca tcccctttgg caaccatccg ctctaccgcg ctctcctcgg atggttcaca 1020
ccttcggtcg ccctgcttaa actcacgcag acggaggcca ttcgcaagac ctacgaggag 1080
cagcacattg tccaggacat gctcgtgcct atctcaaaga cctccgaggc gctcgatgtc 1140
tttgacacgc actaccaaat ttaccccctc tggatctgcc cctaccgcca gtacgactac 1200
agcgacgcca ccacggagca ccgccacttt cttcgcaagc ccaacaagcc caacaaggag 1260
ggctacgaga tgtatgtcga tctcggcgcc tacggtgtcc cgcgcccctg cctcgagaag 1320
aagccttttg atgttatcaa gtcctctcgc gctgtcgaaa agtatgtcac cgaagccaat 1380
ggtagccaga tgctctacgc caccagctac ctcagccgcg atgagttcag ggagatgttc 1440
aaccatgcac actacgacaa gatgaagcaa aaggttgacc ccaacgactt ctttcctcag 1500
gtctacgaga aggtctgcaa gggcgccatg aacttttggc aggcaaaagc tggtgacaag 1560
aagcttgact ga 1572

<210> 30
<211> 34
<212> DNA
<213> Artificial Sequence

<220>
<223> primer

<400> 30
ttgggtaacg ccaggggact gctgcagctg caag 34

<210> 31
<211> 35
<212> DNA
<213> Artificial Sequence

<220>
<223> primer

<400> 31
cgtgactggg aaaacatgaa gttcaccgcc atgtc 35

<210> 32
<211> 34
<212> DNA
<213> Artificial Sequence

<220>
<223> primer

<400> 32
catagctgtt tcctgtgcat cgcctactca tccg 34

<210> 33
<211> 39
<212> DNA
<213> Artificial Sequence

<220>
<223> primer

<400> 33
gataacaatt tcacagaaca aattgagacg agttacgag 39

<210>  34
<211>  17
<212>  DNA
<213>  Artificial Sequence

<220>
<223>  primer

<400>  34
gttttcccag tcacgac                                                      17


<210>  35
<211>  17
<212>  DNA
<213>  Artificial Sequence

<220>
<223>  primer

<400>  35
caggaaacag ctatgac                                                      17


<210>  36
<211>  25
<212>  DNA
<213>  Artificial Sequence

<220>
<223>  primer

<400>  36
cctggcgtta cccaacttaa tcgcc                                             25


<210>  37
<211>  28
<212>  DNA
<213>  Artificial Sequence

<220>
<223>  primer

<400>  37
tgtgaaattg ttatccgctc acaattcc                                          28


<210>  38
<211>  336
<212>  PRT
<213>  Aurantiochytrium sp.1

<400>  38
Met Val Val Leu Trp Glu Lys Gly Glu Asn His Ser Ser Asp Gln Phe
1               5                   10                  15
Val Gly Ala Ile Asn His Ala Met Lys Asn Thr Val Asp Asn Glu Asp
            20                  25                  30
Lys Asp Lys Asn Tyr Ala Pro Val Val Lys Ser Phe Tyr Ala Ser Val
        35                  40                  45
Ala Thr Ser Leu Tyr Glu Tyr Gly Trp Gly Lys Ser Phe His Phe Ala
    50                  55                  60
Pro Arg Asn Lys Gly Glu Gly Phe Lys Glu Ser Leu Leu Arg Gln Glu

65                  70                  75                  80
His Trp Leu Ala Ala Gln Leu Gly Ile Arg Pro Gly Gln Asp Val Cys
                85                  90                  95
Asp Leu Gly Cys Gly Val Cys Gly Pro Leu Val Asn Ile Ala Lys Phe
            100                 105                 110
Ser Arg Ala Asn Ile Thr Gly Val Thr Ile Ser Asp Phe Gln Ile Ala
        115                 120                 125
Arg Gly Asn Ala Trp Ile Ala Gln Asn Gly Leu Ser Asn Arg Cys Lys
    130                 135                 140
Ala Val Glu Gly Asp Phe His Glu Leu Pro Phe Glu Asp Asn Ser Phe
145                 150                 155                 160
Asp Cys Gly Tyr Asp Cys Glu Ala Thr Ala His Ser Thr Lys Leu Asn
                165                 170                 175
Thr Phe Phe Ala Glu Val Glu Arg Val Leu Arg Pro Gly Gly Val Phe
            180                 185                 190
Ala Gly Phe Ala Trp Val Thr Leu Pro Lys His Asn Pro Glu Asp Pro
        195                 200                 205
Glu Glu Val Lys Ile Leu Glu Asp Leu Ala Phe Gly Asn Ala Ile Ala
    210                 215                 220
Val Leu His Ser Phe Asp Asp Tyr Val Asn Ala Ile Lys Ala Asn Pro
225                 230                 235                 240
Gly Leu Glu Leu Val Glu Ala Tyr Asp Ala Asn Lys Leu Gly Asp Asp
                245                 250                 255
Met Glu Trp His Glu Pro Leu Lys Pro Gly Phe Ser Ile Asp Gly Ile
            260                 265                 270
Leu Met Ser Trp Ala Gly Arg Gln Leu Thr Ala Asn Leu Ala Arg Val
        275                 280                 285
Thr Glu Ala Ile Gly Leu Ala Pro Lys Gly Thr Tyr Asn Ala Ser Gln
    290                 295                 300
Val Leu Glu Val Ala Ala Arg Ser Leu Val Asp Ala Gly Asp Arg Gly
305                 310                 315                 320
Ile Tyr Thr Pro Ile Tyr Tyr Tyr Lys Val Arg Lys Val Ala Lys Asn
                325                 330                 335


<210>  39
<211>  556
<212>  PRT
<213>  Aurantiochytrium sp.1

<400>  39
Leu Tyr Glu Ala Arg Ala Arg Gly Arg Arg Arg Lys Gln Ala Gly Lys
1               5                   10                  15
Arg Asp Leu Arg Pro Arg Ala His Arg Ala Cys Glu Arg Gly Arg Glu
            20                  25                  30
Arg Pro Pro Arg Arg Ala Ala Asn Pro Gln Arg Gly Gln Pro Arg Ala
        35                  40                  45
Ala Arg Ala Ala Ala Arg Glu Gly Gly Ala Ala Ala Thr Glu Arg Gln
    50                  55                  60
Pro Glu Lys Gln Thr Arg Lys Gln Thr Arg Lys Ala Arg Ala Arg Ser
65                  70                  75                  80
Arg Met Ala Ala Gly Lys Gln Gly Gly Ala Lys Ala Lys Gly Leu Ala
                85                  90                  95
Pro Ala Gly Ser Ala Ala Ala Ala Ala Lys Lys Lys Ser Glu Met Trp
            100                 105                 110
Ser Gly Ala Ser Thr Ser Glu Ser Val Gly Ile Gly Pro Glu Ala Leu
        115                 120                 125
Arg Ala Thr Leu Met Pro Val Leu Leu Val Leu Ser Thr Pro Leu Thr
    130                 135                 140
Ser Ile Ile Leu Ser Arg Ser Val Val Thr Thr Lys His Asp Ser Ser
145                 150                 155                 160
Phe Leu Glu Asn Ala Trp Asp Val Ile Glu Glu Ile Leu Glu Lys Gly
                165                 170                 175

Ile Val Tyr Thr Tyr Thr Arg Asp Ala Phe Asn Pro Phe Val Trp Lys
            180                 185                 190
Met Ile Gly Thr Tyr Met Val Val Gln Leu Leu Leu Met Arg Phe Met
        195                 200                 205
Pro Gly Pro Thr Tyr Glu Gly Pro Gln Ser Pro Met Gly Asn Arg Pro
    210                 215                 220
Ile Tyr Lys Asp Asn Cys Phe Ala Cys Tyr Val Ala Ser Phe Ala Leu
225                 230                 235                 240
Tyr Gly Leu Gly Val Tyr Ser Gly Leu Phe Asn Gly Gly Ile Ala Phe
                245                 250                 255
Asp Tyr Phe Gln Glu Phe Ile Ala Ser Met Asn Ile Phe Ala Leu Ile
            260                 265                 270
Phe Val Ala Ile Leu Tyr Val Lys Gly Ala Val Ala Pro Ser Ser Ser
        275                 280                 285
Asp Ser Gly Leu Ser Gly Asn Ile Ala Phe Asp Tyr Tyr Trp Gly Thr
    290                 295                 300
Glu Leu Tyr Pro Arg Ile Leu Gly Trp Asp Val Lys Val Phe Thr Asn
305                 310                 315                 320
Cys Arg Tyr Gly Met Thr Gly Trp Ala Leu Leu Cys Thr Ser Phe Ala
                325                 330                 335
Tyr Ala Gln Tyr Glu Arg Phe Gly Glu Val Ser Asn Ser Ile Leu Ile
            340                 345                 350
Ala Ala Ala Leu Gln Val Ile Tyr Leu Ala Lys Phe His Ile Trp Glu
        355                 360                 365
Arg Gly Tyr Met Phe Thr Ile Asp Ile Met His Asp Arg Ala Gly Tyr
    370                 375                 380
Tyr Ile Cys Trp Gly Cys Leu Val Trp Val Pro Ser Leu Tyr Ala Ala
385                 390                 395                 400
Pro Thr Ala Phe Leu Val Leu His Pro Tyr Asn Phe Pro Thr Trp Ala
                405                 410                 415
Ala Ala Ala Gln Phe Val Phe Cys Leu Leu Ser Ile Phe Leu Asn Tyr
            420                 425                 430
Asp Val Asp Arg Gln Arg Gln Glu Phe Arg Ala Lys Asp Gly Lys Met
        435                 440                 445
Lys Ile Trp Gly Lys Asp Ala Glu Tyr Leu Val Ala Glu Tyr Thr Thr
    450                 455                 460
Ser Asp Gly Lys Lys His Ser Ser Met Leu Leu His Ser Gly Trp Trp
465                 470                 475                 480
Gly Gln Ala Arg Lys Ile Asn Tyr Phe Phe Glu Leu Cys Ala Ala Phe
                485                 490                 495
Ser Trp Ser Val Ile Val Ala His Pro Phe His Leu Leu Ala Trp Val
            500                 505                 510
Tyr Phe Ala Phe Leu Phe Ile Leu Leu Ile Asp Arg Ala Trp Arg Asp
        515                 520                 525
Asp Ala Arg Cys Ala Ser Lys Tyr Gly Glu Lys Trp Asp Glu Tyr Lys
    530                 535                 540
Val Lys Val Pro Tyr Leu Ile Ile Pro Gly Val Tyr
545                 550                 555


<210>  40
<211>  523
<212>  PRT
<213>  Aurantiochytrium sp.1

<400>  40
Met Thr Glu Gln Arg Gly Ile Phe Val Thr Val Phe Leu Met Pro Val
1               5                   10                  15
Ser Val Val Trp Asp Ile Met Phe Ala Leu Arg Ser Trp Leu Ile Met
            20                  25                  30
Ala Leu Tyr Ser Ala Pro Glu Leu His Lys Gln Arg Val Gln Glu Ala
        35                  40                  45
Ser Ala Gln Val Arg Asp Ala Thr Thr Asn Ala Pro Pro Gly Thr Lys

50                  55                  60
Leu Cys Thr Ala Arg Gly Gly Trp Leu Ser Ile Ser Leu Ser Leu Arg
65                  70                  75                  80
Pro Tyr Lys Lys Val Ala Thr Asn Ile Ser Ile Asn Met Tyr Asp Ile
                85                  90                  95
Leu Glu Leu Asn Glu Thr Glu Ser Tyr Val His Val Glu Pro Met Val
            100                 105                 110
Asn Met Gly Gln Leu Ser His Tyr Leu Ile Pro Arg Gly Tyr Thr Ile
        115                 120                 125
Pro Val Leu Pro Glu Met Asp Asp Leu Thr Val Gly Gly Leu Phe Met
    130                 135                 140
Gly Thr Gly Ile Glu Ala Ser Ser His Lys Phe Gly Leu Phe Asp Glu
145                 150                 155                 160
Phe Val Ile Glu Ala Glu Val Ile Leu Ala Ser Gly Glu Val Val Thr
                165                 170                 175
Cys Ser Lys Thr Gln Asn Arg Asp Leu Phe Asp Ala Leu Pro Trp Ser
            180                 185                 190
Tyr Gly Thr Leu Gly Phe Leu Val Ser Cys Lys Ile Lys Val Ile Pro
        195                 200                 205
Cys Lys Pro Phe Ile Arg Ile Glu Tyr Arg Gly Cys His Asn Lys Lys
    210                 215                 220
Gln Gly Leu Glu Leu Phe Thr Lys Ala Ser Cys Met Asp Ile Pro Pro
225                 230                 235                 240
Asp Phe Val Glu Cys Ile Ala Tyr Ser Ser Glu Thr Met Val Val Met
                245                 250                 255
Thr Ser Asp Met Val Asp Glu Asp Ala Val Glu Lys Asp Lys Ile Asn
            260                 265                 270
Lys Ile Gly Arg Trp Tyr Lys Pro Trp Tyr Phe Lys His Val Glu Lys
        275                 280                 285
Met Leu Glu Ile Ser Ser Pro Glu Lys Pro His Val Glu Tyr Ile Pro
    290                 295                 300
Ile Arg Gln Phe Phe His Arg Trp Thr Ser Ala Ile Phe Trp Glu Leu
305                 310                 315                 320
Glu Gln Ile Ile Pro Phe Gly Asn His Pro Leu Tyr Arg Ala Leu Leu
                325                 330                 335
Gly Trp Phe Thr Pro Ser Val Ala Leu Leu Lys Leu Thr Gln Thr Glu
            340                 345                 350
Ala Ile Arg Lys Thr Tyr Glu Glu Gln His Ile Val Gln Asp Met Leu
        355                 360                 365
Val Pro Ile Ser Lys Thr Ser Glu Ala Leu Asp Val Phe Asp Thr His
    370                 375                 380
Tyr Gln Ile Tyr Pro Leu Trp Ile Cys Pro Tyr Arg Gln Tyr Asp Tyr
385                 390                 395                 400
Ser Asp Ala Thr Thr Glu His Arg His Phe Leu Arg Lys Pro Asn Lys
                405                 410                 415
Pro Asn Lys Glu Gly Tyr Glu Met Tyr Val Asp Leu Gly Ala Tyr Gly
            420                 425                 430
Val Pro Arg Pro Cys Leu Glu Lys Lys Pro Phe Asp Val Ile Lys Ser
        435                 440                 445
Ser Arg Ala Val Glu Lys Tyr Val Thr Glu Ala Asn Gly Ser Gln Met
    450                 455                 460
Leu Tyr Ala Thr Ser Tyr Leu Ser Arg Asp Glu Phe Arg Glu Met Phe
465                 470                 475                 480
Asn His Ala His Tyr Asp Lys Met Lys Gln Lys Val Asp Pro Asn Asp
                485                 490                 495
Phe Phe Pro Gln Val Tyr Glu Lys Val Cys Lys Gly Ala Met Asn Phe
            500                 505                 510
Trp Gln Ala Lys Ala Gly Asp Lys Lys Leu Asp
        515                 520

<210>  41
<211>  1479

<212>  DNA
<213>  Aurantiochytrium sp.1

<400>  41
atggccgccg catccaccgc cgccgccgtc acaccggcgc gctcgatctt tcgcaaggtc      60
gccgtcgtga tcgtccttct gatggccctc gacatgatcc gctaccactt taagcgcatg     120
aagatgaagg cacgggcaag gtggttctcg ctccccttcc tcggagactt tcctaccatc     180
gtggccgacc ccaccaaatt ctgggataac ctgatcgccc ttgccccgaa cggtatctcg     240
gcctttaaga tcctcacgca ggctgccatc ttcgtgacga accccaagtt cgccaagcag     300
atttttgacg accaggaaaa ctttactctt tttgtgcacc cgaactcgcc caagctgctc     360
ggcgaagaca acctcgtgta catgtctcgc gaggacctcg ttcgctggcg ccgcgccatg     420
gtgcccaacc tcaaaaaggc cgaagtcctg catgccgccg cctacactgc cgtcaatagc     480
tgctcgaaca tgcttcgcaa gatcgccgct cgctcgcccg ctgagcaggc cgaaaacgac     540
gttcgctacg acatccgcct catgaacgcc cacctgtcac tcaaggtctt cctcggaggc     600
tacttgcaaa agggcgaggc agagtggatc gctgacgacc tcatcgttgt cactaacggc     660
ttcatcgcgg cccccgttga ccttcccttc actgacctcg gcaagggcaa ggctgctagc     720
cagcgcatca tgaaggtgct tcaccgcatc tcggacgagt gcttccgccg catgaagaac     780
aacgaggagc ctctctgcgt tcttgaccgc atcacggact cgtgcataaa ggcaggaatc     840
aagggttacg gccgcacccc ggcagatatt gcccacgctg gcctcgactt ttgctttgcg     900
gcccaggatg ccaccacctc cgccctttgc tggtgcatcc actcgatgga gaagcaccca     960
gaggtcgccg acaagattcg cgaggagctt gaccgcgtcc ttgacaagga cgtcgacttc    1020
accgaggctc ttccccgtgc ctacaacgcc aacaagcttc cctaccttcg cgccgccgcc    1080
attaaccagc tgcagtaccg cccacccgtg cccatggtcc cgcatattgc gcgccatgac    1140
actgagcttg gcgaccacaa gattgaaaag ggcaccattg ctttcgcctc tgtcatgggc    1200
tccaaggcca acattgaggg cgctgaccgt ttcgagcctg atccggataa catgcttgac    1260
cctgccttca agaaggttct ctgctttggc ggcggcccac acatgtgccc gggtcgcttt    1320
tacgccagca acactgttgc tctgttcatg gccacgcttg tccacggcta cgacgttgac    1380
cgcatccgca agcctggcgg cgaggacatc atctacctgc ccacactcta cccgcgtgat    1440
tcgctttacg tcatgaagtc tcgcgccgag gccgcatga                           1479

<210>  42
<211>  492
<212>  PRT
<213>  Aurantiochytrium sp.1

<400>  42
Met Ala Ala Ala Ser Thr Ala Ala Ala Val Thr Pro Ala Arg Ser Ile
1               5                   10                  15
Phe Arg Lys Val Ala Val Val Ile Val Leu Leu Met Ala Leu Asp Met
            20                  25                  30
Ile Arg Tyr His Phe Lys Arg Met Lys Met Lys Ala Arg Ala Arg Trp
        35                  40                  45
Phe Ser Leu Pro Phe Leu Gly Asp Phe Pro Thr Ile Val Ala Asp Pro
    50                  55                  60
Thr Lys Phe Trp Asp Asn Leu Ile Ala Leu Ala Pro Asn Gly Ile Ser
65                  70                  75                  80
Ala Phe Lys Ile Leu Thr Gln Ala Ala Ile Phe Val Thr Asn Pro Lys
                85                  90                  95
Phe Ala Lys Gln Ile Phe Asp Asp Gln Glu Asn Phe Thr Leu Phe Val
            100                 105                 110
His Pro Asn Ser Pro Lys Leu Leu Gly Glu Asp Asn Leu Val Tyr Met
        115                 120                 125
Ser Arg Glu Asp Leu Val Arg Trp Arg Arg Ala Met Val Pro Asn Leu
    130                 135                 140
Lys Lys Ala Glu Val Leu His Ala Ala Ala Tyr Thr Ala Val Asn Ser
145                 150                 155                 160
Cys Ser Asn Met Leu Arg Lys Ile Ala Ala Arg Ser Pro Ala Glu Gln
                165                 170                 175
Ala Glu Asn Asp Val Arg Tyr Asp Ile Arg Leu Met Asn Ala His Leu
            180                 185                 190
Ser Leu Lys Val Phe Leu Gly Gly Tyr Leu Gln Lys Gly Glu Ala Glu
        195                 200                 205

Trp Ile Ala Asp Asp Leu Ile Val Val Thr Asn Gly Phe Ile Ala Ala
    210                 215                 220
Pro Val Asp Leu Pro Phe Thr Asp Leu Gly Lys Gly Lys Ala Ala Ser
225                 230                 235                 240
Gln Arg Ile Met Lys Val Leu His Arg Ile Ser Asp Glu Cys Phe Arg
                245                 250                 255
Arg Met Lys Asn Asn Glu Glu Pro Leu Cys Val Leu Asp Arg Ile Thr
            260                 265                 270
Asp Ser Cys Ile Lys Ala Gly Ile Lys Gly Tyr Gly Arg Thr Pro Ala
        275                 280                 285
Asp Ile Ala His Ala Gly Leu Asp Phe Cys Phe Ala Ala Gln Asp Ala
    290                 295                 300
Thr Thr Ser Ala Leu Cys Trp Cys Ile His Ser Met Glu Lys His Pro
305                 310                 315                 320
Glu Val Ala Asp Lys Ile Arg Glu Glu Leu Asp Arg Val Leu Asp Lys
                325                 330                 335
Asp Val Asp Phe Thr Glu Ala Leu Pro Arg Ala Tyr Asn Ala Asn Lys
            340                 345                 350
Leu Pro Tyr Leu Arg Ala Ala Ala Ile Asn Gln Leu Gln Tyr Arg Pro
        355                 360                 365
Pro Val Pro Met Val Pro His Ile Ala Arg His Asp Thr Glu Leu Gly
    370                 375                 380
Asp His Lys Ile Glu Lys Gly Thr Ile Ala Phe Ala Ser Val Met Gly
385                 390                 395                 400
Ser Lys Ala Asn Ile Glu Gly Ala Asp Arg Phe Glu Pro Asp Pro Asp
                405                 410                 415
Asn Met Leu Asp Pro Ala Phe Lys Lys Val Leu Cys Phe Gly Gly Gly
            420                 425                 430
Pro His Met Cys Pro Gly Arg Phe Tyr Ala Ser Asn Thr Val Ala Leu
        435                 440                 445
Phe Met Ala Thr Leu Val His Gly Tyr Asp Val Asp Arg Ile Arg Lys
    450                 455                 460
Pro Gly Gly Glu Asp Ile Ile Tyr Leu Pro Thr Leu Tyr Pro Arg Asp
465                 470                 475                 480
Ser Leu Tyr Val Met Lys Ser Arg Ala Glu Ala Ala
                485                 490


<210>  43
<211>  1782
<212>  DNA
<213>  Aurantiochytrium sp.1

<400>  43
aacctggttg atcctgccag tagccctacg ctcgtctcaa agactaagcc atgcatgtgt      60
aagtataagc gaattatact gtgaaactgc gaacggctca ttatatcagt tataatccct     120
tcggtagttc ctttagacgg atacctgcag taattctgga attaatacgt gctgtacggg     180
cccgactttc ggggagggcc gcacttatta ggtctaagcc aacgttcttg gtgagtcatg     240
ataattgagc agatcgcttt tcggagcgat gaatcgtttg agtttctgcc ccatcagttg     300
tcgacggtag ggtattggcc tacggtgact ataacgggtg acggggagtt agggctcgac     360
tccggagagg gagcctgaga gacggctacc acatccaagg aaggcagcag gcgcgtaaat     420
tacccaatgt ggactccacg aggtagtgac gagaaatatc aatgcggggc gcttcgcgtc     480
ttgctattgg aatgagagca atgtaaaacc ctcatcgagg atcaactgga gggcaagtct     540
ggtgccagca gccgcggtaa ttccagctcc agaagcgtat gctaaagttg ttgcagttaa     600
aaagctcgta gttgaatttc tggtgtggga gcccaggcct cggtgcgaat gcgccttgta     660
ttgccttgcg gctcctttgc catcctcgtt tttctttaga aggacgtcat tcactgtaat     720
caaagcagag tgttccaagc aggccgtagg gccggtatgt ttattatggg atgatcagat     780
aggactcggg tgctattttg ttggtttgca catctgagta atgattaata ggaacagtcg     840
ggggtatccg tatttaggag ctagaggtga aattcttgga tttccgagag acgaactaca     900
gcgaaggcat ttaccaagca tgttttcatt aatcaagaac gaaagtctgg ggatcgaaga     960
tgattagata ccatcgtagt ctagaccgta aacgatgccg acttgcgatt gcgggcagct    1020
tgtattgggc ttccgcagca gcacatgaga aatcaaagtc tttgggttcc ggggggagta    1080
tggtcgcaag gctgaaactt aaaggaattg acggaagggc accaccagga gtggagcctg    1140

cggcttaatt tgactcaaca cgggaaaact taccaggtcc agacataggt aggattgaca 1200
gattgagagc tctttcttga ttctatgggt ggtggtgcat ggccgttctt agttggtgga 1260
gtgatttgtc tggttaattc cgttaacgaa cgagacctcg gcctactaaa tagccgggcg 1320
tatggcgaca tatgtgtttg tggcttctta gagggacatg ttcggtttac gagcaggaag 1380
ttcgaggtaa taacaggtct gtgatgccct tagatgttct gggccgcacg cgcgctacac 1440
tgatgggttc agcgggtctt tgttgtgttt actcacagcg ttgctttgtc ggaaggcatg 1500
gctaatcctt tgaacgccca tcgtgctggg gctagatttt tgcaattatt aatctccaac 1560
gaggaattcc tagtaaacgc aagtcatcag cttgcattga atacgtccct gccctttgta 1620
cacaccgccc gtcgcaccta ccgattgaac ggtccgatga aaccatggga ctaccttttg 1680
agcgtttgtt cgcgatggag gtgggaactc gggtgaatct tattgtttag aggaaggtga 1740
agtcgtaaca aggtttccgt aggtgaacct gcagaaggat ca 1782

<210> 44
<211> 1761
<212> DNA
<213> Aurantiochytrium sp.1

<400> 44
ccttccgcag gttcacctac ggaaaccttg ttacgacttc accttcctct aaacaataag 60
attcacccga gttcccacct ccatcgcgaa caaacgctca aaaggtagtc ccatggtttc 120
atcggaccgt tcaatcggta ggtgcgacgg gcggtgtgta caaagggcag ggacgtattc 180
aatgcaagct gatgacttgc gtttactagg aattcctcgt tggagattaa taattgcaaa 240
aatctagccc cagcacgatg ggcgttcaaa ggattagcca tgccttccga caaagcaacg 300
ctgtgagtaa acacaacaaa gacccgctga acccatcagt gtagcgcgcg tgcggcccag 360
aacatctaag ggcatcacag acctgttatt gcctcgaact tcctgctcgt aaaccgaaca 420
tgtccctcta agaagccaca aacacatatg tcgccatacg cccggctatt tagtaggccg 480
aggtctcgtt cgttaacgga attaaccaga caaatcactc caccaactaa gaacggccat 540
gcaccaccac ccatagaatc aagaaagagc tctcaatctg tcaatcctac ctatgtctgg 600
acctggtaag ttttcccgtg ttgagtcaaa ttaagccgca ggctccactc ctggtggtgc 660
ccttccgtca attcctttaa gtttcagcct tgcgaccata ctccccccgg aacccaaaga 720
ctttgatttc tcatgtgctg ctgcggaggc ccaatacaag ccacccgcaa tcgcaagtcg 780
gcatcgttta cggtctagac tacgatggta tctaatcatc ttcgatcccc agactttcgt 840
tcttgattaa tgaaaacatg cttggtaaat gccttcgctg tagttcgtct ttcggaaatc 900
caagaatttc acctctagct cctaaatacg gatacccccg actgttccta ttaatcatta 960
ctcagatgtg caaaccaaca aaatagcacc cgagtcctat ctgatcatcc cataataaac 1020
ataccggccc tacggcctgc ttggaacact ctgctttgat tacagtgaat gacgttcttc 1080
aaaagaaaaa cgaggatggc aaaggagccg caaggcaata caaggcgcat tcgcaccgag 1140
gcctgggctc ccacaccaga aattcaacta cgagcttttt aactgcaaca actttagcat 1200
acgcttctgg agctggaatt accgcggctg ctggcaccag acttgccctc cagttgatcc 1260
tcgatgaggg ttttacattg ctctcattcc aatagcaaga cgcgaagcgc cccgcattga 1320
tatttctcgt cactacctcg tggagtccac attgggtaat ttacgcgcct gctgccttcc 1380
ttggatgtgg tagccgtctc tcaggctccc tctccggagt cgagccctaa ctccccgtca 1440
cccgttatag tcaccgtagg ccaataccct accgtcgaca actgatgggg cagaaactca 1500
aacgattcat cgctccgaaa agcgatctgc tcaattatca tgactcacca agaacgttgg 1560
cttagaccta ataagtgcgg ccctccccga aagtcggccc cgtacagcac gtattaattc 1620
cagaattact gcaggtatcc gtctaaagga actaccgaag ggattataac tgatataatg 1680
agccgttcgc agtttcacag tataattcgc ttatacttac acatgcatgg cttagtcttt 1740
gagacgagcg tagggctact g 1761

<210> 45
<211> 8109
<212> DNA
<213> Aurantiochytrium sp.1

<400> 45
gacgaaaggg cctcgtgata cgcctatttt tataggttaa tgtcatgata ataatggttt 60
cttagacgtc aggtggcact tttcggggaa atgtgcgcgg aacccctatt tgtttatttt 120
tctaaataca ttcaaatatg tatccgctca tgagacaata accctgataa atgcttcaat 180
aatattgaaa aaggaagagt atgagtattc aacatttccg tgtcgccctt attccctttt 240
ttgcggcatt ttgccttcct gtttttgctc acccagaaac gctggtgaaa gtaaaagatg 300
ctgaagatca gttgggtgca cgagtgggtt acatcgaact ggatctcaac agcggtaaga 360

tccttgagag ttttcgcccc gaagaacgtt ttccaatgat gagcactttt aaagttctgc 420
tatgtggcgc ggtattatcc cgtattgacg ccgggcaaga gcaactcggt cgccgcatac 480
actattctca gaatgacttg gttgagtact caccagtcac agaaaagcat cttacggatg 540
gcatgacagt aagagaatta tgcagtgctg ccataaccat gagtgataac actgcggcca 600
acttacttct gacaacgatc ggaggaccga aggagctaac cgcttttttg cacaacatgg 660
gggatcatgt aactcgcctt gatcgttggg aaccggagct gaatgaagcc ataccaaacg 720
acgagcgtga caccacgatg cctgtagcaa tggcaacaac gttgcgcaaa ctattaactg 780
gcgaactact tactctagct tcccggcaac aattaataga ctggatggag gcggataaag 840
ttgcaggacc acttctgcgc tcggcccttc cggctggctg gtttattgct gataaatctg 900
gagccggtga gcgtgggtct cgcggtatca ttgcagcact ggggccagat ggtaagccct 960
cccgtatcgt agttatctac acgacgggga gtcaggcaac tatggatgaa cgaaatagac 1020
agatcgctga gataggtgcc tcactgatta agcattggta actgtcagac caagtttact 1080
catatatact ttagattgat ttaaaacttc atttttaatt taaaaggatc taggtgaaga 1140
tcctttttga taatctcatg accaaaatcc cttaacgtga gttttcgttc cactgagcgt 1200
cagaccccgt agaaaagatc aaaggatctt cttgagatcc tttttttctg cgcgtaatct 1260
gctgcttgca aacaaaaaaa ccaccgctac cagcggtggt ttgtttgccg gatcaagagc 1320
taccaactct ttttccgaag gtaactggct tcagcagagc gcagatacca aatactgttc 1380
ttctagtgta gccgtagtta ggccaccact tcaagaactc tgtagcaccg cctacatacc 1440
tcgctctgct aatcctgtta ccagtggctg ctgccagtgg cgataagtcg tgtcttaccg 1500
ggttggactc aagacgatag ttaccggata aggcgcagcg gtcgggctga acggggggtt 1560
cgtgcacaca gcccagcttg gagcgaacga cctacaccga actgagatac ctacagcgtg 1620
agctatgaga aagcgccacg cttcccgaag ggagaaaggc ggacaggtat ccggtaagcg 1680
gcagggtcgg aacaggagag cgcacgaggg agcttccagg gggaaacgcc tggtatcttt 1740
atagtcctgt cgggtttcgc cacctctgac ttgagcgtcg atttttgtga tgctcgtcag 1800
gggggcggag cctatggaaa aacgccagca acgcggcctt tttacggttc ctggcctttt 1860
gctggccttt tgctcacatg ttctttcctg cgttatcccc tgattctgtg gataaccgta 1920
ttaccgcctt tgagtgagct gataccgctc gccgcagccg aacgaccgag cgcagcgagt 1980
cagtgagcga ggaagcggaa gagcgcccaa tacgcaaacc gcctctcccc gcgcgttggc 2040
cgattcatta atgcagctgg cacgacaggt ttcccgactg gaaagcgggc agtgagcgca 2100
acgcaattaa tgtgagttag ctcactcatt aggcacccca ggctttacac tttatgcttc 2160
cggctcgtat gttgtgtgga attgtgagcg gataacaatt tcacacagga aacagctatg 2220
accatgatta cgccaagctt gcatgcctgc aggtcgactc tagaggatcc caaaaaaagc 2280
cgttactcac atcaggccgc cactcatccg ggcgaaagct tcgcgcattc gtcctcgtca 2340
cctcgggtcc cctgtgtcgg gacggaaagt gcgacgagac gcggccgcag cagagagccc 2400
cgggggcccg cgtcacgggg ggcctggcgg cagtcctcct taagccaaac cgagggttag 2460
ggctccaggc tgttcggcgg ggtcgcgggc gaggtggaag cacgggccgg cctagcacct 2520
cctagcgcgc gactaccagg atagcccccg cgagcgcgca gggcggtctg cggggcgagg 2580
gcggcccagc agcgcggcgc ggcgggcggg tgcggctgcg taaggtggcg gcgggcgcgg 2640
gcggttaggt tgggttaggt cgcggcgggg ctgtgttctg ggaatccgcc ttacggcggc 2700
gcaatggttg gctgggaggc ggtttgcggg gttagatagg cggccaaggt gagccgcgtt 2760
gggcggataa atccgtggag gcgctcgtgg acggcgcggc agagacggaa cgcggagcaa 2820
cacgaagtaa caagcaggag tagcaggagt agcaagcagc ggcaaaggaa ggctagttgt 2880
acgaggcgcg tgcccggggc agaaggcgca agcaggcagg caaacgcgat ctccgaccgc 2940
gtgcgcatcg tgcgtgcgag cgtgggcgcg agagacctcc tcgccgggcg gcaaatccgc 3000
aacgaggcca gccccgagca gcgagggcag cagcgaggga gggaggagca gcggcgacgg 3060
agagacagcc agagaaacag acgagaaagc agacgcggaa ggcacgcgcg cgaagcagga 3120
tggcggccgg aaagcaggga ggcgccaagg ccaagggcct cgcgcccgcg ggcagcgccg 3180
cagcagccgc caagaagaag tcggaaatgt ggagcggagc gtccacctcc gagagcgtgg 3240
gcatcggccc cgaggccctg cgcgccacgc tcatgccggt tctgctcgta ctttccacgc 3300
cgctcaccag catcatcctg agccgaagtg tcgtcaccac caagcacgac tcctcgttcc 3360
tcgaaaatgc atgggacgtc atcgaggaga tcctcgaaaa gggcatcgtc tacacgtaca 3420
cccgcgacgc cttcaacccc ttcgtgtgga agatgatcgg cacctacatg gtcgtccagc 3480
tcctgctcat gcgattcatg cccggaccca cgtacgaggg cccgcaaagc cccatgggca 3540
accgccccat ctacaaggac aactgctttg cctgctacgt ggcctccttt gccctctacg 3600
ggctcggcgt gtactctggc ctcttcaacg gtggcatcgc ctttgactac tttcaggaat 3660
tcattgcctc catgaacatc ttcgccctca tctttgtggc tatcctctac gtcaagggcg 3720
ccgtggctcc ctccagctcc gactcgggcc tctcgggtaa catcgccttt gactactact 3780
ggggcaccga gctgtacccg cgcatcctcg gctgggacgt caaggtcttt accaactgcc 3840
gctatggcat gactggctgg gccctgctct gcacctcgtt cgcctacgcg cagtacgagc 3900
gctttggcga ggtcagcaac tcaatcctca ttgccgccgc tctccaggtc atctacctgg 3960
ccaagttcca catttgggag cgtggctaca tgttcaccat tgacatcatg cacgaccgcg 4020
ccggctacta catttgctgg ggctgcctcg tctgggtccc cagcctctac gccgcaccca 4080
cggcctttct cgtgctccac ccgtacaact ttcccacttg ggccgccgcg gcgcagttcg 4140

tcttttgcct gctctccatc ttcctcaact acgacgtcga ccgtcagcgc caggaatttc 4200
gcgccaagga cggcaagatg aagatctggg gcaaggacgc cgagtacctc gtcgccgagt 4260
acacgacgag cgacggcaag aagcactcct ccatgctttt gcactcgggc tggtggggtc 4320
aggctcgcaa gatcaactac tttttcgagc tttgcgctgc cttttcctgg tctgtcattg 4380
ttgcccaccc ctttcacctt ctggcatggg tctactttgc ctttctcttt attctcctca 4440
tcgaccgtgc ctggcgtgac gacgcgcgct gtgcatccaa gtatggcgaa aagtgggacg 4500
agtacaaggt caaggtcccc tacctgatca tcccgggtgt ctactaagat ccgcgccggc 4560
tatgcgcccg tgctcgaccg ccacactgcc cacattgcct gcaagttcgc tgagctccag 4620
aacaagatgg accgccgctc gggcaagatt ctcgaggaga cccccaagtt catcaagtcg 4680
ggcgactctg ccatggtcaa gatgatcccc tccaagcgca tgtgcgtcga gtccttcacc 4740
gagtacccgc cgctcggccg ctttgccgtg cgcgacatgc gcgtcaccgt cgctgtcggc 4800
gtcatcaagt ccgtcaccaa gggcgacaaa taaattctac gaaagatttt tttcctcaag 4860
aagcgcccta aagttgaccc cttgcagcga cgactgtgtg tgccgttatg agtcgagttg 4920
cgatgtcgtg cagcgcccgt cgcgtcccat gctcgcgcgc gactccgtct ctgcttttga 4980
tctcagtcaa gagtgggaag ttcccttgct ttatcccact ttttagaggt cgctcacggc 5040
tgctggttcc tcgttgcatg tagcacagcc tcgtccatcg cagcctgcac caccctgctc 5100
gcctgggaaa atgcgctcag cggattcgca ctggcactcc tctcctcgga caggtgcgat 5160
gtggaagcgg tcacatcctc ggcgccctcg gccacgccag catctgcgca atcgctctcc 5220
tcgttctcag ccgcagccgc aggcaggccg acgtcgttta cctcggaatc caccgagcat 5280
ttcgagccca gcgcgctggc gtccacctcg atcatacctt ctccatcgcc gtccgctgcg 5340
gcttccgatt cttctgctgc cgcaaccgcg acgtcggccc ccgtctcctc cgttctttcc 5400
gatgccggcg cagtggccgc gccctctgct cgaaccggct cgtgttcagc gtcagggcct 5460
gcgctgagct cgggcggctc ttccgagtga tccggccccg cgaggcaagg aatcggcggc 5520
tctggagtgt cggggcagcc gctctcctgc cggtctttgg ctggctgtct gtcctgcctc 5580
gcgttggcct ttgcttttgc cttggctttc gccttggtgg cggcgtttgc ctgctgcggc 5640
gacttggcgc ggccgcagaa tagcgcctca aagtcctgct cgaggcgccc cagctctgac 5700
ttgatttgcg aggtcccggt ggcatgagct ccgctgccct cgtccttacg gcccgtcttt 5760
cgctgcaggt cgactctaga ggatccccgg gtacccgtta gaacgcgtaa tacgactcac 5820
tatagggaga gtcgactgag cacaactctg ctgcgagcga gcctcgagag cgtttgcttc 5880
gagccgcgga gcaaggggga tggatcgctc atgcggtcgt gcggccctcg gtcacccggt 5940
gggtcctgca ctgacgcatc tgttctgatc agacacacga acgaacaaac cgaggagccg 6000
cagcgcctgg tgcacccgcc gggcgttgtt gtgtgctctt cttgcctccg agagagagag 6060
cggagcggat gcataggaaa tcgggccacg cgggagggcc atgcgttcgc cccacacgcc 6120
actttccacg cccgctctct ctccggccgg caggcagcgc ataactctcc gacgctggca 6180
ggctggtagc aactggcagg gacaactcgc gcgcgggtcc cggtcgttcg atgtgccaac 6240
ccgagggaat ccagccagca gggcggttgg cctcatcgcc cacctgctat ggtgcagcga 6300
accaactccc gaagctgccg gttctgcgat tccctcttct gaattctgaa ttctgaactg 6360
attccggagg agaaccctct ggaagcgcgg gttgcctctc cagttctgcc gaactagaca 6420
ggggagtgag cagagagtga ccctgacgcg gagcgagctg gttgctggaa aagtcgcgaa 6480
cgctgggctg tgtcacgcgt ccacttcggg cagaccccaa acgacaagca gaacaagcaa 6540
caccagcagc agcaagcgat ctaagcaaca ctagccaaca tgattgaaca ggacggcctt 6600
cacgctggct cgcccgctgc ttgggtggaa cggctgttcg gctacgactg ggctcagcag 6660
acgatcggct gctcggacgc ggccgtgttc cgccttagcg cgcagggccg gccggtcctg 6720
tttgtcaaga ccgaccttag cggcgccctc aacgagctcc aggacgaagc tgcccgcctc 6780
agctggcttg ccacgacggg ggttccgtgc gccgctgtgc tcgacgtcgt caccgaagcc 6840
ggccgcgact ggctgctcct cggggaagtg cccggccagg acctcctcag cagccacctc 6900
gcgcccgctg agaaggtgtc catcatggcc gacgccatgc gccgcctgca caccctcgac 6960
cccgccacct gccccttcga ccaccaggcg aagcacagga tcgaacgcgc ccgcacgcgg 7020
atggaggctg gcctcgtcga ccaagacgac ctcgacgagg agcaccaggg cctcgcgccg 7080
gcggaactgt tcgccaggct taaggctagg atgccggacg gcgaggacct cgtggtcacg 7140
cacggcgacg cctgcctccc caacatcatg gtcgagaacg gccgcttctc gggctttatc 7200
gactgcgggc gcctgggcgt ggcggaccgc taccaagaca tcgcgctcgc cacgcgggac 7260
atcgccgagg agcttggcgg cgagtgggcc gaccgctttc tcgtgctcta cggcatcgcc 7320
gccccggaca gccagaggat tgcgttctac cgcctcctgg acgagttctt ttgagatccg 7380
cgaaatgacc gaccaagcga cgcccaacct gccatcacga gatttcgatt ccaccgccgc 7440
cttctatgaa aggttgggct tcggaatcgt tttccgggac gccggctgga tgatcctcca 7500
gcgcggggat ctcatgctgg agttcttcgc ccaccccaac ttgtttattg cagcttataa 7560
tggttacaaa taaagcaata gcatcacaaa tttcacaaat aaagcatttt tttcactgca 7620
ttctagttgt ggtttgtcca aactcatcaa tgtatcttat catgtctgta taccgtcgac 7680
ctctagctag atctcacatt aattgcgtga attcactggc cgtcgtttta caacgtcgtg 7740
actgggaaaa ccctggcgtt acccaactta atcgccttgc agcacatccc cctttcgcca 7800
gctggcgtaa tagcgaagag gcccgcaccg atcgcccttc ccaacagttg cgcagcctga 7860
atggcgaatg gcgcctgatg cggtattttc tccttacgca tctgtgcggt atttcacacc 7920

gcatatggtg cactctcagt acaatctgct ctgatgccgc atagttaagc cagccccgac 7980
acccgccaac acccgctgac gcgccctgac gggcttgtct gctcccggca tccgcttaca 8040
gacaagctgt gaccgtctcc gggagctgca tgtgtcagag gttttcaccg tcatcaccga 8100
aacgcgcga 8109

<210> 46
<211> 5404
<212> DNA
<213> Aurantiochytrium sp.1

<400> 46
tggccgattc attaatgcag ctggcacgac aggtttcccg actggaaagc gggcagtgag 60
cgcaacgcaa ttaatgtgag ttagctcact cattaggcac cccaggcttt acactttatg 120
cttccggctc gtatgttgtg tggaattgtg agcggataac aatttcacac aggaaacagc 180
tatgaccatg attacgccaa gcttgcatgc ctgcaggtcg actctagagg atcccaaaaa 240
aagccgttac tcacatcagg ccgccactca tccgggcgaa agcttcgcgc attcgtcctc 300
gtcacctcgg gtcccctgtg tcgggacgga aagtgcgacg agacgcggcc gcagcagaga 360
gcccccgggg cccgcgtcac ggggggcctg gcggcagtcc tccttaagcc aaaccgaggg 420
ttagggctcc aggctgttcg gcggggtcgc gggcgaggtg gaagcacggg gccgcctagc 480
acctcctagc gcgcgactac caggatagcc cccgcgagcg cgcagggcgg tctgcggggc 540
gagggcggcc cagcagcgcg gcgcggcggg cgggtgcggc tgcgtaaggt ggcggcgggc 600
gcgggcggtt aggttgggtt aggtcgcggc ggggctgtgt tctgggaatc cgccttacgg 660
cggcgcaatg gttggctggg aggcggtttg cggggttaga taggcggcca aggtgagccg 720
cgttgggcgg ataaatccgt ggaggcgctc gtggacggcg cggcagagac ggaacgcgga 780
gcaacacgaa gtaacaagca ggagtagcag gagtagcaag cagcggcaaa ggaaggctag 840
atgacggagc agcggggcat cttcgtgaca gtcttcctga tgcccgtgag cgtggtctgg 900
gacatcatgt tcgcactgcg ctcgtggctc atcatggcgc tgtactccgc gccagagctg 960
cacaaacagc gtgtgcagga agcctccgct caggtccgtg acgccacgac caacgccccg 1020
cccggcacca agctgtgcac cgcgcgcggg gggtggctct ccatctcgct cagtctgcgg 1080
ccgtacaaaa aggtggccac caacatctcc attaacatgt acgacatcct cgagcttaac 1140
gaaaccgaaa gctatgtcca tgtcgagccc atggtcaaca tgggccagct tagccactac 1200
ctcatccctc gcggctacac catcccggtg ctccccgaaa tggacgacct caccgtcgga 1260
ggtctcttca tgggcaccgg catcgaggcc agctcgcaca aatttggtct ctttgacgag 1320
ttcgtcatcg aggccgaggt catcctcgcc tccggcgagg tcgtcacttg cagcaagacc 1380
cagaaccgcg acctcttcga cgcgctcccc tggtcgtacg gcacgctcgg cttcctcgtc 1440
tcctgcaaga tcaaggtcat cccttgcaag ccctttatcc gcatcgagta ccgcggctgc 1500
cacaacaaaa agcagggcct cgagcttttc accaaggcct cctgcatgga catcccgccc 1560
gactttgtcg agtgcatcgc ctactcatcc gagacaatgg tagttatgac ctccgatatg 1620
gtcgacgaag atgccgtcga aaaggacaag atcaacaaga tcggccgctg gtacaaaccc 1680
tggtacttca agcacgtcga gaagatgctc gagatctcct cgcccgaaaa gccccacgtc 1740
gagtacatcc ccatccgcca gttcttccac cgctggacct cggccatctt ctgggagctc 1800
gagcagatca tcccctttgg caaccatccg ctctaccgcg ctctcctcgg atggttcaca 1860
ccttcggtcg ccctgcttaa actcacgcag acggaggcca ttcgcaagac ctacgaggag 1920
cagcacattg tccaggacat gctcgtgcct atctcaaaga cctccgaggc gctcgatgtc 1980
tttgacacgc actaccaaat ttaccccctc tggatctgcc cctaccgcca gtacgactac 2040
agcgacgcca ccacggagca ccgccacttt cttcgcaagc ccaacaagcc caacaaggag 2100
ggctacgaga tgtatgtcga tctcggcgcc tacggtgtcc cgcgcccctg cctcgagaag 2160
aagccttttg atgttatcaa gtcctctcgc gctgtcgaaa agtatgtcac cgaagccaat 2220
ggtagccaga tgctctacgc caccagctac ctcagccgcg atgagttcag ggagatgttc 2280
aaccatgcac actacgacaa gatgaagcaa aaggttgacc ccaacgactt ctttcctcag 2340
gtctacgaga aggtctgcaa gggcgccatg aacttttggc aggcaaaagc tggtgacaag 2400
aagcttgact gagatccgcg ccggctatgc gcccgtgctc gaccgccaca ctgcccacat 2460
tgcctgcaag ttcgctgagc tccagaacaa gatggaccgc cgctcgggca agattctcga 2520
ggagaccccc aagttcatca agtcgggcga ctctgccatg gtcaagatga tcccctccaa 2580
gcgcatgtgc gtcgagtcct tcaccgagta cccgccgctc ggccgctttg ccgtgcgcga 2640
catgcgcgtc accgtcgctg tcggcgtcat caagtccgtc accaagggcg acaaataaat 2700
tctacgaaag atttttttcc tcaagaagcg ccctaaagtt gaccccttgc agcgacgact 2760
gtgtgtgccg ttatgagtcg agttgcgatg tcgtgcagcg cccgtcgcgt cccatgctcg 2820
cgcgcgactc cgtctctgct tttgatctca gtcaagagtg ggaagttccc ttgctttatc 2880
ccacttttta gaggtcgctc acggctgctg gttcctcgtt gcatgtagca cagcctcgtc 2940
catcgcagcc tgcaccaccc tgctcgcctg ggaaaatgcg ctcagcggat tcgcactggc 3000
actcctctcc tcggacaggt gcgatgtgga agcggtcaca tcctcggcgc cctcggccac 3060

gccagcatct gcgcaatcgc tctcctcgtt ctcagccgca gccgcaggca ggccgacgtc 3120
gtttacctcg gaatccaccg agcatttcga gcccagcgcg ctggcgtcca cctcgatcat 3180
accttctcca tcgccgtccg ctgcggcttc cgattcttct gctgccgcaa ccgcgacgtc 3240
ggcccccgtc tcctccgttc tttccgatgc cggcgcagtg gccgcgccct ctgctcgaac 3300
cggctcgtgt tcagcgtcag ggcctgcgct gagctcgggc ggctcttccg agtgatccgg 3360
ccccgcgagg caaggaatcg gcggctctgg agtgtcgggg cagccgctct cctgccggtc 3420
tttggctggc tgtctgtcct gcctcgcgtt ggcctttgct tttgccttgg ctttcgcctt 3480
ggtggcggcg tttgcctgct gcggcgactt ggcgcggccg cagaatagcg cctcaaagtc 3540
ctgctcgagg cgccccagct ctgacttgat ttgcgaggtc ccggtggcat gagctccgct 3600
gccctcgtcc ttacggcccg tctttcgctg caggtcgact ctagaggatc cccgggtacc 3660
cgttagaacg cgtaatacga ctcactatag ggagagtcga ctgagcacaa ctctgctgcg 3720
agcgagcctc gagagcgttt gcttcgagcc gcggagcaag ggggatggat cgctcatgcg 3780
gtcgtgcggc cctcggtcac ccggtgggtc ctgcactgac gcatctgttc tgatcagaca 3840
cacgaacgaa caaaccgagg agccgcagcg cctggtgcac ccgccgggcg ttgttgtgtg 3900
ctcttcttgc ctccgagaga gagagcggag cggatgcata ggaaatcggg ccacgcggga 3960
gggccatgcg ttcgccccac acgccacttt ccacgcccgc tctctctccg gccggcaggc 4020
agcgcataac tctccgacgc tggcaggctg gtagcaactg gcagggacaa ctcgcgcgcg 4080
ggtcccggtc gttcgatgtg ccaacccgag ggaatccagc cagcagggcg gttggcctca 4140
tcgcccacct gctatggtgc agcgaaccaa ctcccgaagc tgccggttct gcgattccct 4200
cttctgaatt ctgaattctg aactgattcc ggaggagaac cctctggaag cgcgggttgc 4260
ctctccagtt ctgccgaact agacagggga gtgagcagag agtgaccctg acgcggagcg 4320
agctggttgc tggaaaagtc gcgaacgctg ggctgtgtca cgcgtccact tcgggcagac 4380
cccaaacgac aagcagaaca agcaacacca gcagcagcaa gcgatctaag caacactagc 4440
caacatggcc aagttgacca gtgccgttcc ggtgctcacc gcgcgcgacg tcgccggagc 4500
ggtcgagttc tggaccgacc ggctcgggtt ctcccgggac ttcgtggagg acgacttcgc 4560
cggtgtggtc cgggacgacg tgaccctgtt catcagcgcg gtccaggacc aggtggtgcc 4620
ggacaacacc ctggcctggg tgtgggtgcg cggcctggac gagctgtacg ccgagtggtc 4680
ggaggtcgtg tccacgaact tccgggacgc ctccgggccg gccatgaccg agatcggcga 4740
gcagccgtgg gggcgggagt tcgccctgcg cgacccggcc ggcaactgcg tgcacttcgt 4800
ggccgaggag caggactgag atccgcgaaa tgaccgacca agcgacgccc aacctgccat 4860
cacgagattt cgattccacc gccgccttct atgaaaggtt gggcttcgga atcgttttcc 4920
gggacgccgg ctggatgatc ctccagcgcg gggatctcat gctggagttc ttcgcccacc 4980
ccaacttgtt tattgcagct tataatggtt acaaataaag caatagcatc acaaatttca 5040
caaataaagc atttttttca ctgcattcta gttgtggttt gtccaaactc atcaatgtat 5100
cttatcatgt ctgtataccg tcgacctcta gctagatctc acattaattg cgtgaattca 5160
ctggccgtcg ttttacaacg tcgtgactgg gaaaaccctg gcgttaccca acttaatcgc 5220
cttgcagcac atcccccttt cgccagctgg cgtaatagcg aagaggcccg caccgatcgc 5280
ccttcccaac agttgcgcag cctgaatggc gaatggcgcc tgatgcggta ttttctcctt 5340
acgcatctgt gcggtatttc acaccgcata tggtgcactc tcagtacaat ctgctctgat 5400
gccg 5404

<210> 47
<211> 20
<212> DNA
<213> Artificial Sequence

<220>
<223> primer

<400> 47
cggcatcaga gcagattgta 20

<210> 48
<211> 20
<212> DNA
<213> Artificial Sequence

<220>
<223> primer

<400> 48

tggccgattc attaatgcag                                                    20

<210>  49
<211>  21
<212>  DNA
<213>  Artificial Sequence

<220>
<223>  primer

<400>  49
actgcagcgc acaccatgat g                                                 21

<210>  50
<211>  100
<212>  DNA
<213>  Artificial Sequence

<220>
<223>  primer

<400>  50
gacctctagc tagatctcac attaattgcg tgaattcact ggccgtcgtt ttacaacgtc      60
gtgactggga aaactttcac caaagtacta gagcgttgcg                            100

<210>  51
<211>  100
<212>  DNA
<213>  Artificial Sequence

<220>
<223>  primer

<400>  51
aactcgctag ctagcaaagt agctgcaaat tccaaataga tacgtcgagg gacgcaacgc      60
aacgctctag tactttggtg aaagttttcc cagtcacgac                            100

<210>  52
<211>  100
<212>  DNA
<213>  Artificial Sequence

<220>
<223>  primer

<400>  52
tttatgtcct caaagagctt gcgtacatcg tgcggcaagt tgtgaagaaa agtgactgac      60
ggtgatgcgc acgtagaccc ttccaggaaa cagctatgac                            100

<210>  53
<211>  100
<212>  DNA
<213>  Artificial Sequence

<220>
<223>  primer

<400>  53

caggcgctgc ggcggatcct ctagagtcga cctgcaggca tgcaagcttg gcgtaatcat 60
ggtcatagct gtttcctgga agggtctacg tgcgcatcac 100

<210> 54
<211> 20
<212> DNA
<213> Artificial Sequence

<220>
<223> primer

<400> 54
ggagcgctac tgccccacac 20

<210> 55
<211> 1011
<212> DNA
<213> Aurantiochytrium sp.1

<400> 55
atggtcgttc tctgggagaa gggcgagaac cactcgtcgg accagttcgt tggcgccatc 60
aaccatgcca tgaagaacac cgtggacaac gaggacaagg acaagaacta cgcacccgtg 120
gtcaagagct tctacgcctc ggtcgccacc agcctctacg agtacggctg gggcaagtcc 180
ttccacttcg ccccgcgcaa caagggcgag ggctttaagg agtccctcct ccgccaggag 240
cactggctcg ccgcccagct cggcatccgc cccggccagg atgtctgtga cctcggctgc 300
ggtgtctgcg gccccctcgt caacatcgcc aagttctccc gcgccaacat caccggcgtg 360
accatctccg acttccagat cgcccgcggc aacgcctgga tcgcccagaa cggcctctcc 420
aaccgctgca aggccgtcga gggtgacttc cacgagctcc ccttcgagga caactccttc 480
gactgcggct acgactgcga ggccaccgcc cactccacca agctcaacac cttcttcgcc 540
gaggtcgagc gcgtcctccg ccccggtggt gtcttcgccg gcttcgcctg ggtcaccctc 600
cccaagcaca acccggagga ccccgaggag gtcaagattc tcgaggacct tgcctttggc 660
aacgccattg ccgtcctcca cagctttgac gactacgtca acgccatcaa ggctaacccc 720
ggtctcgagc tcgtcgaggc ctacgacgcc aacaagctcg gcgacgacat ggagtggcac 780
gagcccctca agcctggctt ctctctcgac ggcatcctca tgtcctgggc tggccgccag 840
ctcaccgcga acctcgcccg cgtcaccgag gccatcggcc ttgcccccaa gggcacctac 900
aacgcctccc aggtcctcga ggttgctgcc cgctccctcg tcgacgccgg cgaccgcggt 960
atctacaccc ccatctacta ctacaaggtc cgcaaggtcg ccaagaacta a 1011

<210> 56
<211> 336
<212> PRT
<213> Aurantiochytrium sp.1

<400> 56
Met Val Val Leu Trp Glu Lys Gly Glu Asn His Ser Ser Asp Gln Phe
1               5                   10                  15
Val Gly Ala Ile Asn His Ala Met Lys Asn Thr Val Asp Asn Glu Asp
            20                  25                  30
Lys Asp Lys Asn Tyr Ala Pro Val Val Lys Ser Phe Tyr Ala Ser Val
        35                  40                  45
Ala Thr Ser Leu Tyr Glu Tyr Gly Trp Gly Lys Ser Phe His Phe Ala
    50                  55                  60
Pro Arg Asn Lys Gly Glu Gly Phe Lys Glu Ser Leu Leu Arg Gln Glu
65                  70                  75                  80
His Trp Leu Ala Ala Gln Leu Gly Ile Arg Pro Gly Gln Asp Val Cys
                85                  90                  95
Asp Leu Gly Cys Gly Val Cys Gly Pro Leu Val Asn Ile Ala Lys Phe
            100                 105                 110
Ser Arg Ala Asn Ile Thr Gly Val Thr Ile Ser Asp Phe Gln Ile Ala
        115                 120                 125

Arg Gly Asn Ala Trp Ile Ala Gln Asn Gly Leu Ser Asn Arg Cys Lys
    130                 135                 140
Ala Val Glu Gly Asp Phe His Glu Leu Pro Phe Glu Asp Asn Ser Phe
145                 150                 155                 160
Asp Cys Gly Tyr Asp Cys Glu Ala Thr Ala His Ser Thr Lys Leu Asn
                165                 170                 175
Thr Phe Phe Ala Glu Val Glu Arg Val Leu Arg Pro Gly Gly Val Phe
            180                 185                 190
Ala Gly Phe Ala Trp Val Thr Leu Pro Lys His Asn Pro Glu Asp Pro
        195                 200                 205
Glu Glu Val Lys Ile Leu Glu Asp Leu Ala Phe Gly Asn Ala Ile Ala
    210                 215                 220
Val Leu His Ser Phe Asp Asp Tyr Val Asn Ala Ile Lys Ala Asn Pro
225                 230                 235                 240
Gly Leu Glu Leu Val Glu Ala Tyr Asp Ala Asn Lys Leu Gly Asp Asp
                245                 250                 255
Met Glu Trp His Glu Pro Leu Lys Pro Gly Phe Ser Leu Asp Gly Ile
            260                 265                 270
Leu Met Ser Trp Ala Gly Arg Gln Leu Thr Ala Asn Leu Ala Arg Val
        275                 280                 285
Thr Glu Ala Ile Gly Leu Ala Pro Lys Gly Thr Tyr Asn Ala Ser Gln
    290                 295                 300
Val Leu Glu Val Ala Ala Arg Ser Leu Val Asp Ala Gly Asp Arg Gly
305                 310                 315                 320
Ile Tyr Thr Pro Ile Tyr Tyr Tyr Lys Val Arg Lys Val Ala Lys Asn
                325                 330                 335